# EUROPEAN PATENT SPECIFICATION

(11) **EP 1 192 277 B1**
(45) Date of publication and mention of the grant of the patent: **06.08.2008**
(21) Application number: 00937731.8
(22) Date of filing: 24.05.2000
(51) Int. Cl.: C12Q 1/68

(54) **Diagnosis of restenosis**
Diagnose von Restenose
Méthodes diagnostiques pour la resténose

(30) Priority: 24.05.1999 US 317674; 01.11.1999 US 431352
(43) Date of publication of application: 03.04.2002
(73) Proprietor: Interleukin Genetics, Inc., San Antonio, TX 78216-4749 (US)
(72) Inventor: KORNMAN, Kenneth, S., San Antonio, TX 78230 (US); DUFF, Gordon, W., Sheffield S10 3BZ, South Yorkshire (GB); CROSSMAN, David, C., South Yorkshire (GB); FRANCIS, Sheila, E., South Yorkshire (GB); STEPHENSON, Katherine, San Antonio, TX 78230 (US)
(74) Representative: Crump, Julian Richard John
(86) International application number: PCT/US2000/014299
(87) International publication number: WO 2000/071753

(56) References cited:
- WO-A-98/40517
- FRANCIS, S.E. ET AL.: "Interleukin-1 Receptor Antagonist Gene Polymorphism and Coronary Artery Disease" CIRCULATION, vol. 99, no. 7, 23 February 1999 (1999-02-23), pages 861-866, XP001030756
- KASTRATI, A. ET AL.: "Protection Against Restenosis From an Interleukin-1 Receptor Antagonist Gene Polymorphism in Patients Treated with Coronary Stenting" J. AM. COLLEGE CARDIOLOGY, vol. 35, no. 2, Suppl. A, February 2000 (2000-02), page 36A XP001030751
- KORNMAN, K. ET AL.: "Interleukin-1 genotypes and the association between periodontitis and cardiovascular disease" J. PERIODONT. RES., vol. 34, no. 7, October 1999 (1999-10), pages 353-357, XP001030774
- ZEE, R. Y. I. ET AL.: "Genetic Risk Factors for Post-PTCA Restenosis: A Comprehensive Analysis of Multiple Candidate Genes" CIRCULATION, vol. 110, no. 18 Suppl., 2 November 1999 (1999-11-02), page I.755 XP001030778

## Description

### 1. Background of the Invention

### Restenosis

Percutaneous transluminal coronary angioplasty (PTCA) is used to treat obstructive coronary artery disease by compressing atheromatous plaque to the sides of the vessel wall. PTCA is widely used with an initial success rate of over 90%. Approximately 666,000 angioplasties were conducted in the United States alone in 1996, and more of these procedures were performed on men (452,000) than women (214,000). Of this total, 482,000 were percutaneous transluminal coronary angioplasty (P.T.C.A. (American Heart Association; www.amhrt.org). Despite the frequent application of this procedure and its high initial success rate, the long-term success of PTCA is limited by intraluminal renarrowing or restenosis at the site of the procedure. This occurs within 6 months following the procedure in approximately 30% to 40% of patients who undergo a single vessel procedure and in more than 50% of those who undergo multivessel angioplasty.

Stent placement has largely supplanted balloon angioplasty because it is able to more widely restore intraluminal dimensions which has the effect of reducing restenosis by approximately 50%. Ironically, stent placement actually increases neointimal growth at the treatment site, but because a larger lumen can be achieved with stent placement, the tissue growth is more readily accommodate, and sufficient luminal dimensions are maintained, so that the restenosis rate is nearly halved by stent placement compared with balloon angioplasty alone.

The pathophysiological mechanisms involved in restenosis are not fully understood. While a number of clinical, anatomical and technical factors have been linked to the development of restenosis, at least 50% of the process has yet to be explained. However, it is known that following endothelial injury, a series of repair mechanisms are initiated. Within minutes of the injury, a layer of platelets and fibrin is deposited over the damaged endothelium. Within hours to days, inflammatory cells begin to infiltrate the injured area. Within 24 hours after an injury, vascular smooth muscle cells (SMCs) located in the vessel media commence DNA synthesis. A few days later, these activated, synthetic SMCs migrate through the internal elastic lamina towards the luminal surface. A neointima is formed by these cells by their continued replication and their production of extracellular matrix. An increase in the intimal thickness occurs with ongoing cellular proliferation matrix deposition. When these processes of vascular healing progress excessively, the pathological condition is termed intimal hyperplasia or neointimial hyperplasia. Histological studies in animal models have identified neointimal hyperplasia as the central element in restenosis.

Neointimal hyperplasia is understood to figure prominently in peripheral vascular restenosis following reconstructive procedures. One series of 5,000 arterial reconstructions reports 50% of late failures to be due to neointimal hyperplasia (Imparato et al. (1972) Surg. 72:1107-1117). Restenosis following stenting is similarly thought to involve an important component of neointimal hyperplasia (Dussaillant et al. (1995) J. Am. Coll. Cardiol 26:720-724). In the coronary system, by contrast, restenosis following balloon angioplasty involves vascular remodeling as well as neointimal hyperplasia. The importance of vascular remodeling in this setting may be attributable to the nature of the injury to the vessel wall following balloon angioplasty. Commonly, the injury to the vessel wall with this procedure involves dissection planes extending through the atherosclerotic plaque into the vessel media (Mintz et al. (1996) Circ. 94:35043). Furthermore, plaque fracture, medial stretch, focal medial rupture and adventitial stretch all may occur following angioplasty. Repair of the deeper layers of the vessel wall takes place by the general processes of wound healing, including inflammation, neovascularization, fibroblast proliferation and eventual collagen deposition. Cumulatively, these processes lead to remodeling of the coronary vessel wall that may culminate in restenosis.

The biology of vascular wall healing implicated in restenosis therefore includes the general processes of wound healing and the specific processes of neointimal hyperplasia. Inflammation is generally regarded as an important component in both these processes. (Munro and Cotran (1993) Lab. Investig. 58:249-261; and Badimon et al. (1993), Supp II 87:3-6). Understanding the effects of acute and chronic inflammation in the blood vessel wall can thus suggest methods for diagnosing and treating restenosis and related conditions.

In its initial phase, inflammation is characterized by the adherence of leukocytes to the vessel wall. Leukocyte adhesion to the surface of damaged endothelium is mediated by several complex glycoproteins on the endothelial and neutrophil surfaces. Two of these binding molecules have been well-characterized: the endothelial leukocyte adhesion molecule-1 (ELAM-1) and the intercellular adhesion molecule-1 (ICAM-1). During inflammatory states, the attachment of neutrophils to the involved cell surfaces is greatly increased, primarily due to the upregulation and enhanced expression of these binding molecules. Substances thought to be primary mediators of the inflammatory response to tissue injury, including interleukin-1 (IL-1), tumor necrosis factor alpha (TNF-α), lymphotoxin and bacterial endotoxins, all increase the production of these binding substances.

After binding to the damaged vessel wall, leukocytes migrate into it. Once in place within the vessel wall, the leukocytes, in particular activated macrophages, then release additional inflammatory mediators, including IL-1, TNF, prostaglandin E₂, (PGE₂), bFGF, and transforming growth factors α and β (TGFα, TGFβ). All of these inflammatory mediators recruit more inflammatory cells to the damaged area, and regulate the further proliferation and migration of smooth muscle. A well-known growth factor elaborated by the monocyte-macrophage is monocyte- and macrophage-derived growth factor (MDGF), a stimulant of smooth muscle cell and fibroblast proliferation. MDGF is understood to be similar to platelet-derived growth factor (PDGF); in fact, the two substances may be identical. By stimulating smooth muscle cell proliferation, inflammation can contribute to the development and the progression of neointimal hyperplasia.

Leukocytes, attracted to the vessel wall by the abovementioned chemical mediators of inflammation, produce substances that have direct effects on the vessel wall that may exacerbate the local injury and prolong the healing response. First, leukocytes activated by the processes of inflammation secrete lysosomal enzymes that can digest collagen and other structural proteins. Releasing these enzymes within the vessel wall can affect the integrity of its extracellular matrix, permitting SMCs and other migratory cells to pass through the wall more readily. Hence, the release of these lysosomal proteases can enhance the processes leading to neointimal hyperplasia. Second, activated leukocytes produce free radicals by the action of the NADPH system on their cell membranes. These free radicals can damage cellular elements directly, leading to an extension of a local injury or a prolongation of the cycle of injury-inflammation-healing.

The responses to vascular injury that lead to restenosis have certain features in common with the processes leading to the development of the vascular lesions of atherosclerosis. Currently, it is understood that the lesions of atherosclerosis are initiated by some form of injury to arterial endothelium, whether due to hemodynamic factors, endothelial dysfunction or a combination of these or other factors (Schoen, "Blood vessels," pp. 467-516 in Pathological Basis of Disease (Philadelphia: Saunders, 1994)). Inflammation has been implicated in the formation and progression of atherosclerotic lesions. Several inflammatory products, including IL-1β, have been identified in atherosclerotic lesions or in the endothelium of diseased coronary arteries (Galea, et al. (1996) Arterioscler Thromb Vasc Biol. 16:1000-6). Also, serum concentrations of IL-1β are elevated in patients with coronary disease (Hasdai, et al. (1996) Heart, 76:24-8). Realizing the importance of inflammatory processes in the final common pathways of vascular response to injury allows analogies to be drawn between the lesions seen in restenosis and those seen in atherosclerosis.

Currently, approximately 500,000 patients per year undergo vascular reconstructive procedures, with half involving the coronary vessels and the other half involving the periphery. Restenosis and progressive atherosclerosis are the most common mechanisms for late failure in these reconstructions. It would be desirable to determine which patients would respond well to invasive treatments for occlusive vascular disease such as angioplasty and intravascular stent placement. It would be further desirable to identify those patients at increased risk for stenosis so that they could be targeted with appropriate therapies to prevent, modulate or reverse the condition. It would be desirable, moreover, to identify those individuals for whom PTCA and stent placement is a suboptimal therapeutic choice because of the risk of restenosis. Those patients might become candidates at earlier stages for vascular reconstructive procedures, possibly combined with other pharmacological interventions.

### Genetics of the IL-1 Gene Cluster

The IL-1 gene cluster is on the long arm of chromosome 2 (2q13) and contains at least the genes for IL-1α (IL-1A), IL-1β (IL-1B), and the IL-1 receptor antagonist (IL-1RN), within a region of 430 Kb (Nicklin, et al. (1994) Genomics, 19: 382-4). The agonist molecules, IL-1α and IL-1β, have potent pro-inflammatory activity and are at the head of many inflammatory cascades. Their actions, often via the induction of other cytokines such as IL-6 and IL-8, lead to activation and recruitment of leukocytes into damaged tissue, local production of vasoactive agents, fever response in the brain and hepatic acute phase response. All three IL-1 molecules bind to type I and to type II IL-1 receptors, but only the type I receptor transduces a signal to the interior of the cell. In contrast, the type II receptor is shed from the cell membrane and acts as a decoy receptor. The receptor antagonist and the type II receptor, therefore, are both anti-inflammatory in their actions.

Inappropriate production of IL-1 plays a central role in the pathology of many autoimmune and inflammatory diseases, including rheumatoid arthritis, inflammatory bowel disorder, psoriasis, and the like. In addition, there are stable inter-individual differences in the rates of production of IL-1, and some of this variation may be accounted for by genetic differences at IL-1 gene loci. Thus, the IL-1 genes are reasonable candidates for determining part of the genetic susceptibility to inflammatory diseases, most of which have a multifactorial etiology with a polygenic component. Indeed, there is increasing evidence that certain alleles of the IL-1 genes are over-represented in these diseases.

Certain alleles from the IL-1 gene cluster are already known to be associated with particular disease states. For example, IL-1RN allele 2 has been shown to be associated with coronary artery disease (PCT/US/98/04725, and USSN 08/813456), osteoporosis (U.S. Patent No. 5,698,399), nephropathy in diabetes mellitus (Blakemore, et al. (1996) Hum. Genet. 97(3): 369-74), alopecia areata (Cork, et al., (1995) J. Invest. Dermatol. 104(5 Supp.): 15S-16S; Cork et al. (1996) Dermatol Clin 14: 671-8), Graves disease (Blakemore, et al. (1995) J. Clin. Endocrinol. 80(1): 111-5), systemic lupus erythematosus (Blakemore, et al. (1994) Arthritis Rheum 37: 1380-85), lichen sclerosis (Clay, et al. (1994) Hum. Genet. 94: 407-10), and ulcerative colitis (Mansfield, et al. (1994) Gastoenterol. 106(3): 637-42).

In addition, the IL-1A allele 2 from marker -889 and IL-1B (TaqI) allele 2 from marker +3954 have been found to be associated with periodontal disease (U.S. Patent No. 5,686,246; Kornman and diGiovine (1998) Ann Periodont 3: 327-38; Hart and Kornman (1997) Periodontol 2000 14: 202-15; Newman (1997) Compend Contin Educ Dent 18: 881-4; Kornman et al. (1997) J. Clin Periodontol 24: 72-77). The IL-1A allele 2 from marker -889 has also been found to be associated with juvenile chronic arthritis, particularly chronic iridocyclitis (McDowell, et al. (1995) Arthritis Rheum. 38:221-28). The IL-1B (TaqI) allele 2 from marker +3954 of IL-1B has also been found to be associated with psoriasis and insulin dependent diabetes in DR3/4 patients (di Giovine, et al. (1995) Cytokine 7: 606; Pociot, et al. (1992) Eur J. Clin. Invest. 22: 396-402). Additionally, the IL-1RN (VNTR) allele 1 has been found to be associated with diabetic retinopathy (see USSN 09/037472, and PCT/GB97/02790). Furthermore allele 2 of IL-1RN (VNTR) has been found to be associated with ulcerative colitis in Caucasian populations from North America and Europe (Mansfield, J. et al., (1994) Gastroenterology 106: 637-42). Interestingly, this association is particularly strong within populations of ethnically related Ashkenazi Jews (PCT WO97/25445).

### Genotype Screening

Traditional methods for the screening of heritable diseases have depended on either the identification of abnormal gene products (e.g., sickle cell anemia) or an abnormal phenotype (e.g., mental retardation). These methods are of limited utility for heritable diseases with late onset and no easily identifiable phenotypes such as, for example, a predisposition to restenosis. With the development of simple and inexpensive genetic screening methodology, it is now possible to identify polymorphisms that indicate a propensity to develop disease, even when the disease is of polygenic origin. The number of diseases that can be screened by molecular biological methods continues to grow with increased understanding of the genetic basis of multifactorial disorders.

Genetic screening (also called genotyping or molecular screening), can be broadly defined as testing to determine if a patient has mutations (or alleles or polymorphisms) that either cause a disease state or are "linked" to the mutation causing a disease state. Linkage refers to the phenomenon that DNA sequences which are close together in the genome have a tendency to be inherited together. Two sequences may be linked because of some selective advantage of co-inheritance. More typically, however, two polymorphic sequences are co-inherited because of the relative infrequency with which meiotic recombination events occur within the region between the two polymorphisms. The co-inherited polymorphic alleles are said to be in linkage disequilibrium with one another because, in a given human population, they tend to either both occur together or else not occur at all in any particular member of the population. Indeed, where multiple polymorphisms in a given chromosomal region are found to be in linkage disequilibrium with one another, they define a quasi-stable genetic "haplotype." In contrast, recombination events occurring between two polymorphic loci cause them to become separated onto distinct homologous chromosomes. If meiotic recombination between two physically linked polymorphisms occurs frequently enough, the two polymorphisms will appear to segregate independently and are said to be in linkage equilibrium.

While the frequency of meiotic recombination between two markers is generally proportional to the physical distance between them on the chromosome, the occurrence of "hot spots" as well as regions of repressed chromosomal recombination can result in discrepancies between the physical and recombinational distance between two markers. Thus, in certain chromosomal regions, multiple polymorphic loci spanning a broad chromosomal domain may be in linkage disequilibrium with one another, and thereby define a broad-spanning genetic haplotype. Furthermore, where a disease-causing mutation is found within or in linkage with this haplotype, one or more polymorphic alleles of the haplotype can be used as a diagnostic or prognostic indicator of the likelihood of developing the disease. This association between otherwise benign polymorphisms and a disease-causing polymorphism occurs if the disease mutation arose in the recent past, so that sufficient time has not elapsed for equilibrium to be achieved through recombination events. Therefore identification of a human haplotype which spans or is linked to a disease-causing mutational change, serves as a predictive measure of an individual's likelihood of having inherited that disease-causing mutation. Importantly, such prognostic or diagnostic procedures can be utilized without necessitating the identification and isolation of the actual disease-causing lesion. This is significant because the precise determination of the molecular defect involved in a disease process can be difficult and laborious, especially in the case of multifactorial diseases such as inflammatory disorders.

Indeed, the statistical correlation between an inflammatory disorder and an IL-1 polymorphism does not necessarily indicate that the polymorphism directly causes the disorder. Rather the correlated polymorphism may be a benign allelic variant which is linked to (i.e. in linkage disequilibrium with) a disorder-causing mutation which has occurred in the recent human evolutionary past, so that sufficient time has not elapsed for equilibrium to be achieved through recombination events in the intervening chromosomal segment. Thus, for the purposes of diagnostic and prognostic assays for a particular disease, detection of a polymorphic allele associated with that disease can be utilized without consideration of whether the polymorphism is directly involved in the etiology of the disease. Furthermore, where a given benign polymorphic locus is in linkage disequilibrium with an apparent disease-causing polymorphic locus, still other polymorphic loci which are in linkage disequilibrium with the benign polymorphic locus are also likely to be in linkage disequilibrium with the disease-causing polymorphic locus. Thus these other polymorphic loci will also be prognostic or diagnostic of the likelihood of having inherited the disease-causing polymorphic locus. Indeed, a broad-spanning human haplotype (describing the typical pattern of co-inheritance of alleles of a set of linked polymorphic markers) can be targeted for diagnostic purposes once an association has been drawn between a particular disease or condition and a corresponding human haplotype. Thus, the determination of an individual's likelihood for developing a particular disease of condition can be made by characterizing one or more disease-associated polymorphic alleles (or even one or more disease-associated haplotypes) without necessarily determining or characterizing the causative genetic variation.

### 2. Summary of the Invention

In one aspect, the present invention provides novel methods for determining whether a subject has or is predisposed to developing restenosis. Diagnosis of the presence of a restenosis disorder identifies those patients predisposed to the development of a restenosis disease, characterized by clinical events related to the recurrence of the initial vascular stenosis that is being treated by the stent. Determining which patients are at risk for developing the disease because they have the disorder thus opens the possibility of selecting therapies for the initial vascular stenosis most likely to avoid subsequent stenoses. Such patients might be preferred candidates for surgical revascularization rather than percutaneous transluminal angioplasty, for example, or such patients may benefit from pharmacological or topical interventions at an early stage that could affect the progression of the restenosis disorder.

In one embodiment, the method comprises determining whether the restenosis associated allele 1 of IL-1RN (VNTR) is present in a nucleic acid sample obtained from the subject predicting the susceptibility of an individual to developing restenosis. In addition, there are three patterns of alleles at four polymorphic loci in the IL-1 gene cluster that show various associations with particular cardiovascular disorders. These patterns are referred to herein as patterns 1, 2 and 3. Pattern 1 comprises an allelic pattern including allele 2 of IL-1A (+4845) or IL-1B (+3954) and allele 1 of IL-1B (-511) or IL-1RN (+2018), or an allele that is in linkage disequilibrium with one of the aforementioned allele. In a preferred embodiment, this allelic pattern permits the diagnosis of occlusive cardiovascular disorder. Pattern 2 comprises an allelic pattern including allele 2 of IL-1B (-511) or IL-1RN (+2018) and allele 1 of IL-1A (+4845) or IL-1B (+3954), or an allele that is in linkage disequilibrium with one of the aforementioned alleles. In a preferred embodiment, this allelic pattern permits the diagnosis of occlusive cardiovascular disorder. Pattern 3 comprises an allelic pattern including allele 1 of IL-1A (+4845) or allele 1 of IL-1B (+3954), and allele 1 of IL-1B (-511) or allele 1 of IL-1RN (+2018), or an allele that is in linkage disequilibrium with one of the aforementioned alleles.

An IL-1 associated polymorphism that is in linkage disequilibrium with one or more of the aforementioned restenosis-predictive alleles may be detected. For example, the following alleles of the IL-1 (44112332) haplotype are known to be in linkage disequilibrium:

| |
|---|
| allele 4 of the 222/223 marker of IL-1A |
| allele 4 of the gz5/gz6 marker of IL-1A |
| allele 1 of the -889 marker of IL-1A |
| allele 1 of the +3954 marker of IL-1B |
| allele 2 of the -511 marker of IL-1B |
| allele 3 of the gaat.p33330 marker |
| allele 3 of the Y31 marker |
| allele 2 of the VNTR or (+2018) marker of IL-1RN |

Also, the following alleles of the IL-1 (33221461) haplotype are in linkage disequilibrium:

| |
|---|
| allele 3 of the 222/223 marker of IL-1A |
| allele 3 of the gz5/gz6 marker of IL-1A |
| allele 2 of the -889 marker of IL-1A |
| allele 2 of the +3954 marker of IL-1B |
| allele 1 of the -511 marker of IL-1B |
| allele 4 of the gaat.p33330 marker |
| allele 6 of the Y31 marker |
| allele 1 of the VNTR or (+2018) marker of IL-1RN |

A restenosis associated allele can be detected by any of a variety of available techniques, including: 1) performing a hybridization reaction between a nucleic acid sample and a probe that is capable of hybridizing to the allele; 2) sequencing at least a portion of the allele; or 3) determining the electrophoretic mobility of the allele or fragments thereof (e.g., fragments generated by endonuclease digestion). The allele can optionally be subjected to an amplification step prior to performance of the detection step. Preferred amplification methods are selected from the group consisting of: the polymerase chain reaction (PCR), the ligase chain reaction (LCR), strand displacement amplification (SDA), cloning, and variations of the above (e.g. RT-PCR and allele specific amplification). Oligonucleotides necessary for amplification may be selected for example, from within the IL-1 gene loci, either flanking the marker of interest (as required for PCR amplification) or directly overlapping the marker (as in ASO hybridization). In a particularly preferred embodiment, the sample is hybridized with a set of primers, which hybridize 5' and 3' in a sense or antisense sequence to the restenosis associated allele, and is subjected to a PCR amplification.

A restenosis associated allele may also be detected indirectly, e.g. by analyzing the protein product encoded by the DNA. For example, where the marker in question results in the translation of a mutant protein, the protein can be detected by any of a variety of protein detection methods. Such methods include immunodetection and biochemical tests, such as size fractionation, where the protein has a change in apparent molecular weight either through truncation, elongation, altered folding or altered post-translational modifications.

Kits for performing the above-described assays are disclosed. The kit can include a nucleic acid sample collection means and a means for determining whether a subject carries a restenosis associated allele. The kit may also contain a control sample either positive or negative or a standard and/or an algorithmic device for assessing the results and additional reagents and components including: DNA amplification reagents, DNA polymerase, nucleic acid amplification reagents, restrictive enzymes, buffers, a nucleic acid sampling device, DNA purification device, deoxynucleotides, oligonucleotides (e.g. probes and primers) etc..

As described above, the control samples may be positive or negative controls. Further, the control sample may contain the positive (or negative) products of the allele detection technique employed. For example, where the allele detection technique is PCR amplification, followed by size fractionation, the control sample may comprise DNA fragments of the appropriate size. Likewise, where the allele detection technique involves detection of a mutated protein, the control sample may comprise a sample of mutated protein. However, it is preferred that the control sample comprises the material to be tested. For example, the controls may be a sample of genomic DNA or a cloned portion of the IL-1 gene cluster. Preferably, however, the control sample is a highly purified sample of genomic DNA where the sample to be tested is genomic DNA.

The oligonucleotides present in said kit may be used for PCR amplification of the region of interest or for direct allele specific oligonucleotide (ASO) hybridization to the markers in question. Thus, the oligonucleotides may either flank the marker of interest (as required for PCR amplification) or directly overlap the marker (as in ASO hybridization).

Such oligonucleotides can include
5'-CTC.AGC.AAC.ACT.CCT.AT-3' (SEQ ID NO.7) and
5'-TCC.TGG.TCT.GCA.GCT.AA-3' (SEQ ID NO. 8)
which can be used to amplify the human IL-1RN (VNTR) polymorphic locus; and may include but are not limited to:
5' ATG GTT TTA GAA ATC ATC AAG CCT AGG GCA 3' (SEQ ID No. 1) and
5' AAT GAA AGG AGG GGA GGA TGA CAG AAA TGT 3' (SEQ ID No. 2)
which can be used to amplify the human IL-1A (+4845) polymorphic locus;
5' TGG CAT TGA TCT GGT TCA TC 3' (SEQ ID No. 3) and
5' GTT TAG GAA TCT TCC CAC TT-3' (SEQ ID No. 4)
which can be used to amplify the human IL-1B (-511) polymorphic locus;
5'-CTC AGG TGT CCT CGA AGA AAT CAA A-3' (SEQ ID No. 5) and
5' GCT TTT TTG CTG TGA GTC CCG-3' (SEQ ID No. 6)
which can be used to amplify the human IL-1B (+3954) polymorphic locus;
5'-CTA TCT GAG GAA CAA CCA ACT AGT AGC-3' (SEQ ID NO. 9) and
5'-TAG GAC ATT GCA CCT AGG GTT TGT -3' (SEQ ID NO. 10)
which can be used to amplify the human IL-1RN (+2018) polymorphic locus;
5' ATT TTT TTA TAA ATC ATC AAG CCT AGG GCA 3' (SEQ. ID No. 11) and
5' AAT TAA AGG AGG GAA GAA TGA CAG AAA TGT 3' (SEQ. ID No. 12)
which can also be used to amplify the human IL-1A (+4845) polymorphic locus;
5'-AAG CTT GTT CTA CCA CCT GAA CTA GGC.-3' (SEQ. ID NO. 13) and
5'-TTA CAT ATG AGC CTT CCA TG.-3' (SEQ. ID NO. 14)
which can be used to amplify the human IL-1A (-889) polymorphic locus;

Information obtained using the assays and kits described herein (alone or in conjunction with information on another genetic defect or environmental factor, which contributes to restenosis) is useful for determining whether a non-symptomatic subject has or is likely to develop restenosis. In addition, the information can allow a more customized approach to preventing the onset or progression of restenosis. For example, this information can enable a clinician to more effectively prescribe a therapy that will address the molecular basis of restenosis.

Other embodiments and advantages of the invention are set forth in part in the description which follows, and will be obvious from this description.

### 3. Brief Description of the Figures

FIG. 1 shows the nucleic acid sequence for IL-1A (GEN X03833; SEQ ID No. 15).
FIG. 2 shows the nucleic acid sequence for IL-1B (GEN X04500; SEQ ID No. 16).
FIG. 3 shows the nucleic acid sequence for the secreted IL-1RN (GEN X64532; SEQ ID No. 17).
FIG. 4 depicts the organization of the IL-1 genes, and associated polymorphic loci, on human chromosome 2.

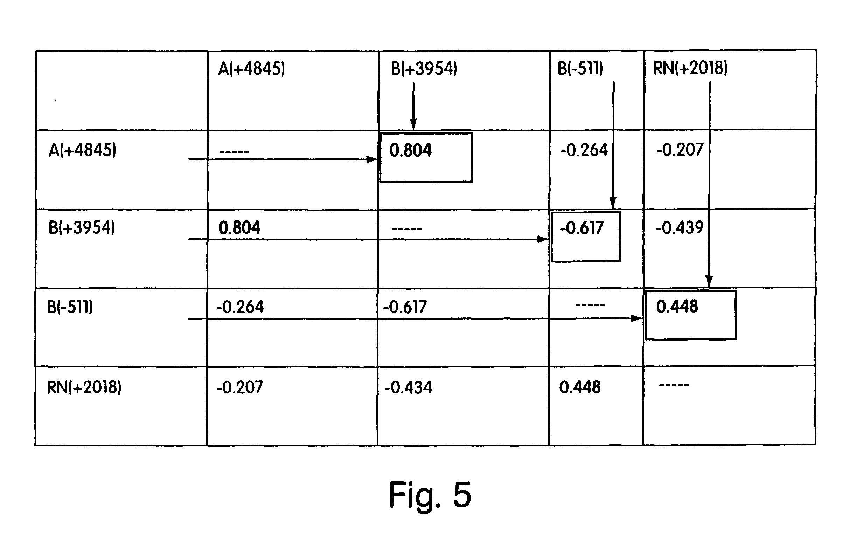

FIG. 5 shows linkage disequilibrium values for the IL-1 polymorphic loci in a Caucasian population.

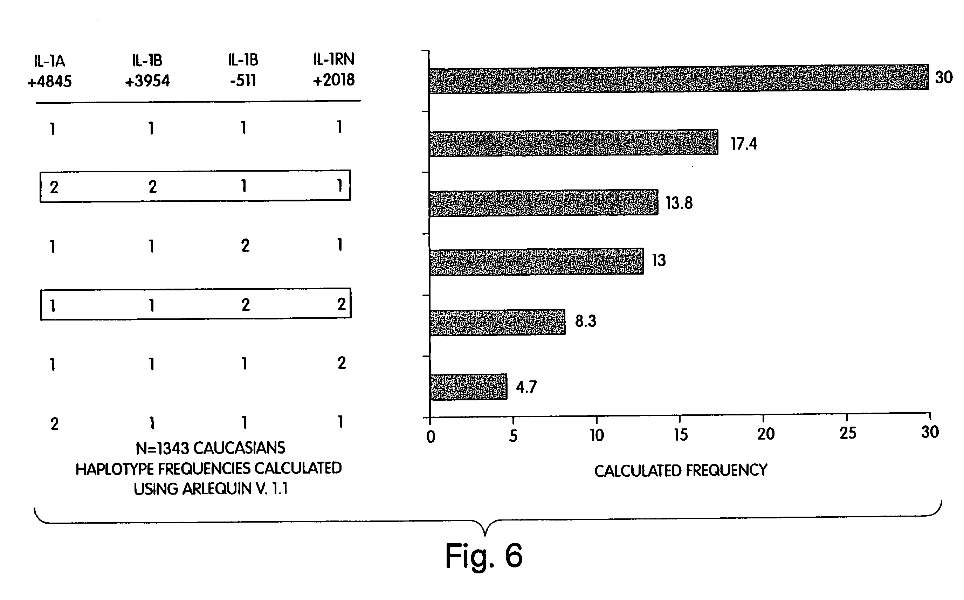

FIG. 6 is a bar graph illustrating the frequency of particular IL-1 polymorphic allelic patterns in a Caucasian population.

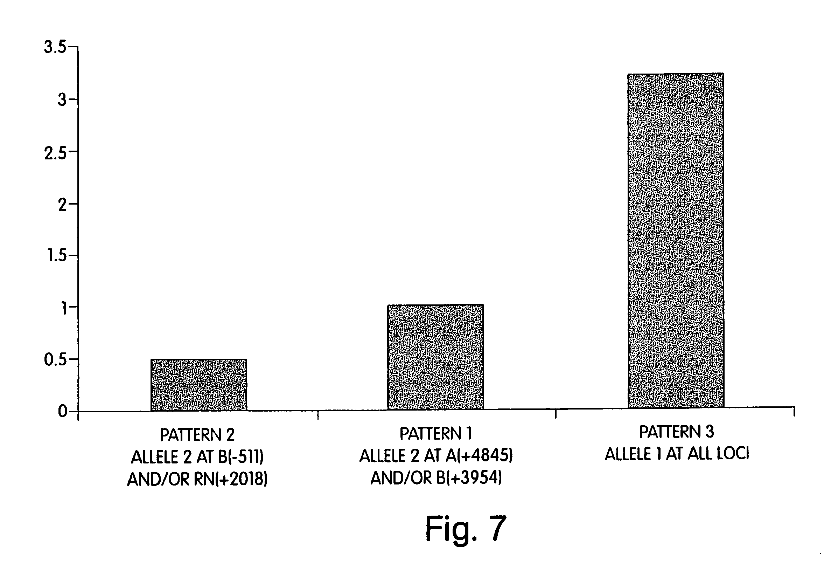

FIG. 7 indicates the relative risk for restenosis associated with each of the IL-1 polymorphic patterns.

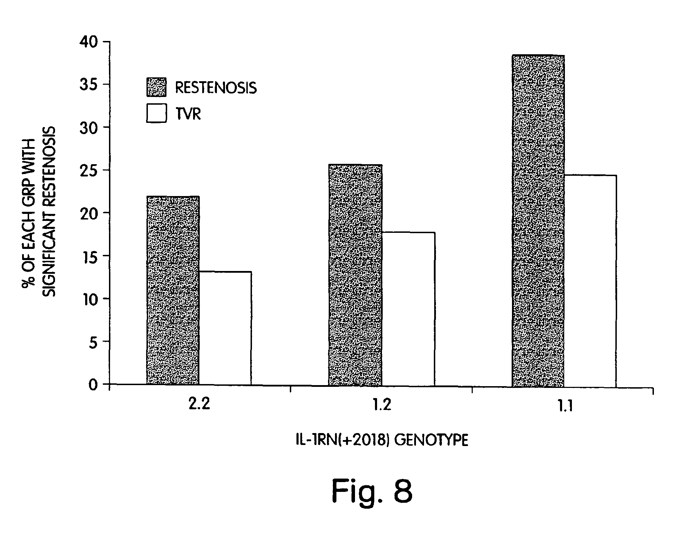

FIG. 8 indicates the association between homozygous and heterozygous allelic patterns at the IL-1RN(+2018) locus and the occurrence of restenosis and target vessel revascularization.
FIG. 9 is a graph showing the odds ratios for clinical events and angiographic restenosis associated with the presence of the IL-1RN*2 allele for the whole population (left panel) and patients <60 years (right panel)
FIG. 10, is a bar graph showing the decrease in the incidence of restenosis and target vessel revascularization (TVR) in patients <60 years with the increase in the number of IL-1RN*2 alleles.

### 4. Detailed Description of the Invention

### 4.1 Definitions

For convenience, the meaning of certain terms and phrases employed in the specification, examples, and appended claims are provided below.

The term "allele" refers to the different sequence variants found at different polymorphic regions. For example, IL-1RN (VNTR) has at least five different alleles. The sequence variants may be single or multiple base changes, including without limitation insertions, deletions, or substitutions, or may be a variable number of sequence repeats.

The term "allelic pattern" refers to the identity of an allele or alleles at one or more polymorphic regions. For example, an allelic pattern may consist of a single allele at a polymorphic site, as for IL-1RN (VNTR) allele 1, which is an allelic pattern having at least one copy of IL-1RN allele 1 at the VNTR of the IL-1RN gene loci. Alternatively, an allelic pattern may consist of either a homozygous or heterozygous state at a single polymorphic site. For example, IL1-RN (VNTR) allele 2,2 is an allelic pattern in which there are two copies of the second allele at the VNTR marker of IL-1RN and that corresponds to the homozygous IL-RN (VNTR) allele 2 state. Alternatively, an allelic pattern may consist of the identity of alleles at more than one polymorphic site.

The term "antibody " as used herein is intended to refer to a binding agent including a whole antibody or a binding fragment thereof which is specifically reactive with an IL-1B polypeptide. Antibodies can be fragmented using conventional techniques and the fragments screened for utility in the same manner as described above for whole antibodies. For example, F(ab)₂ fragments can be generated by treating an antibody with pepsin. The resulting F(ab)₂ fragment can be treated to reduce disulfide bridges to produce Fab fragments. The antibody is further intended to include bispecific, single-chain, and chimeric and humanized molecules having affinity for an IL-1B polypeptide conferred by at least one CDR region of the antibody.

"Biological activity" or "bioactivity" or "activity" or "biological function", which are used interchangeably, for the purposes herein means an effector or antigenic function that is directly or indirectly performed by an IL-1 polypeptide (whether in its native or denatured conformation), or by any subsequence thereof. Biological activities include binding to a target peptide, e.g., an IL-1 receptor. An IL-1 bioactivity can be modulated by directly affecting an IL-1 polypeptide. Alternatively, an IL-1 bioactivity can be modulated by modulating the level of an IL-1 polypeptide, such as by modulating expression of an IL-1 gene.

As used herein the term "bioactive fragment of an IL-1 polypeptide" refers to a fragment of a full-length IL-1 polypeptide, wherein the fragment specifically mimics or antagonizes the activity of a wild-type IL-1 polypeptide. The bioactive fragment preferably is a fragment capable of interacting with an interleukin receptor.

The term "an aberrant activity", as applied to an activity of a polypeptide such as IL-1, refers to an activity which differs from the activity of the wild-type or native polypeptide or which differs from the activity of the polypeptide in a healthy subject. An activity of a polypeptide can be aberrant because it is stronger than the activity of its native counterpart. Alternatively, an activity can be aberrant because it is weaker or absent relative to the activity of its native counterpart. An aberrant activity can also be a change in an activity. For example an aberrant polypeptide can interact with a different target peptide. A cell can have an aberrant IL-1 activity due to overexpression or underexpression of an IL-1 locus gene encoding an IL-1 locus polypeptide.

"Cells", "host cells" or "recombinant host cells" are terms used interchangeably herein to refer not only to the particular subject cell, but to the progeny or potential progeny of such a cell. Because certain modifications may occur in succeeding generations due to either mutation or environmental influences, such progeny may not, in fact be identical to the parent cell, but is still included within the scope of the term as used herein.

A "chimera," "mosaic," "chimeric mammal" and the like, refers to a transgenic mammal with a knock-out or knock-in construct in at least some of its genome-containing cells.

The terms "control" or "control sample" refer to any sample appropriate to the detection technique employed. The control sample may contain the products of the allele detection technique employed or the material to be tested. Further, the controls may be positive or negative controls. By way of example, where the allele detection technique is PCR amplification, followed by size fractionation, the control sample may comprise DNA fragments of an appropriate size. Likewise, where the allele detection technique involves detection of a mutated protein, the control sample may comprise a sample of a mutant protein. However, it is preferred that the control sample comprises the material to be tested. For example, the controls may be a sample of genomic DNA or a cloned portion of the IL-1 gene cluster. However, where the sample to be tested is genomic DNA, the control sample is preferably a highly purified sample of genomic DNA.

A "cardiovascular disease" is a cardiovascular disorder, as defined herein, characterized by clinical events including clinical symptoms and clinical signs. Clinical symptoms are those experiences reported by a patient that indicate to the clinician the presence of pathology. Clinical signs are those objective findings on physical or laboratory examination that indicate to the clinician the presence of pathology. "Cardiovascular disease" includes both "coronary artery disease" and "peripheral vascular disease," both terms being defined below. Clinical symptoms in cardiovascular disease include chest pain, shortness of breath, weakness, fainting spells, alterations in consciousness, extremity pain, paroxysmal nocturnal dyspnea, transient ischemic attacks and other such phenomena experienced by the patient. Clinical signs in cardiovascular disease include such findings as EKG abnormalities, altered peripheral pulses, arterial bruits, abnormal heart sounds, rales and wheezes, jugular venous distention, neurological alterations and other such findings discerned by the clinician. Clinical symptoms and clinical signs can combine in a cardiovascular disease such as a myocardial infarction (MI) or a stroke (also termed a "cerebrovascular accident" or "CVA"), where the patient will report certain phenomena (symptoms) and the clinician will perceive other phenomena (signs) all indicative of an underlying pathology. "Cardiovascular disease" includes those diseases related to the cardiovascular disorders of fragile plaque disorder, occlusive disorder and stenosis. For example, a cardiovascular disease resulting from a fragile plaque disorder, as that term is defined below, can be termed a "fragile plaque disease." Clinical events associated with fragile plaque disease include those signs and symptoms where the rupture of a fragile plaque with subsequent acute thrombosis or with distal embolization are hallmarks. Examples of fragile plaque disease include certain strokes and myocardial infarctions. As another example, a cardiovascular disease resulting from an occlusive disorder can be termed an "occlusive disease." Clinical events associated with occlusive disease include those signs and symptoms where the progressive occlusion of an artery affects the amount of circulation that reaches a target tissue. Progressive arterial occlusion may result in progressive ischemia that may ultimately progress to tissue death if the amount of circulation is insufficient to maintain the tissues. Signs and symptoms of occlusive disease include claudication, rest pain, angina, and gangrene, as well as physical and laboratory findings indicative of vessel stenosis and decreased distal perfusion. As yet another example, a cardiovascular disease resulting from restenosis can be termed an in-stent stenosis disease. In-stent stenosis disease includes the signs and symptoms resulting from the progressive blockage of an arterial stent that has been positioned as part of a procedure like a percutaneous transluminal angioplasty, where the presence of the stent is intended to help hold the vessel in its newly expanded configuration. The clinical events that accompany in-stent stenosis disease are those attributable to the restenosis of the reconstructed artery.

A "cardiovascular disorder" refers broadly to both to coronary artery disorders and peripheral arterial disorders. The term "cardiovascular disorder" can apply to any abnormality of an artery, whether structural, histological, biochemical or any other abnormality. This term includes those disorders characterized by fragile plaque (termed herein "fragile plaque disorders"), those disorders characterized by vaso-occlusion (termed herein "occlusive disorders"), and those disorders characterized by restenosis. A "cardiovascular disorder" can occur in an artery primarily, that is, prior to any medical or surgical intervention. Primary cardiovascular disorders include, among others, atherosclerosis, arterial occlusion, aneurysm formation and thrombosis. A "cardiovascular disorder" can occur in an artery secondarily, that is, following a medical or surgical intervention. Secondary cardiovascular disorders include, among others, post-traumatic aneurysm formation, restenosis, and post-operative graft occlusion.

A "cardiovascular disorder causative functional mutation" refers to a mutation which causes or contributes to the development of a cardiovascular disorder in a subject. Preferred mutations occur within the IL-1 complex. A cardiovascular disorder causative functional mutation occurring within an IL-1 gene (e.g. IL-1A, IL-1B or IL-1RN) or a gene locus, which is linked thereto, may alter, for example, the open reading frame or splicing pattern of the gene, thereby resulting in the formation of an inactive or hypoactive gene product. For example, a mutation which occurs in intron 6 of the IL-1A locus corresponds to a variable number of tandem repeat 46 bp sequences corresponding to from five to 18 repeat units (Bailly, et al. (1993) Eur. J. Immunol. 23: 1240-45). These repeat sequences contain three potential binding sites for transcriptional factors: an SP1 site, a viral enhancer element, and a glucocorticoid-responsive element; therefore individuals carrying IL-1A intron 6 VNTR alleles with large numbers of repeat units may be subject to altered transcriptional regulation of the IL-1A gene and consequent perturbations of inflammatory cytokine production. Indeed, there is evidence that increased repeat number at this polymorphic IL-1A locus leads to decreased IL-1α synthesis (Bailly et al. (1996) Mol Immunol 33: 999-1006). Alternatively, a mutation can result in a hyperactive gene product. For example, allele 2 of the IL-1B (G at +6912) polymorphism occurs in the 3' UTR (untranslated region) of the IL-1B mRNA and is associated with an approximately four-fold increase in the steady state levels of both IL-1B mRNA and IL-1B protein compared to those levels associated with allele 1 of the IL-1B gene at +6912). Further, an IL-1B (-511) mutation occurs near a promoter binding site for a negative glucocorticoid response element (Zhang et al. (1997) DNA Cell Biol 16: 145-52). This element potentiates a four-fold repression of IL-1B expression by dexamethosone and a deletion of this negative response elements causes a 2.5-fold increase in IL-1B promoter activity. The IL-1B (-511) polymorphism may thus directly affect cytokine production and inflammatory responses. These examples demonstrate that genetic variants occurring in the IL-1A or IL-1B gene can directly lead to the altered production or regulation of IL-1 cytokine activity.

A "cardiovascular disorder therapeutic" refers to any agent or therapeutic regimen (including pharmaceuticals, nutraceuticals and surgical means) that prevents or postpones the development of or reduces the extent of an abnormality constitutive of a cardiovascular disorder in a subject. Cardiovascular disorder therapeutics can be directed to the treatment of any cardiovascular disorder, including fragile plaque disorder, occlusive disorder and restenosis. Examples of therapeutic agents directed to each category of cardiovascular disorder are provided herein. It is understood that a therapeutic agent may be useful for more than one category of cardiovascular disorder. The therapeutic can be a polypeptide, peptidomimetic, nucleic acid or other inorganic or organic molecule, preferably a "small molecule" including vitamins, minerals and other nutrients. The therapeutic can modulate at least one activity of an IL-1 polypeptide, e.g., interaction with a receptor, by mimicking or potentiating (agonizing) or inhibiting (antagonizing) the effects of a naturally-occurring polypeptide. An IL-1 agonist can be a wild-type protein or derivative thereof having at least one bioactivity of the wild-type, e.g., receptor binding activity. An IL-1 agonist can also be a compound that upregulates expression of a gene or which increases at least one bioactivity of a protein. An IL-1 agonist can also be a compound which increases the interaction of a polypeptide with another molecule, e.g., a receptor. An IL-1 antagonist can be a compound which inhibits or decreases the interaction between a protein and another molecule, e.g., a receptor or an agent that blocks signal transduction or post-translation processing (e.g., IL-1 converting enzyme (ICE) inhibitor). Accordingly, a preferred antagonist is a compound which inhibits or decreases binding to a receptor and thereby blocks subsequent activation of the receptor. An IL-1 antagonist can also be a compound that downregulates expression of a gene or which reduces the amount of a protein present. The antagonist can be a dominant negative form of a polypeptide, e.g., a form of a polypeptide which is capable of interacting with a target peptide, e.g., a receptor, but which does not promote the activation of the receptor. The antagonist can also be a nucleic acid encoding a dominant negative form of a polypeptide, an antisense nucleic acid, or a ribozyme capable of interacting specifically with an RNA. Yet other antagonists are molecules which bind to a polypeptide and inhibit its action. Such molecules include peptides, e.g., forms of target peptides which do not have biological activity, and which inhibit binding to receptors. Thus, such peptides will bind to the active site of a protein and prevent it from interacting with target peptides. Yet other antagonists include antibodies that specifically interact with an epitope of a molecule, such that binding interferes with the biological function of the polypeptide. In yet another preferred embodiment, the antagonist is a small molecule, such as a molecule capable of inhibiting the interaction between a polypeptide and a target receptor. Alternatively, the small molecule can function as an antagonist by interacting with sites other than the receptor binding site. Preferred therapeutics include lipid lowering drugs, antiplatelet agents, anti-inflammatory agents and antihypertensive agents.

"Cerebrovascular disease," as used herein, is a type of peripheral vascular disease (as defined below) where the peripheral vessel blocked is part of the cerebral circulation. The cerebral circulation includes the carotid and the vertebral arterial systems. This definition of cerebrovascular disease is intended specifically to include intracranial hemorrhage that does not occur as a manifestation of an arterial blockage. Blockage can occur suddenly, by mechanisms such as plaque rupture or embolization. Blockage can occur progressively, with narrowing of the artery via myointimal hyperplasia and plaque formation. Blockage can be complete or partial. Certain degrees and durations of blockage result in cerebral ischemia, a reduction of blood flow that lasts for several seconds to minutes. The prolongation of cerebral ischemia can result in cerebral infarction. Ischemia and infarction can be focal or widespread. Cerebral ischemia or infarction can result in the abrupt onset of a non-convulsive focal neurological defect, a clinical event termed a "stroke" or a "cerebrovascular accident (CVA)". Cerebrovascular disease has two broad categories of pathologies: thrombosis and embolism. Thrombotic strokes occur without warning symptoms in 80-90% of patients; between 10 and 20% of thrombotic strokes are heralded by transient ischemic attacks. A cerebrovascular disease can be associated with a fragile plaque disorder. The signs and symptoms of this type of cerebrovascular disease are those associated with fragile plaque, including stroke due to sudden arterial blockage with thrombus or embolus formation. A cerebrovascular disease can be associated with occlusive disorder. The signs and symptoms of this type of cerebrovascular disease relate to progressive blockage of blood flow with global or local cerebral ischemia. In this setting, neurological changes can be seen, including stroke.

A "clinical event" is an occurrence of clinically discernible signs of a disease or of clinically reportable symptoms of a disease. "Clinically discernible" indicates that the sign can be appreciated by a health care provider. "Clinically reportable" indicates that the symptom is the type of phenomenon that can be described to a health care provider. A clinical event may comprise clinically reportable symptoms even if the particular patient cannot himself or herself report them, as long as these are the types of phenomena that are generally capable of description by a patient to a health care provider.

A "coronary artery disease" ("CAD") refers to a vascular disorder relating to the blockage of arteries serving the heart. Blockage can occur suddenly, by mechanisms such as plaque rupture or embolization. Blockage can occur progressively, with narrowing of the artery via myointimal hyperplasia and plaque formation. Those clinical signs and symptoms resulting from the blockage of arteries serving the heart are manifestations of coronary artery disease. Manifestations of coronary artery disease include angina, ischemia, myocardial infarction, cardiomyopathy, congestive heart failure, arrhythmias and aneurysm formation. It is understood that fragile plaque disease in the coronary circulation is associated with arterial thrombosis or distal embolization that manifests itself as a myocardial infarction. It is understood that occlusive disease in the coronary circulation is associated with arterial stenosis accompanied by anginal symptoms, a condition commonly treated with pharmacological interventions and with angioplasty.

A "disease" is a disorder characterized by clinical events including clinical signs and clinical symptoms. The diseases discussed herein include cardiovascular disease, peripheral vascular disease, CAD, cerebrovascular disease, and those diseases in any anatomic location associated with fragile plaque disorder, with occlusive disorder or with restenosis.

A "disorder associated allele" or "an allele associated with a disorder" refers to an allele whose presence in a subject indicates that the subject has or is susceptible to developing a particular disorder. One type of disorder associated allele is a "cardiovascular disorder associated allele," the presence of which in a subject indicates that the subject has or is susceptible to developing a cardiovascular disorder. These include broadly within their scope alleles which are associated with "fragile plaque disorders," alleles associated with "occlusive disorders," and alleles associated with restenosis. Examples of alleles associated with "fragile plaque disorders" include those alleles comprising the IL-1 pattern 1- i.e. allele 2 of the IL-1A +4825; allele 2 of the +3954 marker of IL-1B; and allele 1 of the +2018 marker of IL-1RN; and allele 1 of the (-511) marker of the IL-1B gene or an allele that is in linkage disequilibrium with one of the aforementioned alleles. Examples of alleles associated with "occlusive disorders" include those comprising the IL-1 pattern 2 - i.e. allele 1 of the IL-1A +4825; allele 1 of the +3954 marker of IL-1B; and allele 2 of the +2018 marker of IL-1RN; and allele 2 of the (-511) marker of the IL-1B gene or an allele that is in linkage disequilibrium with one of the aforementioned alleles. Examples of alleles associated with restenosis include the combination of either allele 1 of the +4825 marker of IL-1A or allele 1 of the +3954 marker as combined with either allele 1 of the -511 marker of IL-1B or allele 1 of the +2018 marker of IL-1RN, or an allele that is in linkage disequilibrium with one of the aforementioned alleles. A "periodontal disorder associated allele" refers to an allele whose presence in a subject indicates that the subject has or is susceptible to developing a periodontal disorders.

The phrases "disruption of the gene" and "targeted disruption" or any similar phrase refers to the site specific interruption of a native DNA sequence so as to prevent expression of that gene in the cell as compared to the wild-type copy of the gene. The interruption may be caused by deletions, insertions or modifications to the gene, or any combination thereof.

The term "haplotype" as used herein is intended to refer to a set of alleles that are inherited together as a group (are in linkage disequilibrium) at statistically significant levels (p_{corr} < 0.05). As used herein, the phrase "an IL-1 haplotype" refers to a haplotype in the IL-1 loci.

The term "hyperplasia" as used herein is intended to refer to an abnormal or unusual increase in growth or division of the cells composing a tissue or organ. It is understood that the term "hyperplasia," as used herein, encompasses a wide variety of specific proliferative states including "neointimal hyperplasia" or "neointimal growth," which refers to hyperplasia of the of cells in the endothelial layer of a blood vessel and "myointimal hyperplasia" or "myointimal growth," which refers to an abnormal proliferation of smooth muscle cells of the vascular wall. The terms myointimal and neointimal are used interchangeably herein.

An "IL-1 agonist" as used herein refers to an agent that mimics, upregulates (potentiates or supplements) or otherwise increases an IL-1 bioactivity or a bioactivity of a gene in an IL-1 biological pathway. IL-1 agonists may act on any of a variety of different levels, including regulation of IL-1 gene expression at the promoter region, regulation of mRNA splicing mechanisms, stabilization of mRNA, phosphorylation of proteins for translation, conversion of proIL-1 to mature IL-1 and secretion of IL-1. Agonists that increase IL-1 synthesis include: lipopolysaccharides, IL-1B, cAMP inducing agents, NfκB activating agents, AP-1 activating agents, TNF-α, oxidized LDL, advanced glycosylation end products (AGE), sheer stress, hypoxia, hyperoxia, ischemia reperfusion injury, histamine, prostaglandin E 2 (PGE2), IL-2, IL-3, IL-12, granulocyte macrophage-colony stimulating factor (GM-CSF), monocyte colony stimulating factor (M-CSF), stem cell factor, platelet derived growth factor (PDGF), complement C5A, complement C5b9, fibrin degradation products, plasmin, thrombin, 9-hydroxyoctadecaenoic acid, 13-hydroxyoctadecaenoic acid, platelet activating factor (PAF), factor H, retinoic acid, uric acid, calcium pyrophosphate, polynucleosides, c-reactive protein, α-antitrypsin, tobacco antigen, collagen, β-1 integrins, LFA-3, anti-HLA-DR, anti-IgM, anti- CD3, phytohemagglutinin (CD2), sCD23, ultraviolet B radiation, gamma radiation, substance P,. isoproterenol, methamphetamine and melatonin. Agonists that stabilize IL-1 mRNA include bacterial endotoxin and IL-1. Other agonists, that function by increasing the number of IL-1 type 1 receptors available, include IL-1, PKC activators, dexamethasone, IL-2, IL-4 and PGE2. Other preferred antagonists interfere or inhibit signal transduction factors activated by IL-1 or utilized in an IL-1 signal transduction pathway (e.g NFκB and AP-1, PI3 kinase, phospholipase A2, protein kinase C, JNK-1, 5-lipoxygenase, cyclooxygenase 2, tyrosine phosphorylation, iNOS pathway, Rac, Ras, TRAF). Still other agonists increase the bioactivity of genes whose expression is induced by IL-1, including: IL-1, IL-1Ra, TNF, IL-2, IL-3, IL-6, IL-12, GM-CSF, G-CSF, TGF-β, fibrinogen, urokinase plasminogen inhibitor, Type 1 and type 2 plasminogen activator inhibitor, p-selectin (CD62), fibrinogen receptor, CD-11/CD18, protease nexin-1, CD44, Matrix metalloproteinase-1 (MMP-1),MMP-3, Elastase, Collagenases, Tissue inhibitor of metalloproteinases-1 (TIMP-1),Collagen, Triglyceride increasing Apo CIII, Apolipoprotein, ICAM-1, ELAM-1, VCAM-1, L-selectin, Decorin, stem cell factor, Leukemia inhibiting factor, IFNα,β,γ, L-8, IL-2 receptor, IL-3 receptor, IL-5 receptor, *c-kit* receptor, GM-CSF receptor, Cyclooxygenase-2 (COX-2), Type 2 phospholipase A2, Inducible nitric oxide synthase (iNOS), Endothelin-1,3, Gamma glutamyl transferase, Mn superoxide dismutase, C- reactive protein, Fibrinogen, Serum amyloid A, Metallothioneins, Ceruloplasmin, Lysozyme, Xanthine dehydrogenase, Xanthine oxidase, Platelet derived growth factor A chain (PDGF), Melanoma growth stimulatory activity (*gro*-α,β,γ), Insulin-like growth factor-1 (IGF-1), Activin A, Pro-opiomelanocortiotropin, corticotropin releasing factor, B amyloid precursor, Basement membrane protein-40, Laminin B1 and B2, Constitutive heat shock protein p70, P42 mitogen, activating protein kinase, ornithine decarboxylase, heme oxygenase and G-protein α subunit).

An "IL-1 antagonist" as used herein refers to an agent that downregulates or otherwise decreases an IL-1 bioactivity. IL-1 antagonists may act on any of a variety of different levels, including regulation of IL-1 gene expression at the promoter region, regulation of mRNA splicing mechanisms, stabilization of mRNA, phosphorylation of proteins for translation, conversion of proIL-1 to mature IL-1 and secretion of IL-1. Antagonists of IL-1production include: corticosteroids, lipoxygenase inhibitors, cyclooxygenase inhibitors, γ-interferon, IL-4, IL-10, IL-13, transforming growth factor β (TGF-β), ACE inhibitors, n-3 polyunsaturated fatty acids, antioxidants and lipid reducing agents. Antagonists that destabilize IL-1mRNA include agents that promote deadenylation. Antagonists that inhibit or prevent phosphorylation of IL-1 proteins for translation include pyridinyl-imadazole compounds, such as tebufelone and compounds that inhibit microtubule formation (e.g. colchicine, vinblastine and vincristine). Antagonists that inhibit or prevent the conversion of proIL-1 to mature IL-1 include interleukin converting enzyme (ICE) inhibitors, such as εICE isoforms, ICE α, β, and γ isoform antibodies, CXrm-A, transcript X, endogenous tetrapeptide competitive substrate inhibitor, trypsin, elastase, chymotrypsin, chymase, and other nonspecific proteases. Antagonists that prevent or inhibit the scretion of IL-1 include agents that block anion transport. Antagonists that interefere with IL-1 receptor interactions, include: agents that inhibit glycosylation of the type I IL-1 receptor, antisense oligonucleotides against IL-1RI, antibodies to IL-1RI and antisense oligonucleotides against IL-1RacP. Other antagonists, that function by decreasing the number of IL-1 type 1 receptors available, include TGF-β, COX inhibitors, factors that increase IL-1 type II receptors, dexamethasone, PGE2, IL-1 and IL-4. Other preferred antagonists interfere or inhibit signal transduction factors activated by IL-1 or utilized in an IL-1 signal transduction pathway (e.g NFκB and AP-1, PI3 kinase, phospholipase A2, protein kinase C, JNK-1, 5-lipoxygenase, cyclooxygenase 2, tyrosine phosphorylation, iNOS pathway, Rac, Ras, TRAF). Still other antagonists interfere with the bioactivity of genes whose expression is induced by IL-1, including: IL-1, IL-1Ra, TNF, IL-2, IL-3, IL-6, IL-12, GM-CSF, G-CSF, TGF-β, fibrinogen, urokinase plasminogen inhibitor, Type 1 and type 2 plasminogen activator inhibitor, p-selectin (CD62), fibrinogen receptor, CD-11/CD18, protease nexin-1, CD44, Matrix metalloproteinase-1 (MMP-1),MMP-3, Elastase, Collagenases, Tissue inhibitor of metalloproteinases-1 (TIMP-1),Collagen, Triglyceride increasing Apo CIII, Apolipoprotein, ICAM-1, ELAM-1, VCAM-1, L-selectin, Decorin, stem cell factor, Leukemia inhibiting factor, IFNα,β,γ, L-8, IL-2 receptor, IL-3 receptor, IL-5 receptor, *c-kit* receptor, GM-CSF receptor, Cyclooxygenase-2 (COX-2), Type 2 phospholipase A2, Inducible nitric oxide synthase (iNOS), Endothelin-1,3, Gamma glutamyl transferase, Mn superoxide dismutase, C- reactive protein, Fibrinogen, Serum amyloid A, Metallothioneins, Ceruloplasmin, Lysozyme, Xanthine dehydrogenase, Xanthine oxidase, Platelet derived growth factor A chain (PDGF), Melanoma growth stimulatory activity (*gro*-α,β,γ), Insulin-like growth factor-1 (IGF-1), Activin A, Pro-opiomelanocortiotropin, corticotropin releasing factor, B amyloid precursor, Basement membrane protein-40, Laminin B1 and B2, Constitutive heat shock protein p70, P42 mitogen, activating protein kinase, ornithine decarboxylase, heme oxygenase and G-protein α subunit). Other preferred antagonists include: hymenialdisine, herbimycines (e.g. herbamycin A), CK-103A and its derivatives (e.g. 4,6-dihydropyridazino[4,5-c]pyridazin-5 (1H)-one), CK-119, CK-122, iodomethacin, aflatoxin B1, leptin, heparin, bicyclic imidazoles (e.g SB203580), PD15306 HCl, podocarpic acid derivatives, M-20, Human [Gly2] Glucagon-like peptide-2, FR167653, Steroid derivatives, glucocorticoids, Quercetin, Theophylline, NO-synthetase inhibitors, RWJ 68354, Euclyptol (1.8-cineole), Magnosalin, N-Acetylcysteine, Alpha-Melatonin-Stimulating Hormone (α-MSH), Triclosan (2,4,4'-trichloro-2'-hydroxyldiphenyl ether), Prostaglandin E2 and 4-aminopyridine Ethacrynic acid and 4,4'-diisothiocyanatostilbene-2,2'-disulfonic acid (DIDS), Glucose, Lipophosphoglycan, aspirin, Catabolism-blocking agents, Diacerhein, Thiol-modulating agents, Zinc, Morphine, Leukotriene biosynthesis inhibitors (e.g. MK886), Platelet-activating factor receptor antagonists (e.g. WEB 2086), Amiodarone, Tranilast, S-methyl-L-thiocitrulline, Beta-adrenoreceptor agonists (e.g.Procaterol, Clenbuterol, Fenoterol, Terbutaline, Hyaluronic acid, anti-TNF-α antibodies, anti-IL-1α autoantibodies, IL-1 receptor antagonist, IL-1R-associated kinase, soluble TNF receptors and antiinflammatory cytokines (e.g IL-4, IL-13, IL-10, IL-6, TGF-β, angiotensin II, Soluble IL-1 type II receptor, Soluble IL-1 type I receptor, Tissue plasminogen activator, Zinc finger protein A20 IL-1 Peptides (e.g (Thr-Lys-Pro-Arg) (Tuftsin), (Ile-Thr-Gly-Ser-Glu) IL-1-alpha, Val-Thr-Lys-Phe-Tyr-Phe, Val-Thr-Asp-Phe-Tyr-Phe, Interferon alpha2b, Interferon beta, IL-1-beta analogues (e.g. IL-1-beta tripeptide: Lys-D-Pro-Thr), glycosylated IL-1-alpha, and IL-1ra peptides.

The terms "IL-1 gene cluster" and "IL-1 loci" as used herein include all the nucleic acid at or near the 2q13 region of chromosome 2, including at least the IL-1A, IL-1B and IL-1RN genes and any other linked sequences. (Nicklin et al., Genomics 19: 382-84, 1994). The terms "IL-1A", "IL-1B", and "IL-1RN" as used herein refer to the genes coding for IL-1, IL-1, and IL-1 receptor antagonist, respectively. The gene accession number for IL-1A, IL-1B, and IL-1RN are X03833, X04500, and X64532, respectively.

"IL-1 functional mutation" refers to a mutation within the IL-1 gene cluster that results in an altered phenotype (i.e. effects the function of an IL-1 gene or protein). Examples include: IL-1A(+4845) allele 2, IL-1B (+3954) allele 2, IL-1B (+6912) allele 2 and IL-1RN (+2018) allele 2.

"IL-1X (Z) allele Y " refers to a particular allelic form, designated Y, occurring at an IL-1 locus polymorphic site in gene X, wherein X is IL-1A, B, or RN or some other gene in the IL-1 gene loci, and positioned at or near nucleotide Z, wherein nucleotide Z is numbered relative to the major transcriptional start site, which is nucleotide +1, of the particular IL-1 gene X. As further used herein, the term "IL-1X allele (Z)" refers to all alleles of an IL-1 polymorphic site in gene X positioned at or near nucleotide Z. For example, the term "IL-1RN (+2018) allele" refers to alternative forms of the IL-1RN gene at marker+2018. "IL-1RN (+2018) allele 1" refers to a form of the IL-1RN gene which contains a cytosine (C) at position +2018 of the sense strand. Clay et al., Hum. Genet. 97:723-26, 1996. "IL-1RN (+2018) allele 2" refers to a form of the IL-1RN gene which contains a thymine (T) at position +2018 of the plus strand. When a subject has two identical IL-1RN alleles, the subject is said to be homozygous, or to have the homozygous state. When a subject has two different IL-1RN alleles, the subject is said to be heterozygous, or to have the heterozygous state. The term "IL-1RN (+2018) allele 2,2" refers to the homozygous IL-1 RN (+2018) allele 2 state. Conversely, the term "IL-1RN (+2018) allele 1,1" refers to the homozygous IL-1 RN (+2018) allele 1 state. The term "IL-1RN (+2018) allele 1,2" refers to the heterozygous allele 1 and 2 state.

"IL-1 related" as used herein is meant to include all genes related to the human IL-1 locus genes on human chromosome 2 (2q 12-14). These include IL-1 genes of the human IL-1 gene cluster located at chromosome 2 (2q 13-14) which include: the IL-1A gene which encodes interleukin-1α, the IL-1B gene which encodes interleukin-1β, and the IL-1RN (or IL-1ra) gene which encodes the interleukin-1 receptor antagonist. Furthermore these IL-1 related genes include the type I and type II human IL-1 receptor genes located on human chromosome 2 (2q12) and their mouse homologs located on mouse chromosome 1 at position 19.5 cM. Interleukin-1α, interleukin-1β, and interleukin-1RN are related in so much as they all bind to IL-1 type I receptors, however only interleukin-1α and interleukin-1β are agonist ligands which activate IL-1 type I receptors, while interleukin-1RN is a naturally occurring antagonist ligand. Where the term "IL-1" is used in reference to a gene product or polypeptide, it is meant to refer to all gene products encoded by the interleukin-1 locus on human chromosome 2 (2q 12-14) and their corresponding homologs from other species or functional variants thereof. The term IL-1 thus includes secreted polypeptides which promote an inflammatory response, such as IL-1α and IL-1β, as well as a secreted polypeptide which antagonize inflammatory responses, such as IL-1 receptor antagonist and the IL-1 type II (decoy) receptor.

An "IL-1 receptor" or "IL-1R" refers to various cell membrane bound protein receptors capable of binding to and/or transducing a signal from IL-1 locus-encoded ligand. The term applies to any of the proteins which are capable of binding interleukin-1 (IL-1) molecules and, in their native configuration as mammalian plasma membrane proteins, presumably play a role in transducing the signal provided by IL-1 to a cell. As used herein, the term includes analogs of native proteins with IL-1-binding or signal transducing activity. Examples include the human and murine IL-1 receptors described in U.S. Patent No. 4,968,607. The term "IL-1 nucleic acid" refers to a nucleic acid encoding an IL-1 protein.

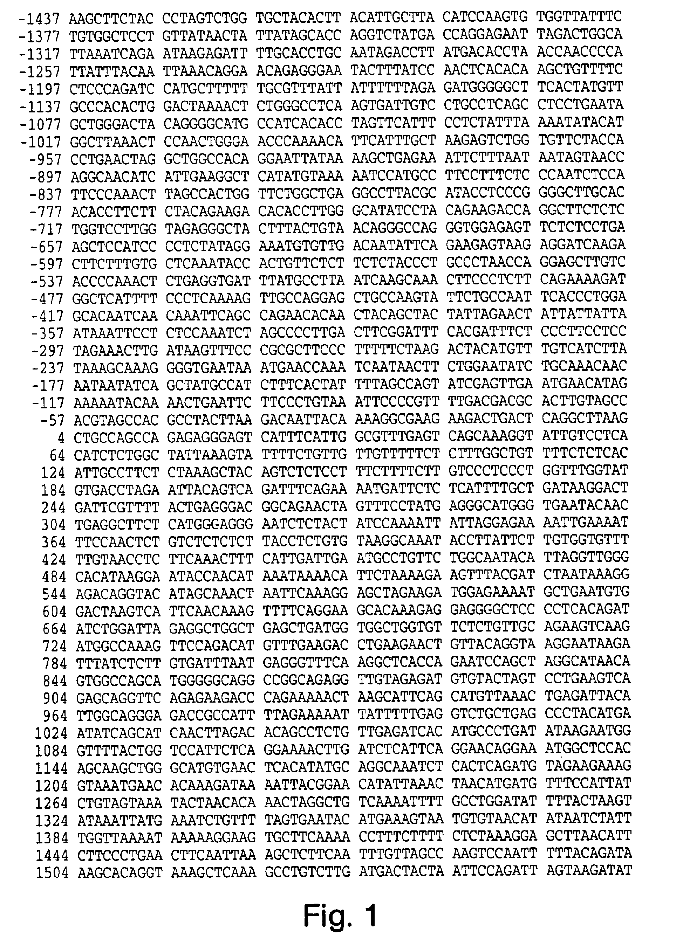

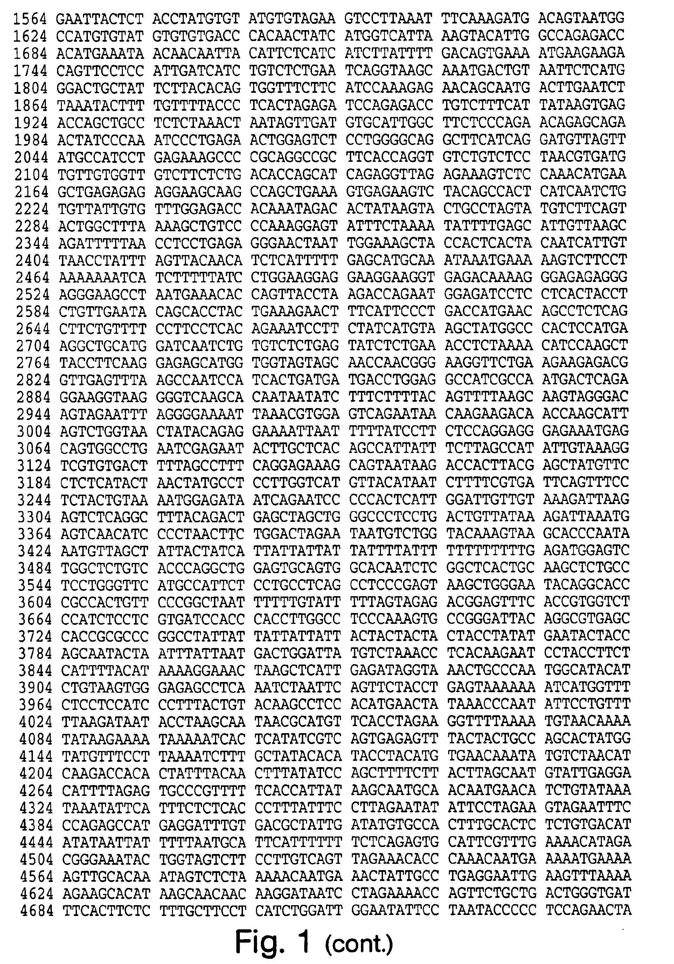

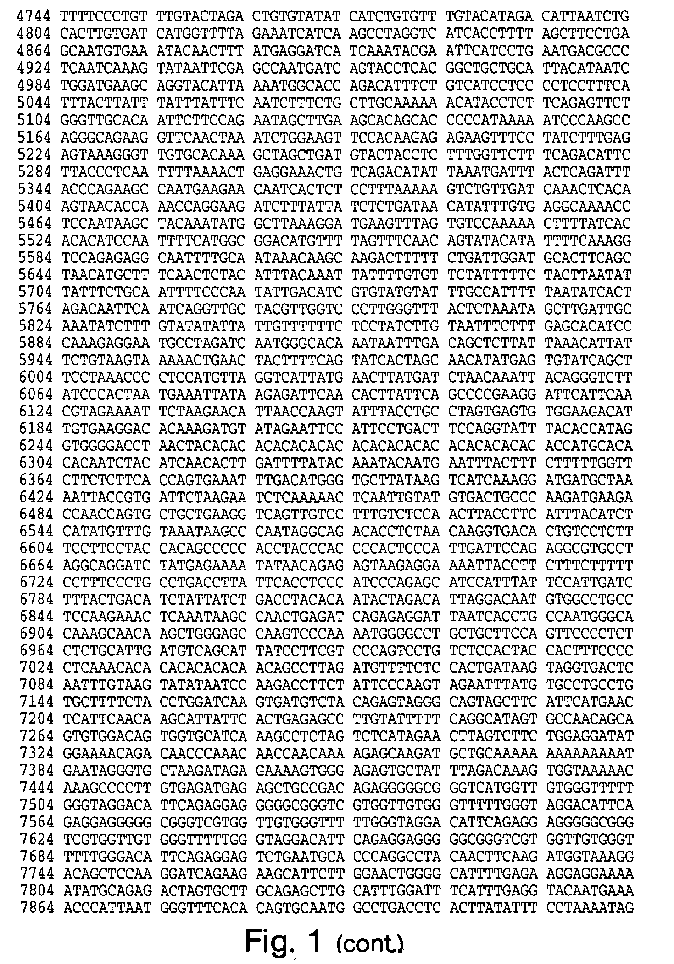

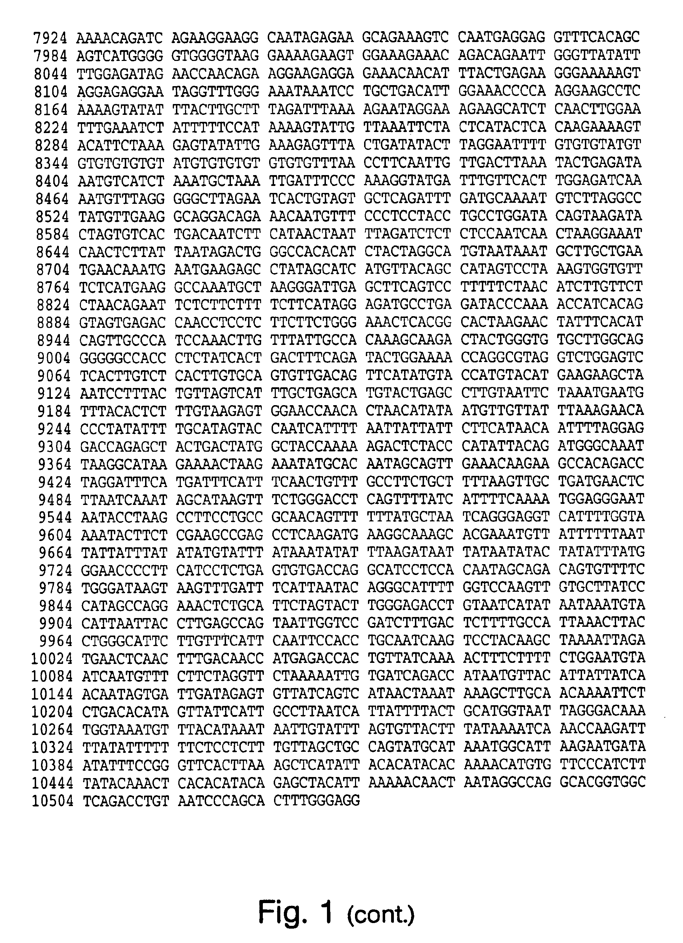

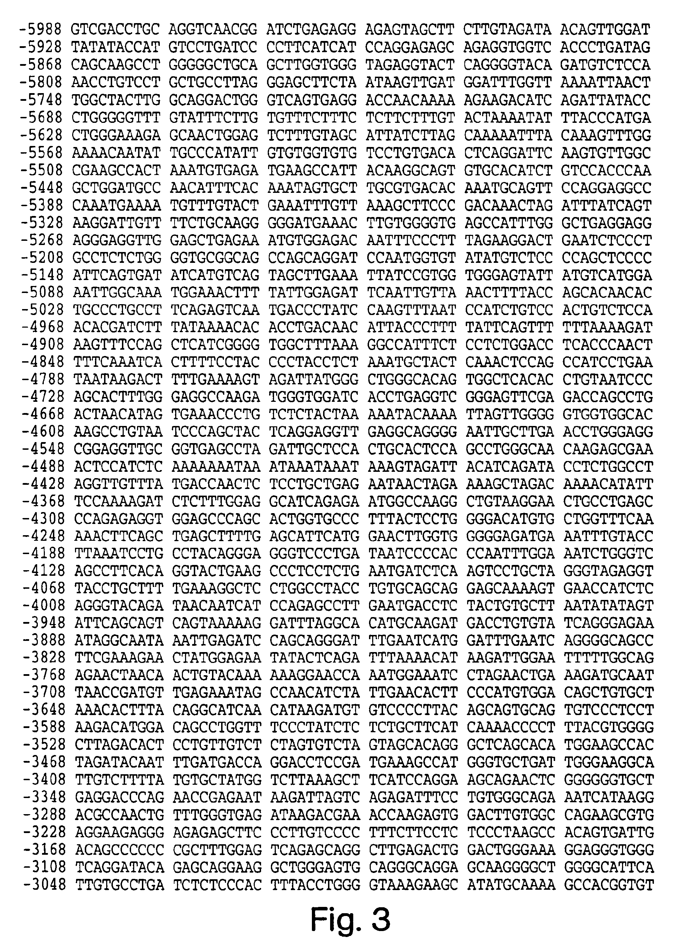

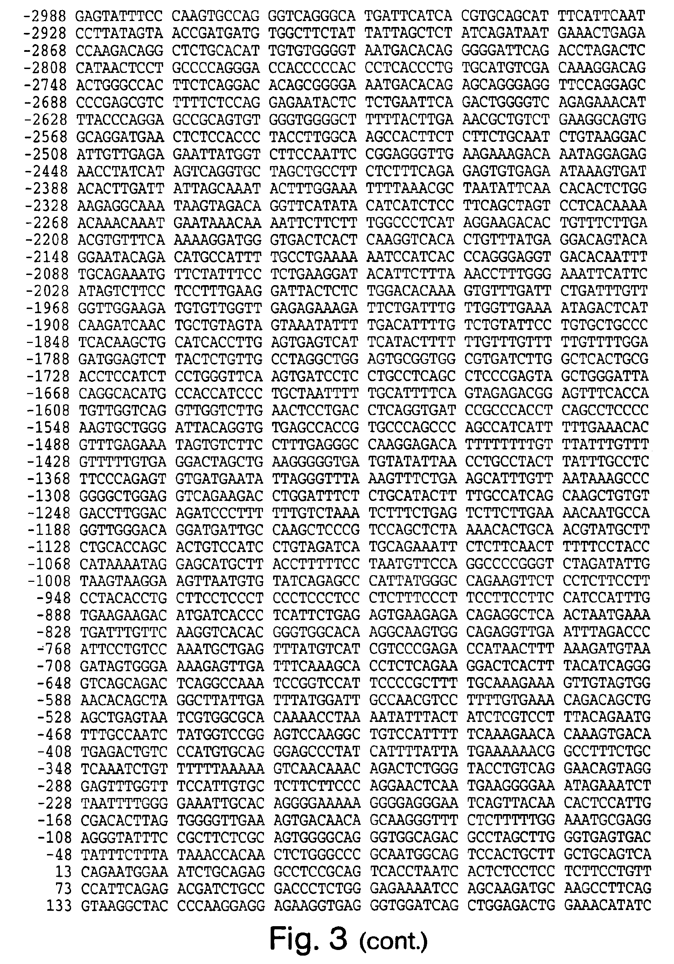

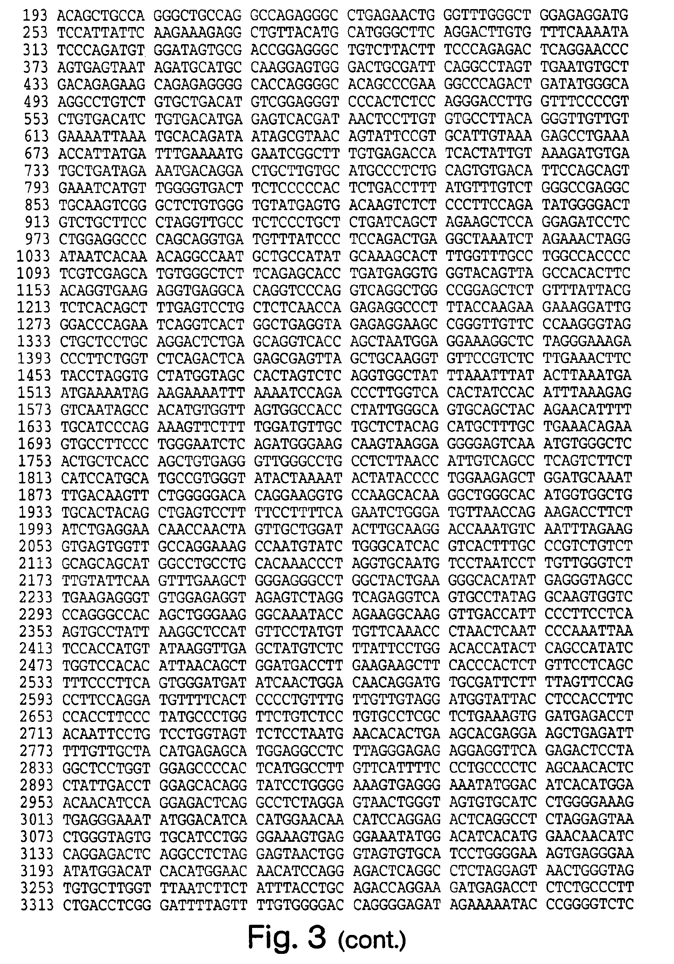

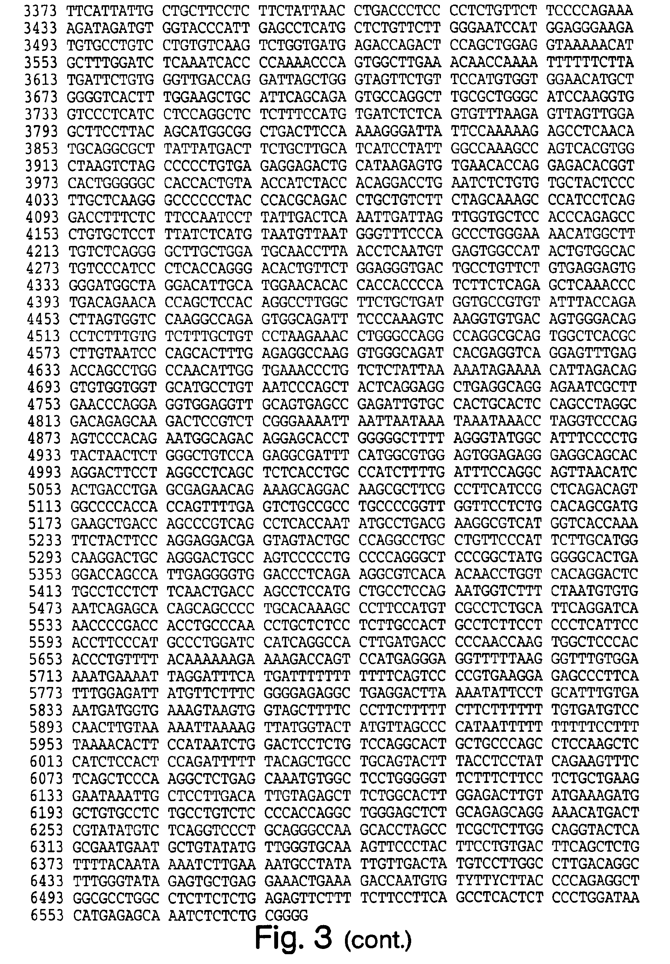

An "IL-1 polypeptide" and "IL-1 protein" are intended to encompass polypeptides comprising the amino acid sequence encoded by the IL-1 genomic DNA sequences shown in Figures 1, 2, and 3, or fragments thereof, and homologs thereof and include agonist and antagonist polypeptides.

"In-stent stenosis" refers to the progressive occlusion within a stent that has been placed during angioplasty. In-stent stenosis is a form of restenosis that takes place within an arterial stent.

"Increased risk" refers to a statistically higher frequency of occurrence of the disease or condition in an individual carrying a particular polymorphic allele in comparison to the frequency of occurrence of the disease or condition in a member of a population that does not carry the particular polymorphic allele.

The term "interact" as used herein is meant to include detectable relationships or associations (e.g. biochemical interactions) between molecules, such as interactions between protein-protein, protein-nucleic acid, nucleic acid-nucleic acid and protein-small molecule or nucleic acid-small molecule in nature.

The term "isolated" as used herein with respect to nucleic acids, such as DNA or RNA, refers to molecules separated from other DNAs, or RNAs, respectively, that are present in the natural source of the macromolecule. For example, an isolated nucleic acid encoding one of the subject IL-1 polypeptides preferably includes no more than 10 kilobases (kb) of nucleic acid sequence which naturally immediately flanks the IL-1 gene in genomic DNA, more preferably no more than 5kb of such naturally occurring flanking sequences, and most preferably less than 1.5kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

A "knock-in" transgenic animal refers to an animal that has had a modified gene introduced into its genome and the modified gene can be of exogenous or endogenous origin.

A "knock-out" transgenic animal refers to an animal in which there is partial or complete suppression of the expression of an endogenous gene (e.g, based on deletion of at least a portion of the gene, replacement of at least a portion of the gene with a second sequence, introduction of stop codons, the mutation of bases encoding critical amino acids, or the removal of an intron junction, etc.).

A "knock-out construct" refers to a nucleic acid sequence that can be used to decrease or suppress expression of a protein encoded by endogenous DNA sequences in a cell. In a simple example, the knock-out construct is comprised of a gene, such as the IL-1RN gene, with a deletion in a critical portion of the gene so that active protein cannot be expressed therefrom. Alternatively, a number of termination codons can be added to the native gene to cause early termination of the protein or an intron junction can be inactivated. In a typical knock-out construct, some portion of the gene is replaced with a selectable marker (such as the neo gene) so that the gene can be represented as follows: IL-1RN 5'/neo/ IL-1RN 3', where IL-1RN5' and IL-1RN 3', refer to genomic or cDNA sequences which are, respectively, upstream and downstream relative to a portion of the IL-1RN gene and where neo refers to a neomycin resistance gene. In another knock-out construct, a second selectable marker is added in a flanking position so that the gene can be represented as: IL-1RN/neo/IL-1RN/TK, where TK is a thymidine kinase gene which can be added to either the IL-1RN5' or the IL-1RN3' sequence of the preceding construct and which further can be selected against (i.e. is a negative selectable marker) in appropriate media. This two-marker construct allows the selection of homologous recombination events, which removes the flanking TK marker, from non-homologous recombination events which typically retain the TK sequences. The gene deletion and/or replacement can be from the exons, introns, especially intron junctions, and/or the regulatory regions such as promoters.

"Linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage equilibrium". The cause of linkage disequilibrium is often unclear. It can be due to selection for certain allele combinations or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in the specific chromosomal region. When referring to allelic patterns that are comprised of more than one allele, a first allelic pattern is in linkage disequilibrium with a second allelic pattern if all the alleles that comprise the first allelic pattern are in linkage disequilibrium with at least one of the alleles of the second allelic pattern. An example of linkage disequilibrium is that which occurs between the alleles at the IL-1RN (+2018) and IL-1RN (VNTR) polymorphic sites. The two alleles at IL-1RN (+2018) are 100% in linkage disequilibrium with the two most frequent alleles of IL-1RN (VNTR), which are allele 1 and allele 2.

The term "marker" refers to a sequence in the genome that is known to vary among individuals. For example, the IL-1RN gene has a marker that consists of a variable number of tandem repeats (VNTR).

"Modulate" refers to the ability of a substance to regulate bioactivity. When applied to an IL-1 bioactivity, an agonist or antagonist can modulate bioactivity for example by agonizing or antagonizing an IL-1 synthesis, receptor interaction, or IL-1 mediated signal transduction mechanism.

A "mutated gene" or "mutation" or "functional mutation" refers to an allelic form of a gene, which is capable of altering the phenotype of a subject having the mutated gene relative to a subject which does not have the mutated gene. The altered phenotype caused by a mutation can be corrected or compensated for by certain agents. If a subject must be homozygous for this mutation to have an altered phenotype, the mutation is said to be recessive. If one copy of the mutated gene is sufficient to alter the phenotype of the subject, the mutation is said to be dominant. If a subject has one copy of the mutated gene and has a phenotype that is intermediate between that of a homozygous and that of a heterozygous subject (for that gene), the mutation is said to be co-dominant.

A "non-human animal" of the invention includes mammals such as rodents, non-human primates, sheep, dogs, cows, goats, etc. Preferred non-human animals are selected from the rodent family including rat and mouse, most preferably mouse, though transgenic amphibians, such as members of the *Xenopus* genus, and transgenic chickens can also provide important tools for understanding and identifying agents which can affect, for example, embryogenesis and tissue formation. The term "chimeric animal" is used herein to refer to animals in which the recombinant gene is found, or in which the recombinant gene is expressed in some but not all cells of the animal. The term "tissue-specific chimeric animal" indicates that one of the recombinant IL-1 genes is present and/or expressed or disrupted in some tissues but not others. The term "non-human mammal" refers to any members of the class Mammalia, except for humans:

As used herein, the term "nucleic acid" refers to polynucleotides or oligonucleotides such as deoxyribonucleic acid (DNA), and, where appropriate, ribonucleic acid (RNA). The term should also be understood to include, as equivalents, analogs of either RNA or DNA made from nucleotide analogs (e.g. peptide nucleic acids) and as applicable to the embodiment being described, single (sense or antisense) and double-stranded polynucleotides.

"Occlusive disorder" refers to that cardiovascular disorder characterized by the progressive thickening of an arterial wall, associated with the presence of an atherosclerotic intimal lesion within an artery. Occlusive disorder leads to progressive blockage of the artery. With sufficient progression, the occlusive disorder can reduce flow in the artery to the point that clinical signs and symptoms are produced in the tissues perfused by the artery. These clinical events relate to ischemia of the perfused tissues. When severe, ischemia is accompanied by tissue death, called infarction or gangrene. Occlusive disorder is associated with the allele pattern 2s at the IL-1 locus.
An "occlusive disorder therapeutic" refers to any agent or therapeutic regimen (including pharmaceuticals, nutraceuticals and surgical means) that prevents or postpones the development of or reduces the extent of an abnormality constitutive of an occlusive disorder in a subject. Examples of occlusive disorder therapeutics include those agents that are anti-oxidants, those that lower serum lipids, those that block the action of oxidized lipids and other agents that influence lipid metabolism or otherwise have lipid-active effects.

A "peripheral vascular disease" ("PVD") is a cardiovascular disease resulting from the blockage of the peripheral (i.e., non-coronary) arteries. Blockage can occur suddenly, by mechanisms such as plaque rupture or embolization, as occurs in fragile plaque disease. Blockage can occur progressively, with narrowing of the artery via myointimal hyperplasia and plaque formation, as in occlusive disease. Blockage can be complete or partial. Those clinical signs and symptoms resulting from the blockage of peripheral arteries are manifestations of peripheral vascular disease. Manifestations of peripheral vascular diseases include, inter alia, claudication, ischemia, intestinal angina, vascular-based renal insufficiency, transient ischemic attacks, aneurysm formation, peripheral embolization and stroke. Ischemic cerebrovascular disease is a type of peripheral vascular disease. The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A specific genetic sequence at a polymorphic region of a gene is an allele. A polymorphic region can be a single nucleotide, the identity of which differs in different alleles. A polymorphic region can also be several nucleotides long.

The term "propensity to disease," also "predisposition" or "susceptibility" to disease or any similar phrase, means that certain alleles are hereby discovered to be associated with or predictive of ILD. The alleles are thus over-represented in frequency in individuals with disease as compared to healthy individuals. Thus, these alleles can be used to predict disease even in pre-symptomatic or pre-diseased individuals.

The term "restenosis" refers to any preocclusive lesion that develops following a reconstructive procedure in a diseased blood vessel. The term is not only applied to the recurrence of a pre-existing stenosis, but also to previously normal vessels such as vein grafts that become partially occluded following vascular bypass. Restenosis refers to any luminal narrowing that occurs following an injury to the vessel wall. Injuries resulting in restenosis can therefore include trauma to an atherosclerotic lesion (as seen with angioplasty), a resection of a lesion (as seen with endarterectomy), an external trauma (e.g., a cross-clamping injury), or a surgical anastomosis. Restenosis typically results from a hyperplasia.

Restenosis can occur as the result of any kind of vascular reconstruction, whether in the coronary vasculature or in the periphery (Colbum and Moore (1998) Myointimal Hyperplasia pp. 690-709 in Vascular Surgery: A Comprehensive Review (Philadelphia: Saunders, 1998)). For example, studies have reported symptomatic restenosis rates of 30-50% following coronary angioplasties (see Berk and Harris (1995) Adv. Intern. Med. 40:455-501). After carotid endarterectomies, as a further example, 20% of patients studied had a luminal narrowing greater than 50% (Clagett et al. (1986) J. Vasc. Surg. 3:10-23). Yet another example ofrestenosis is seen in infrainguinal vascular bypasses, where 40-60% of prosthetic grafts and 20-40% of the vein grafts are occluded at three years (Dalman and Taylor (1990) Ann. Vasc. Surg. 3:109-312, Szilagyi et al. (1973) Ann. Surg. 178:232-246). Different degrees of symptomatology accompany preocclusive lesions in different anatomical locations, due to a combination of factors including the different calibers of the vessels involved, the extent of residual disease and local hemodynamics.

A "restenosis associated allele" refers to an allele whose presence in a subject indicates that the subject has or is susceptible to developing a restenosis. Examples of restenosis associated alleles include allele 1 of the +4845 marker of IL-1A; allele 1 of the +3954 marker of IL-1B; allele 1 of the -511 marker of IL-1B; and allele 1 of the +2018 marker of IL-1RN. Still other linked polymorphic loci associated with restenosis include: the IL-1RN(VNTR) polymorphism, the IL-1RN gene +1731 polymorphism; the IL-1RN gene +1812 polymorphism; the IL-1RN gene + 1868 polymorphism; the IL-1RN gene +1887 polymorphism; the IL-1RN +8006 polymorphism, the IL-1RN +8061 polymorphism, the IL-1B -31 polymorphism and the IL-1B -511 polymorphism. Other restenosis associated alleles that have been described in the art include certain alleles in angiotensin converting enzymes (See e.g. Kasi et al., (1996) Am. J. Cardiol. 77: 875-77).

A "restenosis causative functional mutation" refers to a mutation which causes or contributes to the development of restenosis in a subject. Preferred mutations occur within the IL-1 complex. A restenosis causative functional mutation occurring within an IL-1 gene (e.g. IL-IA, IL-1B or IL-1RN) or a gene locus, which is linked thereto, may alter, for example, the open reading frame or splicing pattern of the gene, thereby resulting in the formation of an inactive or hypoactive gene product. For example, a mutation which occurs in intron 6 of the IL-1A locus corresponds to a variable number of tandem repeat 46 bp sequences corresponding to from five to 18 repeat units (Bailly, et al. (1993) Eur. J. Immunol. 23: 1240-45). These repeat sequences contain three potential binding sites for transcriptional factors: an SP1 site, a viral enhancer element, and a glucocorticoid-responsive element; therefore individuals carrying IL-1A intron 6 VNTR alleles with large numbers of repeat units may be subject to altered transcriptional regulation of the IL-1A gene and consequent perturbations of inflammatory cytokine production. Indeed, there is evidence that increased repeat number at this polymorphic IL-1A locus leads to decreased IL-1α synthesis (Bailly et al. (1996) Mol Immunol 33: 999-1006). Alternatively, a mutation can result in a hyperactive gene product. For example, allele 2 of the IL-1B (G at +6912) polymorphism occurs in the 3' UTR (untranslated region) of the IL-1B mRNA and is associated with an approximately four-fold increase in the steady state levels of both IL-1B mRNA and IL-1B protein compared to those levels associated with allele 1 of the IL-1B gene © at +6912). Further, an IL-1B (-511) mutation occurs near a promoter binding site for a negative glucocorticoid response element (Zhang et al. (1997) DNA Cell Biol 16: 145-52). This element potentiates a four-fold repression of IL-1B expression by dexamethosone and a deletion of this negative response elements causes a 2.5-fold increase in IL-1B promoter activity. The IL-1B (-511) polymorphism may thus directly affect cytokine production and inflammatory responses. These examples demonstrate that genetic variants occurring in the IL-1A or IL-1B gene can directly lead to the altered production or regulation of IL-1 cytokine activity.

A "restenosis therapeutic" refers to any agent or therapeutic regimen (including pharmaceuticals, nutraceuticals and surgical means) that prevents or postpones the development of or alleviates the symptoms of a restenosis in a subject. A restenosis therapeutic can be a polypeptide, peptidomimetic, nucleic acid or other inorganic or organic molecule, preferably a "small molecule" including vitamins, minerals and other nutrients. Preferably a restenosis therapeutic can modulate at least one activity of an IL-1 polypeptide, e.g., interaction with a receptor, by mimicking or potentiating (agonizing) or inhibiting (antagonizing) the effects of a naturally-occurring polypeptide. An agonist can be a wild-type protein or derivative thereof having at least one bioactivity of the wild-type, e.g., receptor binding activity. An agonist can also be a compound that upregulates expression of a gene or which increases at least one bioactivity of a protein. An agonist can also be a compound which increases the interaction of a polypeptide with another molecule, e.g., a receptor. An antagonist can be a compound which inhibits or decreases the interaction between a protein and another molecule, e.g., a receptor or an agent that blocks signal transduction or post-translation processing (e.g., IL-1 converting enzyme (ICE) inhibitor). Accordingly, a preferred antagonist is a compound which inhibits or decreases binding to a receptor and thereby blocks subsequent activation of the receptor. An antagonist can also be a compound that downregulates expression of a gene or which reduces the amount of a protein present. The antagonist can be a dominant negative form of a polypeptide, e.g., a form of a polypeptide which is capable of interacting with a target peptide, e.g., a receptor, but which does not promote the activation of the receptor. The antagonist can also be a nucleic acid encoding a dominant negative form of a polypeptide, an antisense nucleic acid, or a ribozyme capable of interacting specifically with an RNA. Yet other antagonists are molecules which bind to a polypeptide and inhibit its action. Such molecules include peptides, e.g., forms of target peptides which do not have biological activity, and which inhibit binding to receptors. Thus, such peptides will bind to the active site of a protein and prevent it from interacting with target peptides. Yet other antagonists include antibodies that specifically interact with an epitope of a molecule, such that binding interferes with the biological function of the polypeptide. In yet another preferred embodiment, the antagonist is a small molecule, such as a molecule capable of inhibiting the interaction between a polypeptide and a target receptor. Alternatively, the small molecule can function as an antagonist by interacting with sites other than the receptor binding site. Preferred restenosis therapeutics include agents that suppress the development of neointimal hyperplasia, including lipid lowering drugs, antiplatelet agents, anti-inflammatory agents, antihypertensive agents and anticoagulants; and agents that directly inhibit cellular growth. Furthermore, surgical decisions at the time of the primary procedure or at the time of a secondary surgical operation could differ depending on whether the patient was at higher risk for a more prolific inflammation-mediated injury response. The decision to employ a stent as part of an endovascular procedure could be governed, for example, by an awareness of a patient's higher risk for more aggressive vascular response to injury.

A "risk factor" is a factor identified to be associated with an increased risk. A risk factor for a cardiovascular disorder or a cardiovascular disease is any factor identified to be associated with an increased risk of developing those conditions or of worsening those conditions. A risk factor can also be associated with an increased risk of an adverse clinical event or an adverse clinical outcome in a patient with a cardiovascular disorder. Risk factors for cardiovascular disease include smoking, adverse lipid profiles, elevated lipids or cholesterol, diabetes, hypertension, hypercoagulable states, elevated homocysteine levels, and lack of exercise. Carrying a particular polymorphic allele is a risk factor for a particular cardiovascular disorder, and is associated with an increased risk of the particular disorder.

"Small molecule" as used herein, is meant to refer to a composition, which has a molecular weight of less than about 5kD and most preferably less than about 4kD. Small molecules can be nucleic acids, peptides, peptidomimetics, carbohydrates, lipids or other organic or inorganic molecules.

As used herein, the term "specifically hybridizes" or "specifically detects" refers to the ability of a nucleic acid molecule to hybridize to at least approximately 6 consecutive nucleotides of a sample nucleic acid.

"Transcriptional regulatory sequence" is a generic term used throughout the specification to refer to DNA sequences, such as initiation signals, enhancers, and promoters, which induce or control transcription of protein coding sequences with which they are operably linked.

As used herein, the term "transgene" means a nucleic acid sequence (encoding, e.g., one of the IL-1 polypeptides, or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted; into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid.

A "transgenic animal" refers to any animal, preferably a non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extrachromosomally replicating DNA. In the typical transgenic animals described herein, the transgene causes cells to express a recombinant form of one of an IL-1 polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination and antisense techniques. The term is intended to include all progeny generations. Thus, the founder animal and all F1, F2, F3, and so on, progeny thereof are included.

The term "treating" as used herein is intended to encompass curing as well as ameliorating at least one symptom of a disease or at least one abnormality associated with a disorder. Treating a cardiovascular disorder can take place by administering a cardiovascular disorder therapeutic. Treating a cardiovascular disorder can also take place by modifying risk factors that are related to the cardiovascular disorder.

A "treatment plan" refers to at least one intervention undertaken to modify the effect of a risk factor upon a patient. A treatment plan for a cardiovascular disorder or disease can address those risk factors that pertain to cardiovascular disorders or diseases. A treatment plan can include an intervention that focuses on changing patient behavior, such as stopping smoking. A treatment plan can include an intervention whereby a therapeutic agent is administered to a patient. As examples, cholesterol levels can be lowered with proper medication, and diabetes can be controlled with insulin. Nicotine addiction can be treated by withdrawal medications. A treatment plan can include an intervention that is diagnostic. The presence of the risk factor of hypertension, for example, can give rise to a diagnostic intervention whereby the etiology of the hypertension is determined. After the reason for the hypertension is identified, further treatments may be administered.

The term "vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

### 4.2 Predictive Medicine

### 4.2.1. Polymorphisms Associated with Restenosis

The present invention is based at least in part, on the identification of the IL-1RN VNTR allele as being associated (to a statistically significant extent) with the development of a restenosis in subjects. Therefore, detection of thise allele, alone or in conjunction with another means in a subject indicate that the subject has or is predisposed to the development of a restenosis. For example, IL-1 polymorphic alleles which are associated with a propensity for developing restenosis include allele 1 of each of the following markers: IL-1A (+4845), IL-1B (+3954), IL-1B (-511) and IL-1RN (+2018) or an allele that is in linkage disequilibrium with one of the aforementioned alleles. The presence of a particular allelic pattern of one or more of the abovementioned IL-1 polymorphic loci may be used to predict the susceptibility of an individual to developing restenosis. In particular, there are three patterns of alleles at four polymorphic loci in the IL-1 gene cluster that show various associations with particular cardiovascular disorders. These patterns are referred to herein as patterns 1, 2 and 3. Pattern 1 comprises an allelic pattern including allele 2 of IL-1A (+4845) or IL-1B (+3954) and allele 1 of IL-1B (-511) or IL-1RN (+2018), or an allele that is in linkage disequilibrium with one of the aforementioned allele. This allelic pattern may permit the diagnosis of fragile plaque disorder. Pattern 2 comprises an allelic pattern including allele 2 of IL-1B (-511) or IL-1RN (+2018) and allele 1 of IL-1A (+4845) or IL-1B (+3954), or an allele that is in linkage disequilibrium with one of the aforementioned alleles. This allelic pattern may permit the diagnosis of occlusive cardiovascular disorder. Pattern 3 comprises an allelic pattern including allele 1 of IL-1A (+4845) or allele 1 of IL-1B (+3954), and allele 1 of IL-1B (-511) or allele 1 of IL-1RN (+2018), or an allele that is in linkage disequilibrium with one of the aforementioned alleles.

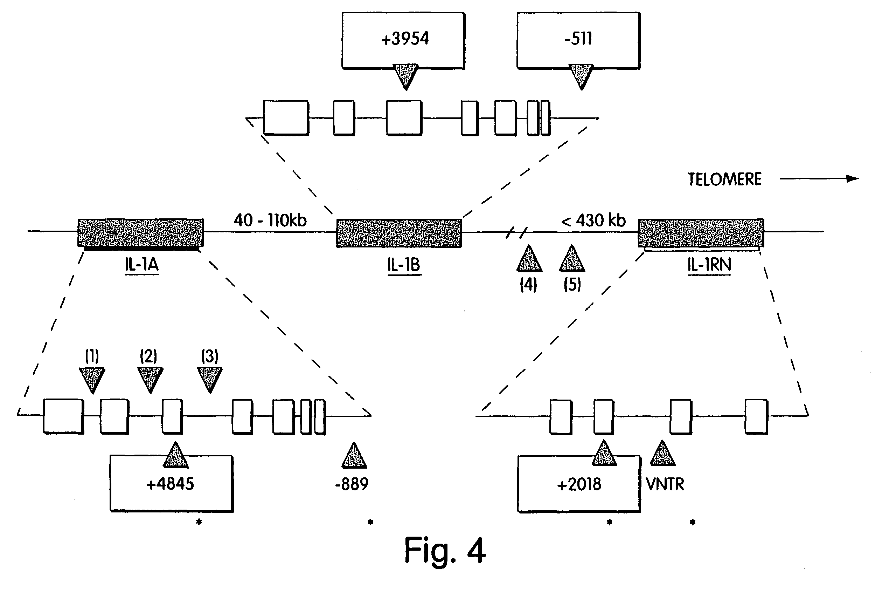

These IL-1 locus polymorphisms represent single base variations within the IL-1A/IL-1B/IL-1RN gene cluster (see Figure 4). The IL-1A (+4845) polymorphism is a single base variation (allele 1 is G, allele 2 is T) at position +4845 within Exon V of the IL-1A gene which encodes the inflammatory cytokine IL-1a (Gubler, et al.(1989) Interleukin, inflammation and disease (Bomford and Henderson, eds.) p.31-45, Elsevier publishers; and Van den velden and Reitsma (1993) Hum Mol Genetics 2:1753-50). The IL-1A (+4845) polymorphism occurs in the coding region of the gene and results in a single amino acid variation in the encoded protein (Van den Velden and Reitsma (1993) Hum Mol Genet 2: 1753). The IL-1B (-511) polymorphism is a single base pair variation (allele 1 is C, allele 2 is T) which occurs 511 base pairs upstream of the site of IL-1B gene transcription initiation (Di Giovine et al. (1992) Hum Mol Genet 1: 450). The IL-1B (+3954) polymorphism was first described as a Taq I restriction fragment length polymorphism (RFLP) (Pociot et al. (1992) Eur J Clin Invest 22: 396-402) and has subsequently been characterized as a single base variation (allele 1 is C, allele 2 is T) at position +3954 in Exon V of the IL-1B gene (di Giovine et al. (1995) Cytokine 7: 600-606). This single nucleotide change in the open reading frame of IL-1B does not appear to qualitatively affect the sequence of the encoded IL-1 beta polypeptide because it occurs at the third position of a TTC phenylalanine codon (F) of allele 1 and therefore allele 2 merely substitutes a TTT phenylalanine codon at this position which encodes amino acid 105 of the IL-1B gene product. Finally, the IL-RN variable number of tandem repeats (VNTR) polymorphism occurs within the second intron the IL-1 receptor antagonist encoding gene (Steinkasserer (1991) Nucleic Acids Res 19: 5090-5). Allele 2 of the of the IL-1RN (VNTR) polymorphism corresponds to two repeats of an 86-base pair sequence, while allele 1 corresponds to four repeats, allele 3 to three repeats, allele 4 to five repeats, and allele 5 to six repeats (Tarlow et al. (1993) Hum Genet 91: 403-4). Detection of any one of these IL-1 allelic variants in an individual suggests an increased likelihood of developing restenosis in comparison to a control individual who does not carry the allele 2 variant at the same locus.

However, because these alleles are in linkage disequilibrium with other alleles, the detection of such other linked alleles can also indicate that the subject has or is predisposed to the development of a restenosis. For example, the following alleles of the IL-1 (33221461) haplotype are in linkage disequilibrium:

| |
|---|
| allele 3 of the 222/223 marker of IL-1A |
| allele 3 of the gz5/gz6 marker of IL-1A |
| allele 2 of the -889 marker of IL-1A |
| allele 2 of the +3954 marker of IL-1B |
| allele 1 of the -511 marker of IL-1B |
| allele 4 of the gaat.p33330 marker |
| allele 6 of the Y31 marker |
| allele 1 of the VNTR or (+2018) marker of IL-1RN |

Therefore, allele 1 of IL-1B (-511) and allele 1 of IL-1RN (VNTR) are in strong linkage disequilibrium with one another and each of these is in linkage disequilibrium with allele 1 of the -511 marker of IL-1B. Furthermore, genotyping analysis at the 222/223 marker of IL-1A, the gz5/gz6 marker of IL-1A, the -889 marker of IL-1A, the +3954 marker of IL-1B, the gaat.p33330 marker of the IL-1B/IL-1RN intergenic region, or the Y31 marker of the IL-1B/IL-1RN intergenic region is determined, and the presence of a polymorphic allele which is linked to one or more of the preferred restenosis-predictive alleles is detected.

In addition, allele 1 of the IL-1RN (+2018) polymorphism (Clay et al. (1996) Hum Genet 97: 723-26), also referred to as exon 2 (8006) (GenBank:X64532 at 8006) is known to be in linkage disequilibrium with allele 1 of the IL-1RN (VNTR) polymorphic locus, which in turn is a part of the 33221461 human haplotype. In contrast, allele 2 of the IL-1RN (+2018) locus (i.e. C at +2018), is an allelic variant associated with the 44112332 haplotype and allele 2 of the IL-1RN (VNTR) polymorphic locus. The IL-1RN (VNTR) therefore provides an alternative target for prognostic genotyping analysis to determine an individual's likelihood of developing restenosis. Similarly, three other polymorphisms in an IL-1RN alternative exon (Exon lic, which produces an intracellular form of the gene product) are also in linkage disequilibrium with allele 2 of IL-1RN (VNTR) (Clay et al. (1996) Hum Genet 97: 723-26). These include: the IL-1RN exon lic (1812) polymorphism (GenBank:X77090 at 1812); the IL-1RN exon lic (1868) polymorphism (GenBank:X77090 at 1868); and the IL-1RN exon lic (1887) polymorphism (GenBank:X77090 at 1887). Furthermore yet another polymorphism in the promoter for the alternatively spliced intracellular form of the gene, the Pic (1731) polymorphism (GenBank:X77090 at 1731), is also in linkage disequilibrium with allele 2 of the IL-1RN (VNTR) polymorphic locus (Clay et al. (1996) Hum Genet 97: 723-26). The corresponding sequence alterations for each of these IL-1RN polymorphic loci is shown below.

| Allele # | Exon 2 (+2018 of IL-1RN) | Exon lic -1 (1812 of GB:X77090) | Exon lic -2 (1868 of GB: X77090 | Exon lic -3 (1887 of GB:X77090) | Pic (1731 of GB: X77090) |
|---|---|---|---|---|---|
| 1 | T | G | A | G | G |
| 2 | C | A | G | C | A |

For each of these polymorphic loci, the allele 1 sequence variant has been determined to be in linkage disequilibrium with allele 1 of the IL-1RN (VNTR) locus (Clay et al. (1996) Hum Genet 97: 723-26).

Further, allele 1 of IL-1B (+3954), which has been pointed out as a prognostic indicator of an increased propensity for developing restenosis is a component of a second haplotype, the 44112332 haplotype of co-inherited IL-1 locus polymorphic alleles (Cox, et al. (1998) Am. J. Hum. Genet. 62: 1180-88). Specifically, the 44112332 haplotype comprises the following genotype:

| |
|---|
| allele 4 of the 222/223 marker of IL-1A |
| allele 4 of the gz5/gz6 marker of IL-1A |
| allele 1 of the -889 marker of IL-1A |
| allele 1 of the +3954 marker of IL-1B |
| allele 2 of the -511 marker of IL-1B |
| allele 3 of the gaat.p33330 marker |
| allele 3 of the Y31 marker |
| allele 2 of the VNTR marker of IL-1RN |

In addition to the allelic patterns described above, as described herein, one of skill in the art can readily identify other alleles (including polymorphisms and mutations) that are in linkage disequilibrium with an allele associated with restenosis. For example, a nucleic acid sample from a first group of subjects without restenosis can be collected, as well as DNA from a second group of subjects with restenosis. The nucleic acid sample can then be compared to identify those alleles that are over-represented in the second group as compared with the first group, wherein such alleles are presumably associated with restenosis. Alternatively, alleles that are in linkage disequilibrium with a restenosis associated allele can be identified, for example, by genotyping a large population and performing statistical analysis to determine which alleles appear more commonly together than expected. Preferably the group is chosen to be comprised of genetically related individuals. Genetically related individuals include individuals from the same race, the same ethnic group, or even the same family. As the degree of genetic relatedness between a control group and a test group increases, so does the predictive value of polymorphic alleles which are ever more distantly linked to a disease-causing allele. This is because less evolutionary time has passed to allow polymorphisms which are linked along a chromosome in a founder population to redistribute through genetic cross-over events. Thus race-specific, ethnic-specific, and even family-specific diagnostic genotyping assays can be developed to allow for the detection of disease alleles which arose at ever more recent times in human evolution, e.g., after divergence of the major human races, after the separation of human populations into distinct ethnic groups, and even within the recent history of a particular family line.

Linkage disequilibrium between two polymorphic markers or between one polymorphic marker and a disease-causing mutation is a meta-stable state. Absent selective pressure or the sporadic linked reoccurrence of the underlying mutational events, the polymorphisms will eventually become disassociated by chromosomal recombination events and will thereby reach linkage equilibrium through the course of human evolution. Thus, the likelihood of finding a polymorphic allele in linkage disequilibrium with a disease or condition may increase with changes in at least two factors: decreasing physical distance between the polymorphic marker and the disease-causing mutation, and decreasing number of meiotic generations available for the dissociation of the linked pair. Consideration of the latter factor suggests that, the more closely related two individuals are, the more likely they will share a common parental chromosome or chromosomal region containing the linked polymorphisms and the less likely that this linked pair will have become unlinked through meiotic cross-over events occurring each generation. As a result, the more closely related two individuals are, the more likely it is that widely spaced polymorphisms may be co-inherited. Thus, for individuals related by common race, ethnicity or family, the reliability of ever more distantly spaced polymorphic loci can be relied upon as an indicator of inheritance of a linked disease-causing mutation.

Appropriate probes may be designed to hybridize to a specific gene of the IL-1 locus, such as IL-1A, IL-1B or IL-1RN or a related gene. These genomic DNA sequences are shown in Figures 1, 2 and 3, respectively, and further correspond to formal SEQ ID Nos. 15, 16 and 17, respectively. Alternatively, these probes may incorporate other regions of the relevant genomic locus, including intergenic sequences. Indeed the IL-1 region of human chromosome 2 spans some 400,000 base pairs and, assuming an average of one single nucleotide polymorphism every 1,000 base pairs, includes some 400 SNPs loci alone. Yet other polymorphisms are obtainable from various public sources. For example, the human genome database collects intragenic SNPs, is searchable by sequence and currently contains approximately 2,700 entries (http://hgbase.interactiva.de). Also available is a human polymorphism database maintained by the Massachusetts Institute of Technology (MIT SNP database (http://www.genome.wi.mit.edu/SNP/human/index.html)). From such sources SNPs as well as other human polymorphisms may be found.

For example, examination of the IL-1 region of the human genome in any one of these databases reveals that the IL-1 locus genes are flanked by a centromere proximal polymorphic marker designated microsatellite marker AFM220ze3 at 127.4 cM (centiMorgans) (see GenBank Acc. No. Z17008) and a distal polymorphic marker designated microsatellite anchor marker AFM087xa1 at 127.9 cM (see GenBank Acc. No. Z16545). These human polymorphic loci are both CA dinucleotide repeat microsatellite polymorphisms, and, as such, show a high degree of heterozygosity in human populations. For example, one allele of AFM220ze3 generates a 211 bp PCR amplification product with a 5' primer of the sequence TGTACCTAAGCCCACCCTT-TAGAGC (SEQ ID No. 18) and a 3' primer of the sequence TGGCCTCCAGAAACCTCCAA (SEQ ID No. 19). Furthermore, one allele of AFM087xa1 generates a 177 bp PCR amplification product with a 5' primer of the sequence GCTGATATTCTGGTGGGAAA (SEQ ID No.20) and a 3' primer of the sequence GGCAAGAGCAAAACTCTGTC (SEQ ID No. 21). Equivalent primers corresponding to unique sequences occurring 5' and 3' to these human chromosome 2 CA dinucleotide repeat polymorphisms will be apparent to one of skill in the art. Reasonable equivalent primers include those which hybridize within about 1 kb of the designated primer, and which further are anywhere from about 17 bp to about 27 bp in length. A general guideline for designing primers for amplification of unique human chromosomal genomic sequences is that they possess a melting temperature of at least about 50°C, wherein an approximate melting temperature can be estimated using the formula Tₘₑₗₜ = [2 x (# of A or T) + 4 x (# of G or C)].

A number of other human polymorphic loci occur between these two CA dinucleotide repeat polymorphisms and provide additional targets for determination of a restenosis prognostic allele in a family or other group of genetically related individuals. For example, the National Center for Biotechnology Information web site (www.ncbi.nlm.nih.gov/genemap/) lists a number of polymorphism markers in the region of the IL-1 locus and provides guidance in designing appropriate primers for amplification and analysis of these markers.

Accordingly, the nucleotide segments may be used for their ability to selectively form duplex molecules with complementary stretches of human chromosome 2 q 12-13 or cDNAs from that region or to provide primers for amplification of DNA or cDNA from this region. The design of appropriate probes for this purpose requires consideration of a number of factors. For example, fragments having a length of between 10, 15, or 18 nucleotides to about 20, or to about 30 nucleotides, will find particular utility. Longer sequences, e.g., 40, 50, 80, 90, 100, even up to full length, are even more preferred for certain embodiments. Lengths of oligonucleotides of at least about 18 to 20 nucleotides are well accepted by those of skill in the art as sufficient to allow sufficiently specific hybridization so as to be useful as a molecular probe. Furthermore, depending on the application envisioned, one will desire to employ varying conditions of hybridization to achieve varying degrees of selectivity of probe towards target sequence. For applications requiring high selectivity, one will typically desire to employ relatively stringent conditions to form the hybrids. For example, relatively low salt and/or high temperature conditions, such as provided by 0.02 M-0.15M NaCl at temperatures of about 50° C to about 70° C. Such selective conditions may tolerate little, if any, mismatch between the probe and the template or target strand.

Other alleles or other indicia of restenosis can be detected or monitored in a subject in conjunction with detection of the alleles described above. For example, echocardiography may be performed during exercise, since studies have found an association between the occurrence of clinical restenosis and both a positive post-percutaneous transluminal coronary angioplasty exercise echo as well as high values of the pre-surgical wall-motion score index and duration of wall-motion abnormalities (Peters et al. (1997) Circulation 95: 2254-61; Dagianti et al. (1997) Circulation 95: 1176-84; Gentile (1994) Cardiologia 39: 651-6). Furthermore, angioscopic studies have shown that the color (yellow versus white) of a patient's arterial plaque is highly predictive of the occurrence of restenosis following balloon angioplasty in individuals with stable angina (Itoh et al. (1995) Circulation 91: 1389-96). In addition, certain polymorphisms in the gene encoding angiotensin converting enzyme have been associated with the occurrence of restenosis after coronary angioplasty in unstable angina pectoris (See e.g. Kasi et al., (1996) Am J Cardiol 77: 875-77).

In addition, behavioral studies have shown an association between hostility and other aspects of a type A behavior pattern and an increased risk for restenosis following percutaneous transluminal coronary angioplasty (Goodman et al. (1996) Mayo Clin Proc 71: 729-34). Still other studies have demonstrated an association between various serum proteins and an increased likelihood of restenosis. For example a drop in the level of antibodies against heat shock protein-65 after percutaneous transluminal coronary angioplasty is associated with a decreased risk of developing restenosis relative to individuals in which no decrease in the level of these antibodies occurred (Mukherjee et al. (1996) Throm Haemost 75: 258-60). Another study has demonstrated an association between an elevation of serum amyloid A and the occurrence of restenosis following angioplasty (Blum et al. (1998) Clin Cardiol 21: 655-58). Relatively high levels of plasminogen activator inhibitor type-1 and relatively low levels of plasmin-plasmin inhibitor complex are also associated with restenosis (Ishiwata et al. (1997) Am Heart J 133: 387-92), as are high levels of serum lipoprotein A (Hearn et al. (1992) Am J Cardiol 69: 736-39) and elevated levels of monounsaturated fatty acids (Foley et al. (1992) Cathet Cardiovasc Diagn 25: 25-30).

### 4.2.2 Detection of Alleles

Many methods are available for detecting specific alleles at human polymorphic loci. The preferred method for detecting a specific polymorphic allele will depend, in part, upon the molecular nature of the polymorphism. For example, the various allelic forms of the polymorphic locus may differ by a single base-pair of the DNA. Such single nucleotide polymorphisms (or SNPs) are major contributors to genetic variation, comprising some 80% of all known polymorphisms, and their density in the human genome is estimated to be on average 1 per 1,000 base pairs. SNPs are most frequently biallelic- occurring in only two different forms (although up to four different forms of an SNP, corresponding to the four different nucleotide bases occurring in DNA, are theoretically possible). Nevertheless, SNPs are mutationally more stable than other polymorphisms, making them suitable for association studies in which linkage disequilibrium between markers and an unknown variant is used to map disease-causing mutations. In addition, because SNPs typically have only two alleles, they can be genotyped by a simple plus/minus assay rather than a length measurement, making them more amenable to automation.

A variety of methods are available for detecting the presence of a particular single nucleotide polymorphic allele in an individual. Advancements in this field have provided accurate, easy, and inexpensive large-scale SNP genotyping. Most recently, for example, several new techniques have been described including dynamic allele-specific hybridization (DASH), microplate array diagonal gel electrophoresis (MADGE), pyrosequencing, oligonucleotide-specific ligation, the TaqMan system as well as various DNA "chip" technologies such as the Affymetrix SNP chips. These methods require amplification of the target genetic region, typically by PCR. Still other newly developed methods, based on the generation of small signal molecules by invasive cleavage followed by mass spectrometry or immobilized padlock probes and rolling-circle amplification, might eventually eliminate the need for PCR. Several of the methods known in the art for detecting specific single nucleotide polymorphisms are summarized below. The method of the present invention is understood to include all available methods.

Several methods have been developed to facilitate analysis of single nucleotide polymorphisms. In one embodiment, the single base polymorphism can be detected by using a specialized exonuclease-resistant nucleotide, as disclosed, e.g., in Mundy, C. R. (U.S. Pat. No.4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA™ is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990); Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA^{™} in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A. -C., et al., Amer. J. Hum. Genet. 52:46-59 (1993)).

For mutations that produce premature termination of protein translation, the protein truncation test (PTT) offers an efficient diagnostic approach (Roest, et. al., (1993) Hum. Mol. Genet. 2:1719-21; van der Luijt, et. al., (1994) Genomics 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential *in vitro* transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

Any cell type or tissue may be utilized to obtain nucleic acid samples for use in the diagnostics described herein. In a preferred embodiment, the DNA sample is obtained from a bodily fluid, e.g, blood, obtained by known techniques (e.g. venipuncture) or saliva. Alternatively, nucleic acid tests can be performed on dry samples (e.g. hair or skin). When using RNA or protein, the cells or tissues that may be utilized must express an IL-1 gene.

Diagnostic procedures may also be performed *in situ* directly upon tissue sections (fixed and/or frozen) of patient tissue obtained from biopsies or resections, such that no nucleic acid purification is necessary. Nucleic acid reagents may be used as probes and/or primers for such *in situ* procedures (see, for example, Nuovo, G.J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

A preferred detection method is allele specific hybridization using probes overlapping a region of at least one allele of an IL-1 proinflammatory haplotype and having about 5, 10, 20, 25, or 30 nucleotides around the mutation or polymorphic region. In a preferred embodiment of the invention, several probes capable of hybridizing specifically to other allelic variants involved in a restenosis are attached to a solid phase support, e.g., a "chip" (which can hold up to about 250,000 oligonucleotides). Oligonucleotides can be bound to a solid support by a variety of processes, including lithography. Mutation detection analysis using these chips comprising oligonucleotides, also termed "DNA probe arrays" is described e.g., in Cronin et al. (1996) Human Mutation 7:244. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

These techniques may also comprise the step of amplifying the nucleic acid before analysis. Amplification techniques are known to those of skill in the art and include, but are not limited to cloning, polymerase chain reaction (PCR), polymerase chain reaction of specific alleles (ASA), ligase chain reaction (LCR), nested polymerase chain reaction, self sustained sequence replication (Guatelli, J.C. et al., 1990, Proc. Natl. Acad. Sci. USA 87:1874-1878), transcriptional amplification system (Kwoh, D.Y. et al., 1989, Proc. Natl. Acad. Sci. USA 86:1173-1177), and Q- Beta Replicase (Lizardi, P.M. et al., 1988, Bio/Technology 6:1197).

Amplification products may be assayed in a variety of ways, including size analysis, restriction digestion followed by size analysis, detecting specific tagged oligonucleotide primers in the reaction products, allele-specific oligonucleotide (ASO) hybridization, allele specific 5' exonuclease detection, sequencing, hybridization, and the like.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize 5' and 3' to at least one allele of an IL-1 proinflammatory haplotype under conditions such that hybridization and amplification of the allele occurs, and (iv) detecting the amplification product. These detection schemes are especially useful for the detection of nucleic acid molecules if such molecules are present in very low numbers.

In a preferred embodiment of the subject assay, the allele of an IL-1 proinflammatory haplotype is identified by alterations in restriction enzyme cleavage patterns. For example, sample and control DNA is isolated, amplified (optionally), digested with one or more restriction endonucleases, and fragment length sizes are determined by gel electrophoresis.

In yet another embodiment, any of a variety of sequencing reactions known in the art can be used to directly sequence the allele. Exemplary sequencing reactions include those based on techniques developed by Maxim and Gilbert ((1977) Proc. Natl Acad Sci USA 74:560) or Sanger (Sanger et al (1977) Proc. Nat. Acad. Sci USA 74:5463). It is also contemplated that any of a variety of automated sequencing procedures may be utilized when performing the subject assays (see, for example Biotechniques (1995) 19:448), including sequencing by mass spectrometry (see, for example PCT publication WO 94/16101; Cohen et al. (1996) Adv Chromatogr 36:127-162; and Griffin et al. (1993) Appl Biochem Biotechnol 38:147-159). It will be evident to one of skill in the art that, for certain embodiments, the occurrence of only one, two or three of the nucleic acid bases need be determined in the sequencing reaction. For instance, A-track or the like, e.g., where only one nucleic acid is detected, can be carried out.

In a further embodiment, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al. (1985) Science 230:1242). In general, the art technique of "mismatch cleavage" starts by providing heteroduplexes formed by hybridizing (labeled) RNA or DNA containing the wild-type allele with the sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S 1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al (1988) Proc. Natl Acad Sci USA 85:4397; and Saleeba et al (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes). For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on an allele of an IL-1 locus haplotype is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify an IL-1 locus allele. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control IL-1 locus alleles are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the movement of alleles in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:12753).

Examples of other techniques for detecting alleles include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al. (1986) Nature 324:163); Saiki et al (1989) Proc. Natl Acad. Sci USA 86:6230). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation or polymorphic region of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238. In addition it may be desirable to introduce a novel restriction site in the region of the mutation to create cleavage-based detection (Gasparini et al (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

In another embodiment, identification of the allelic variant is carried out using an oligonucleotide ligation assay (OLA), as described, e.g., in U.S. Pat. No. 4,998,617 and in Landegren, U. et al. ((1988) Science 241:1077-1080). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al. (1990) Proc. Natl. Acad. Sci. USA 87:8923-27). In this method, PCR is used to achieve the exponential amplification of target DNA, which is then detected using OLA.

Several techniques based on this OLA method have been developed and can be used to detect alleles of an IL-1 locus haplotype. For example, U.S. Patent No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996) Nucleic Acids Res 24: 3728), OLA combined with PCR permits typing of two alleles in a single microtiter well. By marking each of the allele-specific primers with a unique hapten, i.e. digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

Kits for detecting a predisposition for developing a restenosis are disclosed. This kit may contain one or more oligonucleotides, including 5' and 3' oligonucleotides that hybridize 5' and 3' to at least one allele of an IL-1 locus haplotype. PCR amplification oligonucleotides should hybridize between 25 and 2500 base pairs apart, preferably between about 100 and about 500 bases apart, in order to produce a PCR product of convenient size for subsequent analysis.

Particularly preferred primers for use in the diagnostic method of the invention include the following:
5' ATG GTT TTA GAA ATC ATC AAG CCT AGG GCA 3' (SEQ ID No. 1) and 5' AAT GAA AGG AGG GGA GGA TGA CAG AAA TGT 3' (SEQ ID No. 2) 5' TGG CAT TGA TCT GGT TCA TC-3' (SEQ ID No. 3) and 5' GTT TAG GAA TCT TCC CAC TT-3' (SEQ ID No. 4);
5' CTC AGG TGT CCT CGA AGA AAT CAA A 3' (SEQ ID No. 5) and 5' GCT TTT TTG CTG TGA GTC CCG 3' (SEQ ID No. 6);
5'-CTC.AGC.AAC.ACT.CCT.AT-3' (SEQ ID NO. 7) and 5'-TCC.TGG.TCT.GCA.GCT.AA-3' (SEQ ID NO. 8);
5'-CTA TCT GAG GAA CAA ACT AGT AGC-3' (SEQ ID NO. 9) and 5'-TAG GAC ATT GCA CCT AGG GTT TGT -3' (SEQ ID NO. 10);
5' ATT TTT TTA TAA ATC ATC AAG CCT AGG GCA 3' (SEQ. ID No. 11) and 5' AAT TAA AGG AGG GAA GAA TGA CAG AAA TGT 3' (SEQ. ID No. 12) 5'-AAG CTT GTT CTA CCA CCT GAA CTA GGC.-3' (SEQ ID NO. 13) and 5'-TTA CAT ATG AGC CTT CCA TG.-3' (SEQ ID NO. 14);

The design of additional oligonucleotides for use in the amplification and detection of IL-1 polymorphic alleles by the method of the invention is facilitated by the availability of both updated sequence information from human chromosome 2q13 - which contains the human IL-1 locus, and updated human polymorphism information available for this locus. For example, the DNA sequence for the IL-1A, IL-1B and IL-1RN is shown in Figures 1 (GenBank Accession No. X03833), 2 (GenBank Accession No. X04500) and 3 (GenBank Accession No. X64532) respectively. Suitable primers for the detection of a human polymorphism in these genes can be readily designed using this sequence information and standard techniques known in the art for the design and optimization of primers sequences. Optimal design of such primer sequences can be achieved, for example, by the use of commercially available primer selection programs such as Primer 2.1, Primer 3 or GeneFisher (See also, Nicklin M.H.J., Weith A. Duff G.W., "A Physical Map of the Region Encompassing the Human Interleukin-1α, interleukin-1β, and Interleukin-1 Receptor Antagonist Genes" Genomics 19: 382 (1995); Nothwang H.G., et al. "Molecular Cloning of the Interleukin-1 gene Cluster: Construction of an Integrated YAC/PAC Contig and a partial transcriptional Map in the Region of Chromosome 2q13" Genomics 41: 370 (1997); Clark, et al. (1986) Nucl. Acids. Res., 14:7897-7914 [published erratum appears in Nucleic Acids Res., 15:868 (1987) and the Genome Database (GDB) project at the URL http://www.gdb.org).

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

The kit may, optionally, also include DNA sampling means. DNA sampling means are well known to one of skill in the art and can include, but not be limited to substrates, such as filter papers, the AmpliCard™ (University of Sheffield, Sheffield, England S10 2JF; Tarlow, JW, et al., J. of Invest. Dermatol. 103:387-389 (1994)) and the like; DNA purification reagents such as Nucleon™ kits, lysis buffers, proteinase solutions and the like; PCR reagents, such as 10x reaction buffers, thermostable polymerase, dNTPs, and the like; and allele detection means such as the *Hinf*I restriction enzyme, allele specific oligonucleotides, degenerate oligonucleotide primers for nested PCR from dried blood.

### 4.2.3. Pharmacogenomics

Knowledge of the particular alleles associated with restenosis, alone or in conjunction with information on other genetic defects contributing to restenosis, such as the PL(A1/A2) polymorphism in a platelet glycoprotein (See Abbate et al. (1998) Am J Cardiol 82: 524-5), allows a customization of the restenosis therapy to the individual's genetic profile, the goal of "pharmacogenomics". For example, subjects having an allele 2 of any of the following markers: IL-1A (+4845), IL-1B (-511), IL-1B (+3954) or IL-1RN (VNTR) or any nucleic acid sequence in linkage disequilibrium with any of these alleles may have or be predisposed to developing restenosis and may respond better to particular therapeutics that address the particular molecular basis of the disease in the subject. Thus, comparison of an individual's IL-1 profile to the population profile for restenosis, permits the selection or design of drugs or other therapeutic regimens that are expected to be safe and efficacious for a particular patient or patient population (i.e., a group of patients having the same genetic alteration).

In addition, the ability to target populations expected to show the highest clinical benefit, based on genetic profile can enable: 1) the repositioning of marketed drugs with disappointing market results; 2) the rescue of drug candidates whose clinical development has been discontinued as a result of safety or efficacy limitations, which are patient subgroup-specific; and 3) an accelerated and less costly development for drug candidates and more optimal drug labeling (e.g. since measuring the effect of various doses of an agent on a restenosis causative mutation is useful for optimizing effective dose).

The treatment of an individual with a particular therapeutic can be monitored by determining protein (e.g. IL-1α, IL-1β, or IL-1Ra), mRNA and/or transcriptional level. Depending on the level detected, the therapeutic regimen can then be maintained or adjusted (increased or decreased in dose). The effectiveness of treating a subject with an agent comprises the steps of: (i) obtaining a preadministration sample from a subject prior to administration of the agent; (ii) detecting the level or amount of a protein, mRNA or genomic DNA in the preadministration sample; (iii) obtaining one or more post-administration samples from the subject; (iv) detecting the level of expression or activity of the protein, mRNA or genomic DNA in the post-administration sample; (v) comparing the level of expression or activity of the protein, mRNA or genomic DNA in the preadministration sample with the corresponding protein, mRNA or genomic DNA in the postadministration sample, respectively; and (vi) altering the administration of the agent to the subject accordingly.

Cells of a subject may also be obtained before and after administration of a therapeutic to detect the level of expression of genes other than an IL-1 gene to verify that the therapeutic does not increase or decrease the expression of genes which could be deleterious. This can be done, e.g., by using the method of transcriptional profiling. Thus, mRNA from cells exposed in vivo to a therapeutic and mRNA from the same type of cells that were not exposed to the therapeutic could be reverse transcribed and hybridized to a chip containing DNA from numerous genes, to thereby compare the expression of genes in cells treated and not treated with the therapeutic.

### 4.3 Restenosis Therapeutics

Modulators of IL-1 (e.g. IL-1α, IL-1β or IL-1 receptor antagonist) or a protein encoded by a gene that is in linkage disequilibrium with an IL-1 gene can comprise any type of compound, including a protein, peptide, peptidomimetic, small molecule, or nucleic acid. Agonists include nucleic acids (e.g. encoding an IL-1 protein or a gene that is up- or down-regulated by an IL-1 protein), proteins (e.g. IL-1 proteins or a protein that is up- or down-regulated thereby) or a small molecule (e.g. that regulates expression or binding of an IL-1 protein). Antagonists, which can be identified, are for example, using the assays described herein, include nucleic acids (e.g. single (antisense) or double stranded (triplex) DNA or PNA and ribozymes), protein (e.g. antibodies) and small molecules that act to suppress or inhibit IL-1 transcription and/or protein activity.

### 4.3.1. Effective Dose

Toxicity and therapeutic efficacy of such compounds can be determined by standard pharmaceutical procedures in cell cultures or experimental animals, e.g., for determining The LD₅₀ (the dose lethal to 50% of the population) and the Ed₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissues in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### 4.4 Assays to Identify Restenosis Therapeutics

Based on the identification of mutations that cause or contribute to the development of restenosis cell-based or cell free assays, e.g., for identifying restenosis therapeutics are disclosed, a cell expressing an IL-1 receptor, or a receptor for a protein that is encoded by a gene which is in linkage disequilibrium with an IL-1 gene, on the outer surface of its cellular membrane is incubated in the presence of a test compound alone or in the presence of a test compound and another protein and the interaction between the test compound and the receptor or between the protein (preferably a tagged protein) and the receptor is detected, e.g., by using a microphysiometer (McConnell et al. (1992) Science 257:1906). An interaction between the receptor and either the test compound or the protein is detected by the microphysiometer as a change in the acidification of the medium. This assay system thus provides a means of identifying molecular antagonists which, for example, function by interfering with protein- receptor interactions, as well as molecular agonist which, for example, function by activating a receptor.

Cellular or cell-free assays can also be used to identify compounds which modulate expression of an IL-1 gene or a gene in linkage disequilibrium therewith, modulate translation of an mRNA, or which modulate the stability of an mRNA or protein. Accordingly, a cell which is capable of producing an IL-1, or other protein is incubated with a test compound and the amount of protein produced in the cell medium is measured and compared to that produced from a cell which has not been contacted with the test compound. The specificity of the compound vis a vis the protein can be confirmed by various control analysis, e.g., measuring the expression of one or more control genes. In particular, this assay can be used to determine the efficacy of antisense, ribozyme and triplex compounds.

Cell-free assays can also be used to identify compounds which are capable of interacting with a protein, to thereby modify the activity of the protein. Such a compound can, e.g., modify the structure of a protein thereby effecting its ability to bind to a receptor. Cell-free assays for identifying such compounds consist essentially in a reaction mixture containing a protein and a test compound or a library of test compounds in the presence or absence of a binding partner. A test compound can be, e.g., a derivative of a binding partner, e.g., a biologically inactive target peptide, or a small molecule.

Accordingly, one exemplary screening assay includes the steps of contacting a protein or functional fragment thereof with a test compound or library of test compounds and detecting the formation of complexes. For detection purposes, the molecule can be labeled with a specific marker and the test compound or library of test compounds labeled with a different marker. Interaction of a test compound with a protein or fragment thereof can then be detected by determining the level of the two labels after an incubation step and a washing step. The presence of two labels after the washing step is indicative of an interaction.

An interaction between molecules can also be identified by using real-time BIA (Biomolecular Interaction Analysis, Pharmacia Biosensor AB) which detects surface plasmon resonance (SPR), an optical phenomenon. Detection depends on changes in the mass concentration of macromolecules at the biospecific interface, and does not require any labeling of interactants. A library of test compounds can be immobilized on a sensor surface, e.g., which forms one wall of a micro-flow cell. A solution containing the protein or functional fragment thereof is then flown continuously over the sensor surface. A change in the resonance angle as shown on a signal recording, indicates that an interaction has occurred. This technique is further described, e.g., in BIAtechnology Handbook by Pharmacia.

Another exemplary screening assay includes the steps of (a) forming a reaction mixture including: (i) an IL-1 or other protein, (ii) an appropriate receptor, and (iii) a test compound; and (b) detecting interaction of the protein and receptor. A statistically significant change (potentiation or inhibition) in the interaction of the protein and receptor in the presence of the test compound, relative to the interaction in the absence of the test compound, indicates a potential antagonist (inhibitor). The compounds of this assay can be contacted simultaneously. Alternatively, a protein can first be contacted with a test compound for an appropriate amount of time, following which the receptor is added to the reaction mixture. The efficacy of the compound can be assessed by generating dose response curves from data obtained using various concentrations of the test compound. Moreover, a control assay can also be performed to provide a baseline for comparison.

Complex formation between a protein and receptor may be detected by a variety of techniques. Modulation of the formation of complexes can be quantitated using, for example, detectably labeled proteins such as radiolabeled, fluorescently labeled, or enzymatically labeled proteins or receptors, by immunoassay, or by chromatographic detection.

Typically, it will be desirable to immobilize either the protein or the receptor to facilitate separation of complexes from uncomplexed forms of one or both of the proteins, as well as to accommodate automation of the assay. Binding of protein and receptor can be accomplished in any vessel suitable for containing the reactants. Examples include microtitre plates, test tubes, and micro-centrifuge tubes. In one embodiment, a fusion protein can be provided which adds a domain that allows the protein to be bound to a matrix. For example, glutathione-S-transferase fusion proteins can be adsorbed onto glutathione sepharose beads (Sigma Chemical, St. Louis, MO) or glutathione derivatized microtitre plates, which are then combined with the receptor, e.g. an ³⁵S-labeled receptor, and the test compound, and the mixture incubated under conditions conducive to complex formation, e.g. at physiological conditions for salt and pH, though slightly more stringent conditions may be desired. Following incubation, the beads are washed to remove any unbound label, and the matrix immobilized and radiolabel determined directly (e.g. beads placed in scintillant), or in the supernatant after the complexes are subsequently dissociated. Alternatively, the complexes can be dissociated from the matrix, separated by SDS-PAGE, and the level of protein or receptor found in the bead fraction quantitated from the gel using standard electrophoretic techniques such as described in the appended examples. Other techniques for immobilizing proteins on matrices are also available for use in the subject assay. For instance, either protein or receptor can be immobilized utilizing conjugation of biotin and streptavidin. Transgenic animals can also be made to identify agonists and antagonists or to confirm the safety and efficacy of a candidate therapeutic. Transgenic animals of the invention can include non-human animals containing a restenosis causative mutation under the control of an appropriate endogenous promoter or under the control of a heterologous promoter.

The transgenic animals can also be animals containing a transgene, such as reporter gene, under the control of an appropriate promoter or fragment thereof. These animals are useful, e.g., for identifying drugs that modulate production of an IL-1 protein, such as by modulating gene expression. Methods for obtaining transgenic non-human animals are well known in the art. The expression of the restenosis causative mutation may be restricted to specific subsets of cells, tissues or developmental stages utilizing, for example, cis-acting sequences that control expression in the desired pattern. Such mosaic expression of a protein can be essential for many forms of lineage analysis and can additionally provide a means to assess the effects of, for example, expression level which might grossly alter development in small patches of tissue within an otherwise normal embryo. Toward this end, tissue-specific regulatory sequences and conditional regulatory sequences can be used to control expression of the mutation in certain spatial patterns. Moreover, temporal patterns of expression can be provided by, for example, conditional recombination systems or prokaryotic transcriptional regulatory sequences. Genetic techniques, which allow for the expression of a mutation can be regulated via site-specific genetic manipulation *in vivo*, are known to those skilled in the art.

The transgenic animals all include within a plurality of their cells a restenosis causative mutation transgene of the present invention, which transgene alters the phenotype of the "host cell". In an illustrative embodiment, either the *cre*/*loxP* recombinase system ofbacteriophage P1 (Lakso et al. (1992) PNAS 89:6232-6236; Orban et al. (1992) PNAS 89:6861-6865) or the FLP recombinase system of *Saccharomyces cerevisiae* (O'Gorman et al. (1991) Science 251:1351-1355; PCT publication WO 92/15694) can be used to generate *in vivo* site-specific genetic recombination systems. Cre recombinase catalyzes the site-specific recombination of an intervening target sequence located between *loxP* sequences. *loxP* sequences are 34 base pair nucleotide repeat sequences to which the Cre recombinase binds and are required for Cre recombinase mediated genetic recombination. The orientation of *loxP* sequences determines whether the intervening target sequence is excised or inverted when Cre recombinase is present (Abremski et al. (1984) J. Biol. Chem. 259:1509-1514); catalyzing the excision of the target sequence when the *loxP* sequences are oriented as direct repeats and catalyzes inversion of the target sequence when *loxP* sequences are oriented as inverted repeats.

Accordingly, genetic recombination of the target sequence is dependent on expression of the Cre recombinase. Expression of the recombinase can be regulated by promoter elements which are subject to regulatory control, e.g., tissue-specific, developmental stage-specific, inducible or repressible by externally added agents. This regulated control will result in genetic recombination of the target sequence only in cells where recombinase expression is mediated by the promoter element. Thus, the activation of expression of the causative mutation transgene can be regulated via control of recombinase expression.

Use of the *cre*/*loxP* recombinase system to regulate expression of a causative mutation transgene requires the construction of a transgenic animal containing transgenes encoding both the Cre recombinase and the subject protein. Animals containing both the Cre recombinase and the restenosis causative mutation transgene can be provided through the construction of "double" transgenic animals. A convenient method for providing such animals is to mate two transgenic animals each containing a transgene.

Similar conditional transgenes can be provided using prokaryotic promoter sequences which require prokaryotic proteins to be simultaneous expressed in order to facilitate expression of the transgene. Exemplary promoters and the corresponding trans-activating prokaryotic proteins are given in U.S. Patent No. 4,833,080.

Moreover, expression of the conditional transgenes can be induced by gene therapy-like methods wherein a gene encoding the transactivating protein, e.g. a recombinase or a prokaryotic protein, is delivered to the tissue and caused to be expressed, such as in a cell-type specific manner. By this method, the transgene could remain silent into adulthood until "turned on" by the introduction of the transactivator.

In an exemplary embodiment, the "transgenic non-human animals" are produced by introducing transgenes into the germline of the non-human animal. Embryonal target cells at various developmental stages can be used to introduce transgenes. Different methods are used depending on the stage of development of the embryonal target cell. The specific line(s) of any animal are selected for general good health, good embryo yields, good pronuclear visibility in the embryo, and good reproductive fitness. In addition, the haplotype is a significant factor. For example, when transgenic mice are to be produced, strains such as C57BL/6 or FVB lines are often used (Jackson Laboratory, Bar Harbor, ME). Preferred strains are those with H-2^{b}, H-2^{d} or H-2^{q} haplotypes such as C57BL/6 or DBA/1. The line(s) used may themselves be transgenics, and/or may be knockouts (i.e., obtained from animals which have one or more genes partially or completely suppressed).

The transgene construct may be introduced into a single stage embryo. The zygote is the best target for microinjection. In the mouse, the male pronucleus reaches the size of approximately 20 micrometers in diameter which allows reproducible injection of 1-2 pl of DNA solution. The use of zygotes as a target for gene transfer has a major advantage in that in most cases the injected DNA will be incorporated into the host gene before the first cleavage (Brinster et al. (1985) PNAS 82:4438-4442). As a consequence, all cells of the transgenic animal will carry the incorporated transgene. This will in general also be reflected in the efficient transmission of the transgene to offspring of the founder since 50% of the germ cells will harbor the transgene.

Normally, fertilized embryos are incubated in suitable media until the pronuclei appear. At about this time, the nucleotide sequence comprising the transgene is introduced into the female or male pronucleus as described below. In some species such as mice, the male pronucleus is preferred. It is most preferred that the exogenous genetic material be added to the male DNA complement of the zygote prior to its being processed by the ovum nucleus or the zygote female pronucleus. It is thought that the ovum nucleus or female pronucleus release molecules which affect the male DNA complement, perhaps by replacing the protamines of the male DNA with histones, thereby facilitating the combination of the female and male DNA complements to form the diploid zygote. Thus, it is preferred that the exogenous genetic material be added to the male complement of DNA or any other complement of DNA prior to its being affected by the female pronucleus. For example, the exogenous genetic material is added to the early male pronucleus, as soon as possible after the formation of the male pronucleus, which is when the male and female pronuclei are well separated and both are located close to the cell membrane. Alternatively, the exogenous genetic material could be added to the nucleus of the sperm after it has been induced to undergo decondensation. Sperm containing the exogenous genetic material can then be added to the ovum or the decondensed sperm could be added to the ovum with the transgene constructs being added as soon as possible thereafter.

Introduction of the transgene nucleotide sequence into the embryo may be accomplished by any means known in the art such as, for example, microinjection, electroporation, or lipofection. Following introduction of the transgene nucleotide sequence into the embryo, the embryo may be incubated *in vitro* for varying amounts of time, or reimplanted into the surrogate host, or both. In vitro incubation to maturity is within the scope of this invention. One common method in to incubate the embryos in vitro for about 1-7 days, depending on the species, and then reimplant them into the surrogate host.

For the purposes of a description a zygote is essentially the formation of a diploid cell which is capable of developing into a complete organism. Generally, the zygote will be comprised of an egg containing a nucleus formed, either naturally or artificially, by the fusion of two haploid nuclei from a gamete or gametes. Thus, the gamete nuclei must be ones which are naturally compatible, i.e., ones which result in a viable zygote capable of undergoing differentiation and developing into a functioning organism. Generally, a euploid zygote is preferred. If an aneuploid zygote is obtained, then the number of chromosomes should not vary by more than one with respect to the euploid number of the organism from which either gamete originated.

In addition to similar biological considerations, physical ones also govern the amount (e.g., volume) of exogenous genetic material which can be added to the nucleus of the zygote or to the genetic material which forms a part of the zygote nucleus. If no genetic material is removed, then the amount of exogenous genetic material which can be added is limited by the amount which will be absorbed without being physically disruptive. Generally, the volume of exogenous genetic material inserted will not exceed about 10 picoliters. The physical effects of addition must not be so great as to physically destroy the viability of the zygote. The biological limit of the number and variety of DNA sequences will vary depending upon the particular zygote and functions of the exogenous genetic material and will be readily apparent to one skilled in the art, because the genetic material, including the exogenous genetic material, of the resulting zygote must be biologically capable of initiating and maintaining the differentiation and development of the zygote into a functional organism.

The number of copies of the transgene constructs which are added to the zygote is dependent upon the total amount of exogenous genetic material added and will be the amount which enables the genetic transformation to occur. Theoretically only one copy is required; however, generally, numerous copies are utilized, for example, 1,000-20,000 copies of the transgene construct, in order to insure that one copy is functional. More than one functioning copy of each of the inserted exogenous DNA sequences to enhance the phenotypic expression of the exogenous DNA sequences may be advantageous.

Any technique which allows for the addition of the exogenous genetic material into nucleic genetic material can be utilized so long as it is not destructive to the cell, nuclear membrane or other existing cellular or genetic structures. The exogenous genetic material is preferentially inserted into the nucleic genetic material by microinjection. Microinjection of cells and cellular structures is known and is used in the art.

Reimplantation is accomplished using standard methods. Usually, the surrogate host is anesthetized, and the embryos are inserted into the oviduct. The number of embryos implanted into a particular host will vary by species, but will usually be comparable to the number of off spring the species naturally produces.

Transgenic offspring of the surrogate host may be screened for the presence and/or expression of the transgene by any suitable method. Screening is often accomplished by Southern blot or Northern blot analysis, using a probe that is complementary to at least a portion of the transgene. Western blot analysis using an antibody against the protein encoded by the transgene may be employed as an alternative or additional method for screening for the presence of the transgene product. Typically, DNA is prepared from tail tissue and analyzed by Southern analysis or PCR for the transgene. Alternatively, the tissues or cells believed to express the transgene at the highest levels are tested for the presence and expression of the transgene using Southern analysis or PCR, although any tissues or cell types may be used for this analysis.

Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

Progeny of the transgenic animals may be obtained by mating the transgenic animal with a suitable partner, or by *in vitro* fertilization of eggs and/or sperm obtained from the transgenic animal. Where mating with a partner is to be performed, the partner may or may not be transgenic and/or a knockout; where it is transgenic, it may contain the same or a different transgene, or both. Alternatively, the partner may be a parental line. Where *in vitro* fertilization is used, the fertilized embryo may be implanted into a surrogate host or incubated *in vitro*, or both. Using either method, the progeny may be evaluated for the presence of the transgene using methods described above, or other appropriate methods.

The transgenic animals produced will include exogenous genetic material. Further, the sequence will be attached to a transcriptional control element, e.g., a promoter, which preferably allows the expression of the transgene product in a specific type of cell.

Retroviral infection can also be used to introduce the transgene into a non-human animal. The developing non-human embryo can be cultured *in vitro* to the blastocyst stage. During this time, the blastomeres can be targets for retroviral infection (Jaenich, R. (1976) PNAS 73:1260-1264). Efficient infection of the blastomeres is obtained by enzymatic treatment to remove the zona pellucida (Manipulating the Mouse Embryo, Hogan eds. (Cold Spring Harbor Laboratory Press, Cold Spring Harbor, 1986). The viral vector system used to introduce the transgene is typically a replication-defective retrovirus carrying the transgene (Jahner et al. (1985) PNAS 82:6927-6931; Van der Putten et al. (1985) PNAS 82:6148-6152). Transfection is easily and efficiently obtained by culturing the blastomeres on a monolayer of virus-producing cells (Van der Putten, *supra*; Stewart et al. (1987) EMBO J. 6:383-388). Alternatively, infection can be performed at a later stage. Virus or virus-producing cells can be injected into the blastocoele (Jahner et al. (1982) Nature 298:623-628). Most of the founders will be mosaic for the transgene since incorporation occurs only in a subset of the cells which formed the transgenic non-human animal. Further, the founder may contain various retroviral insertions of the transgene at different positions in the genome which generally will segregate in the offspring. In addition, it is also possible to introduce transgenes into the germ line by intrauterine retroviral infection of the midgestation embryo (Jahner et al. (1982) *supra*).

A third type of target cell for transgene introduction is the embryonal stem cell (ES). ES cells are obtained from pre-implantation embryos cultured *in vitro* and fused with embryos (Evans et al. (1981) Nature 292:154-156; Bradley et al. (1984) Nature 309:255-258; Gossler et al. (1986) PNAS 83: 9065-9069; and Robertson et al. (1986) Nature 322:445-448). Transgenes can be efficiently introduced into the ES cells by DNA transfection or by retrovirus-mediated transduction. Such transformed ES cells can thereafter be combined with blastocysts from a non-human animal. The ES cells thereafter colonize the embryo and contribute to the germ line of the resulting chimeric animal. For review see Jaenisch, R (1988) Science 240:1468-1474.

The practice of the present invention will employ, unless otherwise indicated, conventional techniques that are within the skill of the art. Such techniques are explained fully in the literature. See, for example, Molecular Cloning A Laboratory Manual, (2nd ed., Sambrook, Fritsch and Maniatis, eds., Cold Spring Harbor Laboratory Press: 1989); DNA Cloning, Volumes I and II (D. N. Glover ed., 1985); Oligonucleotide Synthesis (M. J. Gait ed., 1984); U.S. Patent No. 4,683,195; U.S. Patent No. 4,683,202; and Nucleic Acid Hybridization (B. D. Hames & S. J. Higgins eds., 1984).

### 5. Examples

### 5.1. IL-1 RN*2 Allele Association with Decreased Risk of Restenosis

In this example, DNA samples collected form 171 patients attending for elective percutaneous transluminal coronary angioplasty were studied at 4 and 6 months post-surgery using angiography. At follow-up angiography, the patients were separated into restenosers (>50% luminal narrowing) and non-restenosers (<50% luminal narrowing), and were further assessed for their genotype at the following IL-1 polymorphisms: IL-1A (-889), IL-1B (-511), IL-1B (+3954), IL-1RN (intron 2 VNTR).

### Methods

### Patients

171 patients who were scheduled to undergo follow-up angiography after elective PTCA without stenting as part of other protocols were studied. Quantitive coronary angiography was performed on-line (Philips Integris HM 3000, (S); Siemens Micor (L)). Patients were electively recruited in Sheffield where follow-up angiography was performed at 6 months. 117 patients were recruited from Leicester. These patients had been part of the SHARP study (Subcutaneous Heparin and Angioplasty Restenosis Prevention) where follow-up had been performed at 4 months ± 2 weeks (Samani NJ, et al., *Lancet*. 1995;**345**:1013-1016), and 67% of the original cohort were electively recalled for the current study. The SHARP study did not show any effect of subcutaneous heparin upon rates of restenosis.

A dichotomous definition of restenosis was used setting restenosis as a luminal narrowing >50% and non-restenosis <50%, at follow-up angiography. Using this definition, the cohort comprised 39% restenosers and 61 % non-restenosers.

These studies were approved by the North Sheffield Ethics Committee and by the Leicester Ethics Committee, and patients gavie their written informed consent.

### Analysis of genetic polymorphisms

Genomic DNA was extracted from whole blood using standard methods and PCR for variants within the IL-1 locus performed as previously described (Francis SE, et al. Circulation 1999;99:861-866). or using an automated Taqman^{™} FRET-based system. The less common IL-RN gene variant is referred to as IL-1RN.*2.

Differences in genotype distribution were assessed by chi-square analysis of the relevant 2*2 contingency table (table 2). Odds ratios with 95% confidence intervals were also calculated. To summarize results over the Leicester and Sheffield cohorts Mantel Haenszel analyses were performed. A p-value of less than 0.05 was used to indicate nominal significance. For an overall type 1 error of 0.05, a corrected critical p-value of 0.013 should be used to account for muliple testing. Here we have corrected accounting for the 4 loci tested. However, due to linkage disequilibrium between these loci, this correction is likely to be conservative. IL-1RN (VNTR) was collapsed and analysed as a biallelic marker since very few genotypes were recorded with the rarer alleles. Neither of the cohorts studied were significantly different from the Hardy Weinberg Equilibrium for any of the polymorphisms.

Demographic data were expressed as percent with actual counts in parentheses. These variables were compared by χ² test.

### Results

### Demographics

The Sheffield and Leicester combined cohorts were well matched for baseline clinical features (Table 1).

### Genetic analysis

The Mantel-Haenzel results summarized over the Leicester and Sheffield cohorts showed no significant differences in genotypic distributions at the IL-1A (-889), IL-1B (+3954) and IL-1B (-511) loci between restenosers and non-restenosers (Table II).

The frequency of allele 2 (IL-IRN *2) was however increased in the non-restenosers: 34% versus 23% in restenosers. Genotype distribution analysis indicated a significant association between homozygosity for allele *2 and non-restenosis (MH, p=0.0196 (L+S); p=0.0131 (L+S, SVD only, Table III)). When the populations are analysed separately, the data trends concur, but are only significant in the Sheffield SVD cohort (p=0.0384 (S); p=0.1573 (L)). This is most likely because of the low statistical power of these tests, since sample sizes are small due to data subdivision.

Interestingly, and a further implication that the results are more specifically applicable to SVD only, when carriage of IL-1RN*2 is compared between SVD and MVD groups in the Leicester cohort, there is a significant increase of carriage of IL-RN*2 in the SVD group (p=0.0342). This result is strengthened when the Sheffield SVD patients are added (p=0.0314).

### Discussion

These data suggest a genetic susceptibility to restenosis mediated by polymorphism at the IL-1 locus.

Specifically, the data presented here indicate that IL-1RN*2 is associated with a lower restenosis rate in patients with SVD. This supports previous data indicating that distinct populations with different prepensitites to restenosis exist, and that the precess is at least to some extent patient-related rather than lesion dependent or both (Lehmann KG, et al. Circulation, 1996;93:1123-1132; Weintraub WS, et al. Am J Cardiol. 1993;72:1107-1113). Our previous data (Francis SE, et al. Circulation 1999;99:861-866)., that IL-1RN*2 is associated with SVD on the basis of angiography, led us to speculate that there may be a true genetic distinction between SVD and MVD. If so, this might indicate that IL-1RN*2 genotype could either lead more rapidly to SVD or protest against progression to MVD. The data presented here add to this.

Since restenosis is a biological phenomenon characterized by an early inflammatory response, these new data suggest that IL-1RN*2 may modulate the arterial wall response to injury in such a way as to reduce the likelihood of restenosis. Whilst there are many potential mechanisms by which this could occur, a protection or beneficial effect of IL-1RN*2 upon vessel wall healing in response to injury is suggested. This might also support the hypothesis that IL-1RN*2 slows progression toward MVD made in our earlier study (Francis SE, et al. Circulation 1999;99:861-866)..

The mechanism by which IL-1RN*2 modulates the vessel wall response to injury is unclear. This polymorphism has functional correlates but these appear highly cell-type specific. In monocytes, IL-1RN*2 is associated with increased IL-lra production under basal and stimulated conditions (Wilkinson RJ, et al. J Exp Med. 1999;189:1863-1873). In contrast, within cells of the columnar epithelium in inflammatory bowel disease (Carter MJ, Gastroenterology. 1998;114(4):3882), and in endothelial cells (Dewberry RM, et al. Heart. 1999;81(Suppl 1); 78 [abstract]), IL-1RN*2 os associated with reduced production of IL-1ra. Since the inflammatory influx seen following experimental PTCA in pigs is highly neutrophilic and IL-IB staining abundant, predominantly in the luminal endothelium even into the late phase of healing (Chamberlin J, et al. Cardiovasc Res. 1999;44(1):156-165), we speculate that the relatively pro-inflammatory endothelial cell phenotype created by the IL-1RN*2 genotype may be important to PTCA. This suggests that modifying the inflammatory response at the time of injury may indeed be beneficial acting to limit the healing response that leads to luminal re-narrowing.

The IL-1RN VNTR polymorphism is known to be in linkage disequilibrium with other genes in the IL-1 locus (Cox A, et al. Am J Hum Genet. 1998;62(5):1180-1188), and although there are some weakly consistent trends which exist for IL-1A (=4845) and IL-1B (+3954), there are no other significant associations with restenosis or non-restenosis for the other IL-1 polymorphisms within the cluster. Hence, a specific complex haplotype is not supported by these data. However, linkage disequilibrium between this polymorphism and other unidentified gene polymorphisms cannot be excluded.

Due to sub-division of the data, this study has small sample sizes for many of the analyses performed. This reduces power and to some extent the reliability and confidence in these findings. However, the results here are strengthened by the fact that two separate cohorts were collected, and that very similar directional trends were found in both populations. It was consistently found that evidence for association was strengthened by summarizing over the two cohorts, which further illustrates the concordance. It is, of course, possible that spurious results could have arisen due to genetic admixture within the cohorts, but again the consistence between the two populations argues away from this.

We favor the interpretation that polymorphic variation within the IL-1 locus has an important inpact on arterial disease. Our original published work (Francis SE, et al. Circulation 1999;99:861-866). showed an association with single vessel coronary disease in two independent populations (Sheffield and London). The study reported here shows association with a different clinical phenotype in a population predominantly from Leicester. Other investigators have demonstrated association between IL-1RN+2016, a single nucleotide polymorphism (SNP) in linkage disequilibrium with IL-1RN*2, and carotid intimal/medial changes in African Americans (Pankow JS, et al. Association of Interleukin-1 gene variants and carotid arterial wall thickness: the ARID Study. *71st EAS Congress and Satellite Symposia*) These all argue strongly that polymorphism within the IL-1 locus does have an impact on the pathogenesis of atherosclerotic lesions, although the mechanism remains to be elucidated.

The biological control of IL-1 is complex (Dinarello CA. Blood. 1991;77:1627-1632). IL-1 actions are inhibited by a non-signaling receptor IL-1RII in membrane bound or soluble form and also by IL-lra (Symons JA, et al. J Exp Med. 1991;177:557-560) which binds without agonist activity to be signaling receptor IL-1RI (Symons JA, et al. Proc Natl Acad Sci. 1995;92:1714-1718). IL-1ra is an acute phase protein and induced by cytokines and bacterial products (Arend WP. Adv Immunol. 1993;54:167-227). Levels of IL-1 and IL-lra *in vivo* vary in parallel suggesting a coordinated pattern of regulation (Arend WP. Adv Immunol. 1993;54:167-227). IL-lra is detected in the endothelium of diseased coronary arteries (Dewberry RM, et al. Heart. 1999;81(Suppl 1); 78 [abstract]) and inhibits fatty streak formation in the apolipoprotein E deficient mouse (Hirsch E, et al. Proc Natl Acad Sci. 1996;93:11008-11013). These data taken together strongly implicate IL-lra in the control of inflammation in the arterial wall.

In conclusion, the results reported here suggest an important association between IL-1RN*2 and protection from restenosis in individuals with SVD. They also might suggest that inflammation may be a positive influence rather than wholly negative after arterial injury. Validation studies in larger study groups including a post-stenting and a reappraisal of the complex injury-repair mechanisms employed by the arterial wall are indicated.

**Table I.**

| **Clinical Characteristics of Patients with and without Restenosis** | | | |
|---|---|---|---|
| | **Restenosis** | **Non-restenosis** | **P** |
| **Leicester** | | | |
| no. of patients | 49 | 69 | |
| age (yrs) mean±SEM | 59.08±1.19 | 57.08±0.91 | ns |
| Women (%) | 12.2[6] | 17.4[12] | nd |
| Hypertnesion (%) | 24[12] | 17.3[12] | nd |
| Smoking (%) | 29[14] | 34.7[24] | nd |
| Diabetes (%) | 2.04 [1] | 4.34 [3] | nd |
| MI (%) | 48.9 [24] | 43.4 [30] | nd |
| Multivessel disease (%) | 48.9 [24] | 39.1 [27] | nd |

| **Sheffield** | | | |
|---|---|---|---|
| no. of patients | 18 | 35 | |
| age (yrs) mean±SEM | 53.61±1.77 | 53.88±1.45 | ns |
| Women (%) | 17 [3] | 11.4 [4] | nd |
| Hypertension (%) | 61.1 [11] | 37.1 [13] | nd |
| Smoking (%) | 77.7 [14] | 74.2 [26] | nd |
| Diabetes (%) | 5.5 [1] | 11.4 [4] | nd |
| MI (%) | 57.1 [8] | 42.8 [15] | nd |
| Multivessel disease (%) | 0 | 0 | nd |

| **Sheffield and Leicester** | | | |
|---|---|---|---|
| no. of patients | 67 | 104 | |
| age (yrs) mean±SEM | 57.97±1.44 | 55.98±0.98 | ns |
| Women (%) | 13.4 [9] | 15.3 [16] | ns |
| Hypertension (%) | 34.3 [23] | 24.0 [25] | ns |
| Smoking (%) | 41.7 [28] | 48.0 [50] | ns |
| Diabetes (%) | 2.98 [2] | 6.7 [7] | ns |
| MI (%) | 47.7 [32] | 43.2 [45] | ns |
| Multivessel disease (%) | 35.8 [24] | 25.9 [27] | ns |

| | | | |
|---|---|---|---|
| Values in parentheses are the number of patients affected in that cohort. Hypertension defined as diastolic bp >95mmHg (Leicester); Sytolic bp >160mmHg. Smoking: current or former (Sheffield), current (Leicester). ns - not significant, where normal statistical significance, P<0.05.nd - not done. | | | |

**Table II**

| **Carriage of alleles within the IL-1 locus in Sheffield and Leicester restenosis and non-restenosis cohorts.** | | | | | | | | |
|---|---|---|---|---|---|---|---|---|
| | 11 | 12/2 2 | 11 | 12/22 | 11/12 | 22 | 11/12 | 22 |
| Leicester SVD & MVD | | | | | | | | |
| restenosis | 20 | 24 | 26 | 19 | 36 | 7 | 46 | 3 |
| non | 34 | 27 | 42 | 19 | 47 | 12 | 58 | 10 |
| p-value | 0.2399 | | 0.2982 | | 0.6194 | | 0.6191 | |
| OR 95% CI | 1.6 0.7,3.6 | | 1.5 0.7,3.3 | | 1.3 0.5,3.7 | | 2.6 0.7, 10.2 | |

| **Sheffield** SVD only | | | | | | | | |
|---|---|---|---|---|---|---|---|---|
| restenosis | 8 | 10 | 10 | 8 | 15 | 3 | 16 | 0 |
| non | 20 | 14 | 22 | 11 | 29 | 4 | 25 | 7 |
| p-value | 0.4329 | | 0.3244 | | 0.6691 | | 0.0384 | |
| OR | 1.6 | | 1.8 | | 0.7 | | N/A | |
| 95% CI | 0.5,5.2 | | 0.6, 5.7 | | 01., 3.5 | | N/A | |

| **MH** | | | | | | | | |
|---|---|---|---|---|---|---|---|---|
| p-value | 0.1604 | | 0.1594 | | 0.8333 | | 0.0196 | |

| | | | | | | | | |
|---|---|---|---|---|---|---|---|---|
| **MH** Mantel-Haenzsel summary statistic **N/A** OR and p-value not applicable since one of the values in the contingency table is 0. **Note:** Alleles are grouped according to previously described commonest haplotype (Cox A, et al. Am J Hum Genet. 1998;62(5):1180-1188), carriage of *2 for IL-1A [+4845]; IL1B [+3954] and carriage of *1 for IL-1B [-511] and IL-1Rn [VNTR]. | | | | | | | | |

**Table III**

| **Homozygosity at IL-1RN*2 illustrates the difference between SVD and MVD in the Sheffield and Leicester cohorts.** | | | | |
|---|---|---|---|---|
| | **SVD** | | **MVD** | |
| | 11/12 | 22 | 11/12 | 22 |
| **Leicester** | | | | |
| restenosis | 24 | 1 | 22 | 2 |
| non | 35 | 7 | 23 | 3 |
| p-value | 0.1573 | | 0.7699 | |
| OR | 4.8 | | 1.4 | |
| 95% CI | 0.6, 41.6 | | 0.1,4.6 | |

| Sheffield | | | | |
|---|---|---|---|---|
| restenosis | 16 | 0 | N/A | |
| non | 25 | 7 | | |
| p-value | 0.0384 | | | |
| OR 95% CI | N/A | | | |

| **MH** | | | | |
|---|---|---|---|---|
| p-value | 0.0131 | | | |

| | | | | |
|---|---|---|---|---|
| **MH** Mantel Naenszel summary statistic **N/A** OR and p-value not applicable since one of the values in the contingency table is 0.. | | | | |

### 5.2. Protective Role Against Restenosis from an Interleukin-1 Receptor Antagonist Gene Polymorphism in Patients Treated with Coronary Stenting (The Munich Study)

### Patients

The study included 1850 consecutive Caucasian patients with symptomatic coronary artery disease who underwent coronary stent implantation at Deutsches Herzzentrum München and 1. Medizinische Klinik rechts der Isar der Technischen Universität München. All patients were scheduled for angiographic follow-up at 6 months. All patients participating in this study gave written informed consent for the intervention, follow-up angiography, and genotype determination. The study protocol conformed to the Declaration of Helsinki and was approved by the institutional ethics committee.

**Table 3. Baseline clinical characteristics.**

| | | IL-1RN 1/2 or 2/2 (n=896) | IL-1RN 1/1 (n=954) | P |
|---|---|---|---|---|
| Age - yr | | 63.4±10.0 | 62.6±10.0 | 0.11 |
| Women - % | | 22.4 | 19.9 | 0.19 |
| Arterial hypertension - % | | 67.2 | 68.9 | 0.44 |
| Diabetes - % | | 22.7 | 19.4 | 0.08 |
| Current or former smoker - % | | 38.7 | 41.2 | 0.28 |
| Elevated total cholesterol - % | | 42.5 | 43.1 | 0.81 |
| Acute myocardial infarction - % | | 20.3 | 20.2 | 0.97 |
| Unstable angina - % | | 27.9 | 27.8 | 0.95 |
| Prior bypass surgery - % | | 10.6 | 11.5 | 0.53 |
| Reduced left ventricular function - % 31.3 27.7 0.09 | | | | |
| Number of diseased coronary vessels | | | | 0.39 |
| | - 1 vessel - % | 29.2 | 27.3 | |
| | - 2 vessels - % | 32.9 | 31.9 | |
| | - 3 vessels - % | 37.8 | 40.9 | |
| Periprocedural abciximab therapy - % | | 19.8 | 19.6 | 0.93 |
| Data are proportions or mean SD | | | | |

The protocol of stent placement and poststenting therapy is familiar to practitioners in the arts. Most of the stents were implanted hand-mounted on conventional angioplasty balloons. Postprocedural therapy consisted of aspirin (100 mg twice daily, indefinitely) and ticlopidine (250 mg twice daily for 4 weeks). Patients with suboptimal results due to residual thrombus or dissection with flow impairment after stent implantation received additional therapy with abciximab given as bolus injection during stent insertion procedure and as a 12-hours continuous infusion thereafter. The decision to give abciximab was taken at the operator's discretion.

### Determination of the IL-1RN Genotype

Genomic DNA was extracted from 200 ml of peripheral blood leukocytes with the QIAamp Blood Kit (Qiagen, Hilden, Germany) and the High Pure PCR Template Preparation Kit (Boehringer Mannheim, Mannheim, Germany).

IL-1RN genotyping was performed with the ABI Prism Sequence Detection System (PE Applied Biosystems, Weiterstadt, Germany). The use of allele-specific fluorogenic probes in the 5' nuclease reaction combines DNA amplification and genotype determination into a single assay 33. IL-1RN (+2018), a single base pair polymorphism in exon 2, was the polymorphism typed for this study 26. The nucleotide sequences of primers and probes were as follows: forward primer 5' GGG ATG TTA ACC AGA AGA CCT TCT ATC T 3'(SEQ ID NO. 22), reverse primer 5' CAA CCA CTC ACC TTC TAA ATT GAC ATT 3' (SEQ ID NO. 23), allele 1 probe 5' AAC AAC CAA CTA GTT GCT GGA TAC TTG CAA 3'(SEQ ID NO. 24), allele 2 probe 5' ACA ACC AAC TAG TTG CCG GAT ACT TGC 3'(SEQ ID NO. 25). The probes for allele 1 were labeled with the fluorescent dye 6-carboxy-fluorescein (FAM) and for allele 2 with the fluorescent dye tetrachloro-6-carboxy-fluorescein (TET) at the 5' end. Both probes were labeled with the quencher 6-carboxy-tetramethyl-rhodamine (TAMRA) at their 3' ends. The thermocycling protocol consisted of 40 cycles of denaturation at 95 C for 15 seconds and annealing/extension at 64 C for 1 minute. Genotype validation was performed by repeating the determination in 20% of the patients using a duplicate DNA sample with a novel subject code unrelated to the original subject code. There was a 100% matching between the 2 results.

### Angiographic Assessment

Coronary lesions were classified according to the modified American College of Cardiology/American Heart Association grading system. Left ventricular function was assessed qualitatively on the basis of biplane angiograms using a 7 segment division; the diagnosis of reduced left ventricular function was established in the presence of at least two hypokinetic segments in the contrast angiogram. Quantitative computer-assisted angiographic analysis was performed off-line on angiograms obtained just before stenting, immediately after stenting, and at follow up using the automated edge-detection system CMS (Medis Medical Imaging Systems, Nuenen, The Netherlands). Operators were unaware of the patient's IL-1RN genotype. Identical projections of the target lesion were used for all assessed angiograms. Minimal lumen diameter, interpolated reference diameter, diameter stenosis, lesion length and diameter of the maximally inflated balloon were the angiographic parameters obtained with this analysis system. Acute lumen gain was calculated as the difference between minimal lumen diameter at the end of intervention and minimal lumen diameter before the intervention. Late lumen loss was calculated as the difference between minimal lumen diameter at the end of intervention and minimal lumen diameter at the time of follow-up angiography. Loss index was calculated as the ratio between late lumen loss and acute lumen gain.

### Definitions and Study Endpoints

Primary endpoint of the study was restenosis. Two measures of restenosis were assessed: the incidence of angiographic restenosis defined as a diameter stenosis of 50% at 6-month follow-up angiography, and the need for target vessel revascularization (PTCA or aortocoronary bypass surgery [CABG]) due to symptoms or signs of ischemia in the presence of angiographic restenosis at the stented site over 1 year after the intervention. Other major adverse events evaluated were: death from any cause and myocardial infarction. All deaths were considered due to cardiac causes unless an autopsy established a noncardiac cause. The diagnosis of acute myocardial infarction was based on the criteria applied in the EPISTENT trial (new pathological Q waves or a value of creatine kinase [CK] or its MB isoenzyme at least 3 times the upper limit) 35. CK was determined systematically over the 48 hours following stenting procedure. Clinical events were monitored throughout the 1-year follow-up period. The assessment was made on the basis of the information provided by hospital readmission records, referring physician or phone interview with the patient. For all those patients who revealed cardiac symptoms during the interview, at least one clinical and electrocardiographic check-up was performed at the outpatient clinic or by the referring physician.

### Statistical Analysis

Discrete variables are expressed as counts or percentages and compared with Chi-square or Fisher's exact test, as appropriate. Continuous variables are expressed as mean SD and compared by means of the unpaired, two-sided t-test or analysis of variance for more than 2 groups. Risk analysis was performed calculating the odds ratio and the 95% confidence interval. The main analysis consisted in comparing combined heterozygous and homozygous carriers of the IL-11RN*2 allele with homozygous carriers of the IL-11RN*1 allele. Moreover, the association between IL-1RN genotype and restenosis was assessed in a multivariate logistic regression model including also those clinical and lesion-related characteristics for which the comparison between carriers and noncarriers of the IL-1RN*2 allele showed a P-value 0.30. In this multivariate model, we tested for the possible interaction between IL-1RN genotype and age. Since the relative contribution of genetic factors to multifactorial processes such as restenosis may decrease with the age, we carried out an additional analysis for a prespecified subgroup of patients < 60 years. Successively, we used test for trend for assessing gene dose effect, i.e. a stepwise increasing phenotypic response with the presence of 0, 1 or 2 putative alleles. Statistical significance was accepted for P-values 0.05.

### Results

### Patients Characteristics

The observed IL-1RN genotypes in the study population were 1/1 in 954 (51.6%), 1/2 in 742 (40.1%) and 2/2 in 154 (8.3%). Thus, allele 2 frequency was 0.28. The observed distribution complied with Hardy-Weinberg equilibrium. Main baseline characteristics of the patients are listed in Table 6 and compared between carriers and noncarriers of the IL-1RN*2 allele. There was a trend to a higher frequency of diabetes and reduced left ventricular function among carriers of the IL-1RN*2 allele. The other characteristics were evenly distributed between the 2 groups. The angiographic and procedural characteristics at the time of intervention are listed in Table 7 and show no significant differences between carriers and noncarriers of the IL-1RN*2 allele.

**Table 5 Lesion and procedural characteristics at the time of intervention.**

| IL-1RN | 1/2 or 2/2 (n=896) | IL-1RN 1/1 (n=954) | **P** |
|---|---|---|---|
| **Target coronary vessels** | | | 0.89 |
| Left main - % | 1.3 | 1.6 | |
| LAD - % | 40.1 | 39.3 | |
| LCx - % | 19.9 | 20.0 | |
| RCA - % | 32.6 | 31.9 | |
| Venous bypass graft - % | 6.1 | 7.2 | |
| Complex lesions - % | 75.2 | 74.1 | 0.58 |
| Restenotic lesions - % | 25.3 | 23.3 | 0.30 |

| **Before stenting** | | | |
|---|---|---|---|
| Reference diameter, mm | 3.02±0.53 | 3.05±0.54 | 0.29 |
| Diameter stenosis - % | 79.1±14.9 | 78.7±15.7 | 0.57 |
| Lesion length - mm | 12.1±6.9 | 12.1±6.6 | 0.98 |
| Procedural data | | | |
| Measured balloon diameter - mm | 3.2±0.5 | 3.2±5 | 0.45 |
| Maximal balloon pressure - atm | 13.9±3.3 | 13.8±3.2 | 0.20 |
| Stented segment length - mm | 20.0±14.3 | 20.3±13.6 | 0.70 |

| **Immediately after stenting** | | | |
|---|---|---|---|
| Diameter stenosis - % | 5.2+9.1 | 5.4±7.6 | 0.47 |

| | | | |
|---|---|---|---|
| Data are proportions or mean±SD LAD indicates left anterior descending coronary artery; LCx, left circumflex coronary artery; RCA, right coronary artery; complex lesions were defined as ACC/AHA lesion types B2 and C, according to the American College of Cardiology/American Heart Association grading system. | | | |

### IL-1RN Polymorphism, Mortality and Myocardial Infarction After Stenting

Table 6 shows the adverse clinical events observed within the first 30 days after coronary stenting in carriers and noncarriers of the IL-1RN*2 allele. There was no association between the presence of the IL-1RN*2 allele and death, myocardial infarction or target vessel revascularization, showing no significant influence of the polymorphism in the IL-lra gene in the risk for early thrombotic events after coronary stenting.

**Table 6 Incidence of adverse events recorded during the early 30 days**

| | | IL-1RN 1/2 or 2/2 (n=896) | IL-1RN 1/1 (n=954) | **P** |
|---|---|---|---|---|
| Death - % | | 0.9 | 0.9 | 0.91 |
| Nonfatal myocardial infarction - % | | 3.3 | 2.6 | 0.52 |
| | - Q-wave - % | 1.1 | 0.7 | 0.39 |
| | - non-Q-wave - % | 2.2 | 1.9 | 0.60 |
| Target vessel revascularization - % | | 3.0 | 2.3 | 0.34 |

One-year follow-up indicated also that there is no correlation between the presence of the IL-1RN*2 allele and mortality or incidence of myocardial infarction after the intervention. During the 1-year period, mortality rate was 2.8% in the combined group of IL-1RN 1/2 and IL-1RN 2/2 patients and 2.2% in IL-1 1/1 patients (P=0.42), yielding an odds ratio of 1.28 (95% confidence interval, 0.71-2.29). The incidence of nonfatal myocardial infarction was 3.5% in IL-1RN*2 allele carriers and 3.9% in homozygous carriers of the IL-1RN*1 allele (P=0.54), and the respective odds ratio was 0.86 (0.53-1.4).

### IL-1RN Polymorphism and Restenosis After Stenting

Control angiography was performed in 84% of the patients after a median of 188 days (interquartile range, 171-205 days). The proportion of patients with control angiography was similar in the 2 groups defined by the presence or absence of the IL-1RN*2 allele. Table 7 lists the results of the quantitative assessment of 6-month angiograms.

**Table 7 Results at follow-up angiography.**

| | IL-1RN 1/2 or 2/2 (n=758) | IL-1RN 1/1 (n=798) | **P** |
|---|---|---|---|
| Late lumen loss - mm | 1.16±0.82 | 1.24±0.86 | 0.07 |
| Loss index | 0.53±0.38 | 0.59±0.45 | 0.009 |
| Diameter stenosis - % | 41.8±26.2 | 45.2±28.7 | 0.015 |
| Restenosis rate - % | 30.2 | 35.6 | 0.024 |

| | | | |
|---|---|---|---|
| Data are proportions or mean±SD | | | |

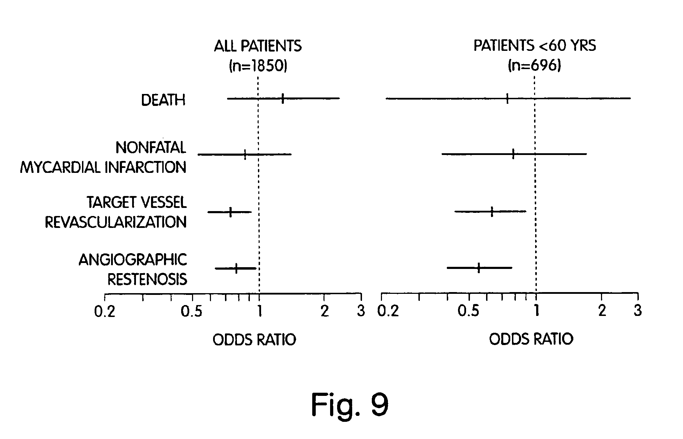

Of note, loss index which reflects the hyperplastic response after stenting was significantly lower in patients who carried the IL-1RN*2 allele. The incidence of angiographic restenosis was also significantly lower in carriers of the IL-1RN*2 allele, with 30.2% vs. 35.6% in patients of the IL-1RN 1/1 genotype. Thus, the presence of the IL-1RN*2 allele was associated with a 22% decrease in restenosis rate (odds ratio, 0.78 [0.63-0.97]; Figure 9, left panel). Clinical restenosis expressed as the need for target vessel revascularization was also significantly lower, with 17.7% in IL-1RN*2 allele carriers vs. 22.7% in homozygous patients for the IL-1RN*1 allele (P=0.026), yielding an odds ratio of 0.73 (0.58-0.92) as shown in Figure 9, left panel.

Age, gender, the presence or absence of diabetes, smoking habit, reduced left ventricular function and restenotic lesions, vessel size (all variables differing in univariate analysis by a P-value 0.30) were entered into the multivariate model for angiographic restenosis along with the presence or absence of the IL-1RN*2 allele. Older age (P=0.005), the presence of diabetes (P<0.001), restenotic lesion (P<0.001) and small vessel size (P<0.001) were independently correlated with an increased risk of restenosis. On the opposite, the presence of the IL-1RN*2 allele was independently (P<0.001) correlated with a decreased risk for restenosis with an adjusted odds ratio of 0.81 (0.71-0.92). In addition, there was a significant interaction between the presence of the IL-1RN*2 allele and age (P=0.009) as reflected by a progressively stronger protective effect of this allele in younger patients.

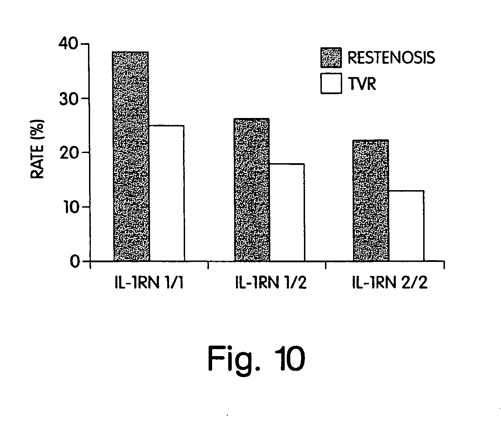

The results of the analysis in the prespecified subgroup of patients < 60 years (n=696) are presented in Table 8, Figure 9, right panel and Figure 10. During the 1-year follow-up period, 17.1% of the IL-1RN*2 allele carriers and 24.9% of the homozygous IL-1RN*1 allele carriers needed target vessel revascularization (P=0.013). Thus, the presence of the IL-1RN*2 allele was associated with a 37% reduction (odds ratio: 0.63 [0.43-0.91]; Figure 1, right panel) of the need of ischemia-driven reinterventions. Quantitative angiographic data obtained for the control study at 6 months (performed in 590 or 85% of patients < 60 years) are displayed in Table 8.

**Table 8 Results at follow-up angiography in patients < 60 years.**

| | IL-1RN 1/2 or 2/2 (n=273) | IL-1RN 1/1 (n=317) | **P** |
|---|---|---|---|
| Late lumen loss - mm | 1.08±0.77 | 1.27±0.93 | 0.008 |
| Loss index | 0.49±0.35 | 0.59±0.48 | 0.003 |
| Diameter stenosis - % | 39.3±24.1 | 46.7±30.5 | 0.001 |
| Restenosis rate - % | 25.6 | 38.5 | <0.001 |

| | | | |
|---|---|---|---|
| Data are proportions or mean±SD | | | |

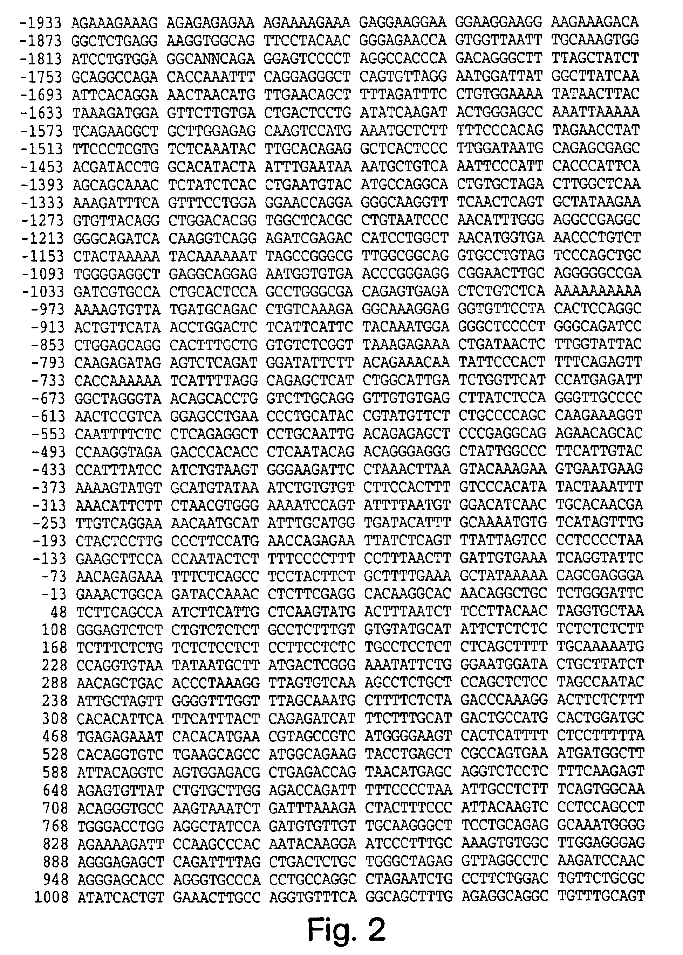

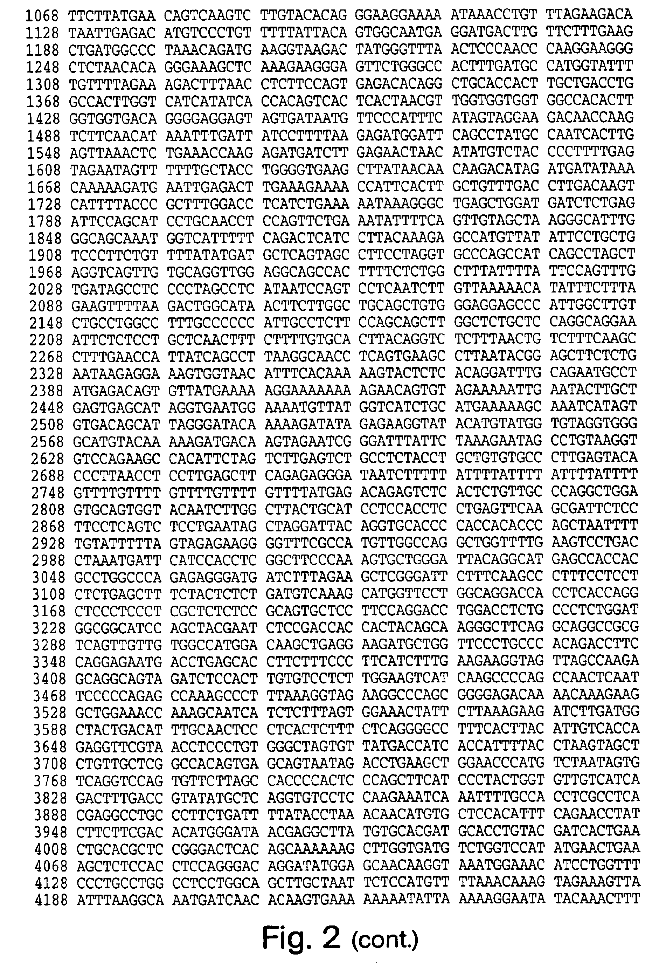

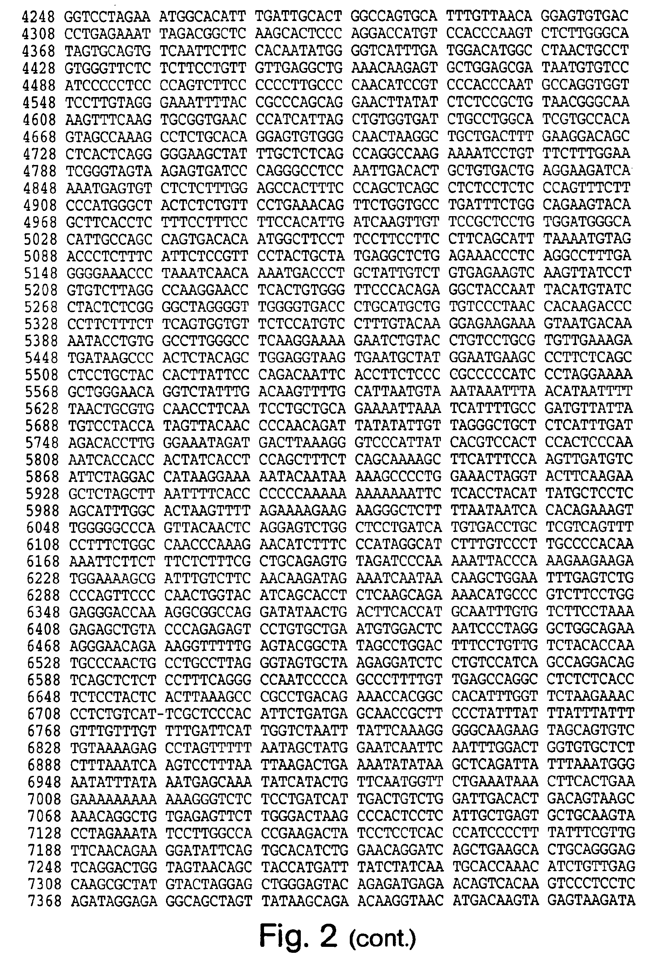

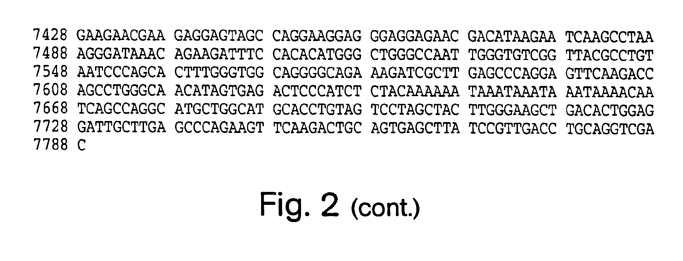

The incidence of angiographic restenosis was 25.6% in the combined group of IL-1RN 1/2 and IL-1RN 2/2 patients and 38.5% among IL-1RN 1/1 patients (P<0.001), which corresponds to a 45% reduction (odds ratio: 0.55 [0.39-0.78]; Figure 1, right panel). Figure 2 illustrates the gene dose effect verified in the subgroup of younger patients. The incidence of restenosis decreased progressively with heterozygosity and homozygosity for the IL-1RN*2 allele. The rate of angiographic restenosis was 38.5% in IL-1RN 1/1 patients, 26.3% in IL-1RN 1/2 patients and 22.4% in IL-1RN 2/2 patients (P=0.001, test for trend). The target vessel revascularization rate was 24.9% in IL-1RN 1/1 patients, 17.9% in IL-1RN 1/2 patients and 13.2% in IL-1RN 2/2 patients (P=0.01, test for trend; Figure 2).

### 5.3. Example 3: The IL-1 Haplotype Patterns Associated with Occlusive Cardiovascular Disorders and Periodontitis

The association between periodontitis, cardiovascular disease and four basic biallelic markers (IL-1A (+4845), IL-1B (+3954), IL-1B (-511), and IL-1RN (+2018)) in the interleukin-1 (IL-1) gene cluster on chromosome 2 was investigated.

Two haplotype patterns may be defined by four polymorphic loci in the IL-1 gene cluster as shown in Table 9 (IL-1A(+4845), IL-1B(+3954), IL-1B (-511), IL-1RN(+2018)). One pattern includes allele 2 at both the IL-1A (+4845) and at the IL-1B (+3954) loci. The other pattern includes allele 2 at both the IL-1B(-511), and at the IL-1RN(+2018) loci.

**Table 9**

| Haplotypes | IL-1A (+4845) | IL-1B (+3954) | IL-1B (-511) | IL-1RN (+2018) |
|---|---|---|---|---|
| Pattern 1 | Allele 2 | Allele 2 | Allele 1 | Allele 1 |
| Pattern 2 | Allele 1 | Allele 1 | Allele 2 | Allele 2 |

The haplotype pattern indicates that when allele 2 is found at one locus, it is highly likely that it will be found at other loci. Previous data (Cox et al. (1998) Am. J. Hum. Genet. 62:1180-1188) indicate that when allele 2 is found at the IL-1A (+4845) locus allele 2 will also be present at the IL-1B (+3954) locus approximately 80% of the time. Haplotype patterns are relevant only for a single copy of a chromosome. Since there are two copies of chromosome 2 and standard genotyping procedures are unable to identify on which chromosome copy a specific allele is found, special statistical programs are used to infer haplotype patterns from the genotype pattern that is determined.

The distribution of these genetic patterns was evaluated in a new population that was part of a study of atherosclerosis (Pankow et al. (1999) The ARIC study. European Atherosclerosis Society Annual Meeting, Abstract, #646). In this population (N=1,368), IL-1A(+4845) genotype 2.2 was found in 10.2% of the subjects. However, in the subjects with genotype IL-1B (+3954) = 2.2 (N=95), the IL-1A (+4845) genotype 2.2 was found in 71.6% of the subjects. This indicates that allele 2 at IL-1A (+4845) is inherited together with allele 2 at IL-1B (3954) at a much higher rate than one would expect given the distribution of each of these markers in the population. Similar data exists for allele 2 at the 2 loci that are characteristic of Pattern 2. In addition, when genotype Pattern 1 is found it is highly unlikely that allele 2 will be present at either of the loci that are characteristic of the other pattern.

The two genotype patterns are also associated with specific differences in the functional biology of interleukin-1. For example, peripheral monocytes from individuals with one or two copies of allele 2 at IL-1B (+3954) produced 2 to 4 times as much IL-1β when stimulated with LPS as monocytes from individuals who have the genotype pattern IL-1B (+3954)=1.1 (DiGiovini, FS et al. (1995) Cytokine, 7:606). Similar data have recently been reported for peripheral blood polymorphonuclear leukocytes isolated from individuals with severe periodontitis (Gore, EA et al. (1998) J. Clin. Periodontol., 25:781). In addition gingival crevice fluid (GCF) from subjects with the composite genotypes indicative of Pattern 1 have 2 to 3 times higher levels of IL-1β than GCF from individuals who are negative for those genotypes (Engelbretson, SP et al. (1999) J. Periodontol., in press). There are also data indicating that for Pattern 2, allele 2 at IL-1RN +2018 is associated with decreased levels of IL-1 receptor antagonist protein. Thus, Pattern 1 genotypes appear to be associated with increased IL-1 agonists, and Pattern 2 appears to be associated with decreased levels of IL-1 receptor antagonist.

The composite IL-1 genotypes that are consistent with Pattern 1 are associated with increased susceptibility to severe adult periodontitis (Kornman, KS et al. (1997), supra; Gore, EA et al. (1998), supra; McGuire, MK et al. (1999) J. Periodontol., in press; McDevitt, MJ et al. (1999) J. Periodontol., in press). One aspect of the IL-1 genotype influence on periodontitis appears to be an enhancement of the subgingival levels of specific bacterial complexes that include accepted periodontal pathogens (Socransky, SS et al. (1999) IADR Annual Meeting, Abstract#3600). Pattern 1 genotypes were not, however, associated with increased risk for occlusive cardiovascular disease. In data from the Atherosclerosis Risk in Communities (ARIC) study that was presented by Pankow and co-workers (see Pankow et al., supra), individuals with ultrasound measurements of carotid wall intima-medial thickness (IMT) that were indicative of occlusive cardiovascular disorders were compared to a stratified random control population for IL-1 gene polymorphisms. Neither IL-1A (+4845) or IL-1B (+3954) showed any association with risk for high IMT.

Genotypes that are characteristic of pattern 2 have recently been associated with increased susceptibility to occlusive coronary artery disease, but not increased risk for periodontitis. In a report on coronary artery disease, patients with angiographic evidence of coronary stenoses were significantly more likely to be carriers of allele 2 at either the IL-1RN (+2018) locus or the IL-1B (-511) locus (see Francis et al., supra). Both loci are characteristic of the haplotype Pattern 2. In the ARIC study, as discussed above, carriage of IL-1RN (+2018) allele 2 in African-Americans with high IMT measurements was significantly higher than ethnically matched controls. In Caucasians with high IMT measurements the carriage of one copy of allele 2 at IL-1RN (+2018) was significantly greater than in controls, however individuals homozygous at this locus were not different from controls. It should be noted that the prevalence of individuals homozygous for allele 2 at IL-1RN (+2018) in Caucasians in the study was substantially lower than that observed in other populations.

When individuals with periodontitis and gingival health were evaluated for genotype patterns consistent with Pattern 1 and Pattern 2, individuals with severe adult periodontitis were found to have a predominance of genotypes consistent with Pattern 1, whereas individuals with a healthy periodontal condition had genotype patterns that were dominated by neither Pattern 1 nor Pattern 2. It appears therefore that IL-1 genotypes consistent with the haplotype Pattern 1 are associated with severe periodontitis and plaque fragility disorders and not occlusive cardiovascluar diseases whereas IL-1 genotypes consistent with the haplotype Pattern 2 are associated with occlusive cardiovascular diseases but not periodontitis or plaque fragility. One mechanism may be that IL-1 genotype Pattern 1 directly influences plaque fragility; another mechanism may be that Pattern 1 influences periodontitis directly, which may lead to indirect influences on cardiovascular disease through the periodontal micororganisms found as part of the oral chronic inflammatory process. Another mechanism may be that IL-1 genotype Pattern 2 directly influences cardiovascular occlusive disorders but has no influence on periodontitis. It is thus likely that IL-1 genetic polymorphisms can influence both cardiovascular disease and severe periodontitis, by a common underlying mechanism that directly alters the immunoinflammatory responses in both diseases in an identical fashion and by an indirect mechanism that enhances the oral bacterial load and then influences cardiovascular disease. The IL-1 genotypes that are consistent with haplotype Pattern 1 may influence the association between periodontidis and cardiovascular disease in one segment of the population by amplifying both the immuno-inflammatory response and the subgingival bacterial load.

### 5.4 Example 4 Genotyping Methods

### Preparation of DNA

Blood is taken by venipuncture and stored uncoagulated at -20°C prior to DNA extraction. Ten milliliters of blood are added to 40 ml of hypotonic red blood cell (RBC) lysis solution (10 mM Tris, 0.32 Sucrose , 4 mM MgCl_{2,} 1% Triton X-100) and mixed by inversion for 4 minutes at room temperature (RT). Samples are then centrifuged at 1300 g for 15 minutes, the supernatant aspirated and discarded, and another 30 ml of RBC lysis solution added to the cell pellet. Following centrifugation, the pellet is resuspended in 2ml white blood cell (WBC) lysis solution (0.4 M Tris, 60 mM EDTA, 0.15 M NaCl, 10% SDS) and transferred into a fresh 15 ml polypropylene tube. Sodium perchlorate is added at a final concentration of 1M and the tubes are first inverted on a rotary mixer for 15 minutes at RT, then incubated at 65°C for 25 minutes, being inverted periodically. After addition of 2 ml of chloroform (stored at-20° C), samples are mixed for 10 minutes at room temperature and then centrifuged at 800 G for 3 minutes. At this stage, a very clear distinction of phases can be obtained using 300 1 Nucleon Silica suspension (Scotlab, UK) and centrifugation at 1400 G for 5 minutes. The resulting aqueous upper layer is transferred to a fresh 15 ml polypropylene tube and cold ethanol (stored at-20° C) is added to precipitate the DNA. This is spooled out on a glass hook and transferred to a 1.5 ml eppendorf tube containing 5001 TE or sterile water. Following overnight resuspension in TE, genomic DNA yield is calculated by spectrophotometry at 260 nm. Aliquots of samples are diluted at 100 ug/ml, transferred to microtiter containers and stored at 4°C. Stocks are stored at -20°C for future reference.

### 5.4.1 Polymerase Chain Reaction

Oligonucleotide primers designed to amplify the relevant region of the gene spanning the polymorphic site (as detailed below) are synthesized, resuspended in Tris-EDTA buffer (TE), and stored at -20°C as stock solutions of 200 uM. Aliquots of working solutions (1:1 mixture of forward and reverse, 20 µM of each in water) are prepared in advance.

Typically, PCR reaction mixtures are prepared as detailed below.

| | **Stock Concentration** | **Volume** | **Final Concentration** |
|---|---|---|---|
| Sterile H₂0 | | 29.5 µl | |
| 10xPCR buffer | 200 mM Tris-HCl (pH 8.4) | 5.00 µl | 20 mM Tris-HCI, |
| MgCl₂ dNTP | 50 mM | 1.75 µl | 1.75 mM |
| | mix 10 mM of each | 4.00 µl | 0.2 mM of each |
| primer forward prime reverse | 20 uM | 2.5 µl | 1 uM |
| | 20 uM | 2.5 µl | 1 uM |
| *Taq* polymerase | 5 U / µl | 0.25 µl | 1.25 units/50 µl |
| Detergent (eg W-1, Gibco) | 1% | 2.5 µl | 0.05% |
| Template | 200 ng/µl | 2.00 µl | 2 ng/ l |
| Final Volume | | 50.00 µl | |

DNA template is dotted at the bottom of 0.2 ml tubes or microwells. The same volume of water or negative control DNA is also randomly tested. A master-mix (including all reagents except templates) is prepared and added to the wells or tubes, and samples are transferred to the thermocycler for PCR.

PCR can be performed in 0.5 ml tubes, 0.2 ml tubes or microwells, according to the thermocycler available. The reaction mixture is overlaid with mineral oil if a heated lid (to prevent evaporation) is not available.

### 5.4.2 Restriction Enzyme Digestion

A master mix of restriction enzyme buffer and enzyme is prepared and aliquotted in suitable volumes in fresh microwells. Digestion is carried out with an oil overlay or capped microtubes at the appropriate temperature for the enzyme on a dry block.

Restriction buffer dilutions are calculated on the whole reaction volume (i.e. ignoring salt concentrations of PCR buffer). Restriction enzymes are used 3-5 times in excess of the recommended concentration to compensate for the unfavorable buffer conditions and to ensure complete digestion.

### 5.4.3 Electrophoresis

Polyacrylamide-gel electrophoresis (PAGE) of the PCR sample is carried out in Tris-HCI-EDTA buffer and at constant voltage. Depending on the size discrimination need, different PAGE conditions are used (9 to 12% acrylamide, 1.5 mm x 200) and different DNA size marker (X174-Hae III or X 174-Hinf 1). A 2% agarose horizontal gel can be used for genotyping the IL-1RN (VNTR) marker.

### 5.4.4 Allele Detection Methods

The following Table 10 provides methods for detecting particular alleles that are associated with the existence of or susceptibility to developing restenosis.

**TABLE 10**

| | **IL-1A (+4845)** | | |
|---|---|---|---|
| **5' Primer** | ATG.GTT.TTA.GAA.ATC.ATC.AAG.CCT.AGG.GCA (+4814/+4843) (SEQ ID No. 1) | | |
| **3' Primer** | AAT.GAA.AGG.AGG.GGA.GGA.TGA.CAG.AAA.TGT (+5015/+5044) (SEQ ID No. 2) | | |
| **PCR Conditions** | MgCl₂ is used at 1 mM final, and PCR primers at 0.8 mM. DMSO is added at 5%, DNA template at 150ng/50 ml, and TaqMan 1.25u/50µl. | | |
| **Cycling conditions** | 1X [95°C 1 min.]; 35X [94°C 1 min., 56°C 1 min., 72°C 2 min.]; 1X [72°C 5 min.]; 4°C. | | |
| **Analysis** | Cleavage with 2.5 units of Fnu4H1 in addition to 2 ml of the specific 10 restriction buffer at 37 °C overnight, followed by 9% PAGE analysis yields a constant band of 76 bp (absence indicates incomplete digestion) and two further bands of 29 and 124 bp (allele 1) , or a single band of 153 bp (allele 2). Allele frequencies in North British Caucasian population are 0.71 and 0.29. | | |
| **Reference** | Gubler, et al.(1989) Interleukin, inflammation and disease (Bomford and Henderson, eds.) p.31-45, Elsevier publishers; and Van den velden and Reitsma (1993) Hum Mol Genetics 2:1753-50). GenBank Accession No. X03833. | | |

| | **IL-1B (-511)** | | |
|---|---|---|---|
| **5' Primer** | TGG.CAT.TGA.TCT.GGT.TCA.TC (-702/-682) (SEQ ID No: 3) | | |
| **3' Primer** | GTT.TAG.GAA.TCT.TCC.CAC.TT (-417/-397) (SEQ ID No: 4) | | |
| **PCR Conditions** | 50 mM KCI, 10 mM Tris-HCI, pH 9.0, 1.5 mM MgCl₂,200 mM dNTPs, 25 ng primers, 50 ng template, 0.004% W-1 (Gibco-BRL), 0.2 U Taq polymerase, 50 µl total volume | | |
| **Cycling conditions** | 1X [95°C 2 min.]; 35X [95°C 1 min., 53°C 1 min., 72°C 1 min.];1X [72°C, 5 min.]; 4°C. | | |
| **Analysis** | Each PCR reaction is divided into two 25 µl aliquots: one is added of 3 units of Ava I restriction endonuclease, the other 3.7 units of Bsu 36 I, in addition to 3 µl of the specific 10x restriction buffer. Incubation is at 37°C overnight. Electrophoresis is by PAGE 9%. Cleavage with *Ava* I and *Bsu* 36I. **Allele 1** (C) produces 190 and 114 bp fragments when digested with Ava I and a 304 bp fragment when digested with *Bsu* 36I*.* **Allele 2** (T) produces a 304 bp fragment when digested with *Ava* I and 190 and 114 bp fragments when digested with *Bsu* 36I. The restriction pattern obtained should be the inverse in the two aliquots (identifying homozygotyes) or identical (heterozygotes). Frequencies in North British Caucasian population are 0.61 and 0.39 for allele 1 and 2 respectively. | | |
| **Reference** | diGiovine, Hum. Molec. Genet., 1(6):450 (1992); Clark, et al., Nucl. Acids. Res., 14:7897-7914 (1986) [published erratum appears in Nucleic Acids Res., 15(2):868 (1987)]; GenBank Accession No. X04500. | | |

| | **IL-1B (+3954)** | | |
|---|---|---|---|
| **5' Primer** | CTC.AGG.TGT.CCT.CGA.AGA.AAT.CAA.A (+3844/+3868) (SEQ ID No: 5) | | |
| **3' Primer** | GCT.TTT.TTG.CTG.TGA.GTC.CCG (+4017/+4037) (SEQ ID No: 6) | | |
| **PCR Conditions** | 50 mM KCI, 10 mM Tris-HCl, pH 9.0, 1.5 mM MgCl₂,200 mM dNTPs, 25 ng primers, 50 ng template, 0.004% W-1 (Gibco-BRL), 0.2 U Taq polymerase, 50 µl total volume | | |
| **Cycling conditions** | 1X [95°C 2 min.]; 35 X [95°C 1 min., 67.5°C 1 min., 72°C 1 min]; 1X [72°C, 5 min.]; 4°C. | | |
| **Analysis** | Each PCR reaction is added of 10 u of Taq 1 restriction endonuclease in addition to 3 µl of the specific 10x restriction buffer. Incubation is at 65°C overnight. Electrophoresis is by PAGE 9%. Following digestion with *Taq* I, **Allele 1** produces 97, 85 and 12 bp fragments; **Allele 2** produces 182 and 12 bp fragments. The absence of the 12 bp band indicates incomplete digestion. Frequencies in a North British Caucasian population are 0.82 (allele 1) and 0.18 (allele 2). For 90% power at 0.05 level of significance in a similar genetic pool, 408 cases should be studied to detect 1.5 fold increase in the frequency, or 333 for 0.1 absolute increase in frequency. | | |

| **Reference** | di Giovine, et al. Cytokine 7(6): 606 (1995) | | |
|---|---|---|---|
| | **IL-1RN (VNTR)** | | |
| **5' Primer** | CTC.AGC.AAC.ACT.CCT.AT (+2879/+2895) (SEQ ID NO. 7) | | |
| **3' Primer** | TCC.TGG.TCT.GCA.GGT.AA (+3274/+3290) (SEQ ID NO. 8) | | |
| **PCR Conditions** | 50 mM KCI, 10 mM Tris-HCI pH 9.0, 1.7 mM MgCl₂, 200 mM dNTPs, 25 ng primers, 50 ng template, 0.004% W-1 (Gibco-BRL) 0.2u Taq polymerase | | |
| **Cycling conditions** | 1 X [96°C for 1min.]; 30 X [94°C for 1min., 60°C for 1min., 70°C for 1min.]; 1 [70°C for 2min.]. | | |
| **Analysis** | The variable number of tandem repeats (VNTR) in intron 2 of IL1-RN corresponds to a variable number (2 to 6) of an 86 bp repeat and so the PCR product sizes are a direct indication of the number of repeats. Electrophoresis is by 2% agarose, 90V, 30 min. | | |
| | **Allele 1** | 4 repeats | 412 bp PCR product |
| | **Allele 2** | 2 repeats | 240 bp PCR product |
| | **Allele 3** | 3 repeats | 326 bp PCR product |
| | **Allele 4** | 5 repeats | 498 bp PCR product |
| | **Allele 5** | 6 repeats | 584 bp PCR product |
| | Frequencis in a North British Caucasian population for the four most frequent alleles are 0.734, 0.241, 0.021 and 0.004. | | |
| **Reference** | Steinkasserer et al. (1991) Nucleic Acids Research 19: 5090-95; Tarlow, et al., Hum. Genet. 91: 403-4 (1993) | | |
| | | | |

| **IL-1RN (+2018)** | | | |
|---|---|---|---|
| **5' Primer** CTA.TCT.GAG.GAA.CAA.CCA.ACT.AGT.AGC-3' (+1992/+2017) (SEQ ID No. 9) | | | |
| **3' Primer** TAG.GAC.ATT.GCA.CCT.AGG.GTT.TGT-3' (+2135/+2158) (SEQ ID No. 10) | | | |
| **PCR Conditions** Each PCR reaction is divided in two 25µl aliquots; to one is added 5 Units of *Alu I*, the other 5 Units of *Msp I*, in addition to 3µl of the specific 10X restriction buffer. Incubation is a 37°C overnight. Electrophoresis is by PAGE 9%. | | | |
| **Cycling conditions** 1 X [96°C for 1 min]; 35 X [94°C for 1, min., 57°C for 1 min 70°C for 2 min.]; 1 X [70° for 5 min.]; 4°C. | | | |
| **Allele Detection** | The above described PCR primers incorporate mismatches to the genomic sequence so as to engineer two different restriction sites on the alleles. The two alleles are 100% in linkage disequilibrium with the two most frequent alleles of IL-1RN (VNTR). *Alu I* will produce 126 + 28 bp fragments for **Allele 1,** while it does not digest **Allele 2** (154 bp). *Msp I* will produce 125 + 29 bp with **Allele 2,** while **Allele 1** is uncut (154 bp). Hence the two reactions (separated side by side in PAGE) will give inverted patterns of digestion for homozygote individuals, and identical patterns in heterozygotes. Allelic frequencies in a North British Caucasion population are 0.74 and 0.26. For 90% power at 0.05 level of significance in a similar genetic pool, 251 cases should be studied to detect 1.5 fold increase in frequency, or 420 for 0.1 absolute increase in frequency. | | |
| **Reference** | Clay, et al.(1996) Hum. Genet. 97: 723-26. | | |

Results: Typing of additional numbers of individuals is required to bring the results to significance, but preliminary results indicate that allele 2 of the 4845, -511, +3954 and VNTR markers in the IL-IRN gene will be over-represented in restenosis. It is predicted that individuals with at least one copy of allele 2 from one of the above markers are more likely to have restenosis than those who are negative for allele 2. Individuals who are homozygous for any of these alleles, or have allele 2 from more than one marker are estimated to have even higher risk for restenosis.

### 5.5 Example 5

In this example, the preparation of template DNA is described. PCR-based genotyping does not require particularly high-MW DNA (< 20 Kb DNA is often an excellent template). As 100 ng genomic DNA is more than sufficient for single-copy gene amplification, direct amplification from dried blood spots or cell lysates can be used for genotyping, and two of the protocols that we have used are here described below.

However, if DNA banks need to be established for population studies where DNA needs to be stored for future reference or genotyping at different loci, or where genomic Southern blotting might be needed, good quality high-MW genomic DNA needs to be extracted. Basic buffers and the composition of chemical solutions can be found in major protocol textbooks (Sambrook et al. (1989) Molecular cloning: a laboratory manual, Cold Spring Harbor Press; Ausubel and Frederick (1994) Current protocols in molecular biology, John Wiley and Sons).

Sample DNA can also be obtained from dried blood spots. Such a means of sample collection (Guthrie spots) has been used for many years in neonatal diagnosis of phenylketonuria. In the last few years dried blood spots have proved useful in PCR-based diagnostics (Raskin et al. (1991) Am J Hum Genet 49: 320-29). Uncoagulated blood is spotted evenly using a sterile Pasteur pipette onto a clean sheet of filter paper. This is left to dry overnight in a clean area (physically isolated from post-PCR events) and stored subsequently at room temperature.

For PCR, a mastermix is prepared as described later in this chapter, where *Taq* polymerase is omitted. This is aliquotted in reaction tubes, and approximately 1mm² of the blood spot is cut out and placed into the reaction mix. This is overlaid with 40 µl mineral oil. The lid of each tube is pierced with a sterile needle, and samples are then heated at 98 °C for 15 minutes. Following cooling for a few minutes, Taq polymerase is added and standard PCR cycling follows.

Sample DNA can also be obtained from cell lysates. White blood cells, buccal cells or homogenised tissue is suspended in PK buffer (0.1M NaCl, 10 mM Tris-HCl, 25 mM EDTA, 0.5% SDS pH 8.0, 0.1 mg/ml fresh Proteinase K) and incubated on a tumbler at 37°C for 1 hour. Samples are heated at 95°C for 10 mins, spun at 13,000 rpm in a microfuge and supernatants stored at -20°C prior to PCR. For higher quality DNA, a phenol/chloroform extraction followed by ethanol precipitation can be added.

Sample genomic DNA can also be obtained from whole blood. Blood is taken by venepuncture and stored uncoagulated at -20°C prior to DNA extraction. When possible, we prefer to collect two 10 ml samples, extract DNA form the first and keep the second for future reference. Ten milliliters of blood are added to 40 ml of hypotonic red blood cell (RBC) lysis solution (10mM Tris-HCl, 0.32 Sucrose, 4mM MgCl₂, 1% Triton X-100) and mixed by inversion for 4 minutes at room temperature. Samples are then centrifuged at 1300g for 15 minutes, the supernatant aspirated and discarded, and another 30 ml of RBC lysis solution added to the cell pellet. Following centrifugation, the pellet is resuspended in 2ml white blood cell (WBC) lysis solution (0.4M Tris-HCl, 60mM EDTA, 0.15M NaCl, 10% SDS) and transferred into a fresh 15ml polypropylene tube. Sodium perchlorate is added at a final concentration of 1M and the tubes are first inverted on a rotary mixer for 15 minutes at room temperature (RT), then incubated at 65°C for 25 minutes, being inverted periodically. After addition of 2ml of chloroform (stored at -20°C), samples are mixed for 10 minutes at room temperature and then centrifuged at 800g for 3 minutes. At this stage a very clear distinction of phases can be obtained using 300µl Nucleon Silica suspension (Scotlab, UK) and centrifugation at 1400 G for 5 minutes. The resulting aqueous upper layer is transferred to a fresh 15ml polypropylene tube and cold ethanol (stored at -20°C) is added to precipitate the DNA. This is spooled out on a glass hook and transferred to a 1.5ml eppendorf tube or containing 500µl TE or sterile water. Following overnight resuspension in TE, genomic DNA yield is calculated by spectrophotometry at 260nm. Aliquots of samples are diluted at 100µg/ml, transferred to microtiter containers and stored at 4°C. Stocks are stored at -20°C for future reference.

### 5.6 Example 6

In this example, the conditions for conducting appropriate polymerase chain reactions on the collected samples are described. Oligonucleotide primers designed to amplify the relevant region of the gene spanning the polymorphic site (as detailed below) are synthesised, resuspended in Tris-HCl-EDTA buffer (TE) and stored at -20°C as stock solutions of 200 µM. Aliquots of working solutions (1:1 mixture of forward and reverse, 20µM of each in water) are prepared in advance of the experiment. Typically PCR reaction mixtures are prepared as detailed below. Divergence from the scheme below can be made for each specific protocol.

| | **Stock Concentration** | **Volume** | **Final Concentration** |
|---|---|---|---|
| Sterile H₂0 | | 29.5 µl | |
| 10xPCR buffer | 200mM Tris-HCl (pH | 5.00 µl | 20mM Tris-HCl, |
| | 8.4), 500mM KCl | | 50mM KCl |
| MgCl₂ | 50mM | 1.75 µl | 1.75 mM |
| dNTP | mix 10mM of each | 4.00 µl | 0.2 mM of each |
| primer forward | 20 µM | 2.5 µl | 1 µM |
| primer reverse | 20 µM | 2.5 µl | 1 µM |
| Taq polymerase | 5 U / µl | 0.25 µl | 1.25 units/50 µl |
| Detergent (eg W-1 Gibco) | 1% | 2.5 µl | 0.05% |
| Template Final volume | 200 ng/µl | 2.00 µl 50.00 µl | 2ng/µl |

DNA template is dotted at the bottom of 0.2 ml tubes or microwells. The same volume of water or negative control DNA is also randomly tested. A master-mix (including all reagents except templates) is prepared and added to the wells or tubes, and samples are transferred to the thermocycler for PCR.

PCR can be performed in 0.5 ml tubes, 0.2 ml tubes or microwells, according to the thermocycler available and to the needs of the project. The reaction mixture is overlaid with mineral oil if a heated lid (to prevent evaporation) is not available. We use 96-well format microplates, because they allow use of multichannel pipettes both for transfer of template DNA (stored in 1 ml/microwell plates) and for dispensing of the reaction mastermix.

### 5.7 Example 7

In this example, the conditions for conducting appropriate polymerase chain reactions on the collected samples are described. A master mix of restriction enzyme buffer and enzyme is prepared and aliquotted in suitable volumes in fresh microwells. We use a multichannel pipette to transfer and mix 25-30 µl of PCR product in the microwells. Digestion is carried out with an oil overlay or capped microtubes at the appropriate temperature for the enzyme on a dry block. Restriction buffer dilutions are calculated on the whole reaction volume (i.e. ignoring salt concentrations of PCR buffer). Restriction enzymes are used 3-5 times in excess of the recommended concentration, to compensate for the unfavorable buffer conditions and to ensure complete digestion.

### 5.8 Example 8

In this example, the conditions for conducting gel electorphoresis analysis of the products of pcr amplification and restriction endonuclease digestion are considered. Polyacrylamide-gel electrophoresis (PAGE) of 20-40 µl PCR sample is carried out in Tris-HCl-EDTA buffer and at constant voltage. Depending on the size discrimination needed, different PAGE conditions are used (9 to 12% acrylamide, 1.5 mm x 200) and different DNA size markers (ϕX174*-Hae III* or ϕX 174*-Hinf I*). A 2% agarose horizontal gel can be used for IL-1RN (VNTR).

### 5.9 Example 9

In this example, quality controls for these genotyping protocols are considered. Incomplete digestion is the most common cause of mis-typing in PCR-RFLP genotyping methods. Most of the protocols described herein are based on a double-cut strategy, for which either a second restriction cutting site is used for digestion control on the diagnostic cleavage, or one enzyme cuts one allelic DNA form, and a different enzyme cuts the other allele. In this case each reaction is the control for the other. PCR conditions are tested (and, if necessary, re-optimised) for each DNA preparation not performed in our laboratory. Template DNA quality is assessed by spectrophotometry and by gel electrophoresis.

The possibility of cross-contamination is very high in PCR-based techniques. Although the genotyping is physically separated from any lab where relevant cloned fragments are being handled, it is still possible to have PCR-product carryover from previous experiments (from labcoat, hair, skin, etc.). A "PCR-carryover prevention kit" is available from Perkin-Elmer. This is based on UNG treatment of samples prior to PCR, which will cleave all dUTP-containing DNA. As all PCRs are performed using dUTP instead of dTTP, all previous PCR products, but not native templates, will be cleaved in this digestion step. This enzyme is inactivated by the first temperature ramping (94°C) and therefore normal PCR can take place without UNG activity. If laboratories do not use this system (which is expensive), there are stringent rules that can be used to reduce the risk of artefacts due to contamination.

### 5.10 Example 10

In this example, the prevention of contamination in these genotyping protocols is considered. Incomplete digestion is the most common cause of mis-typing in PCR-RFLP genotyping methods.

Laboratories are divided into GREEN (Pre-PCR) and RED (Post-PCR) areas. All laboratories have dedicated white coats, and workers are encouraged to change lab gloves as frequently as possible. GREEN laboratories have the most stringent requirements. Only goods coming from other green areas can enter, anything (equipment included) that leaves them cannot re-enter. These usually include a store-room, a "sample reception" area, a "clean DNA room" (where DNA extraction and PCR preparation are performed) and offices. RED laboratories have open access, but material and equipment can only move to other red areas or disposed of in bags for autoclaving or incineration. Red areas are where PCR and electrophoresis take place. Results and images are stored in computer files and transferred to the offices by local network.

All PCR's carry 10% negative controls which are randomly placed within the experiment. These are routinely represented by water controls. In the case of amplicards, negative controls are represented also by fragments (2-3-mm²) of paper from the edge of the card. For human blood DNA preparations, murine T cell lysates are extracted at the same time as each new batch of frozen blood, and resulting DNA used as negative control.

### 5.11 Example 11

In this example, the design of human polymorphic marker association studies are examined and the resulting data is analyzed. Traditional parametric analyses (requiring the specification of a distribution and/or the mode of inheritance) have been used successfully to locate genes for monogenic diseases following simple Mendelian modes of inheritances. More commonly used in the genetic analysis of complex diseases are non-parametric methods since these work independently of inheritance specifications, and are generally more powerful than parametric methods when parameters are mis-specified. The choice of method of analysis depends on whether the investigator wishes to perform a whole genome screen or use a candidate gene approach, since certain methods are best suited to just one of these two approaches or to specific pedigree structures. The following sections contain an outline of most commonly used non-parametric methods of analysis and their suitability to the candidate gene approach.

An allele at a certain locus is said to be associated with a disease if the frequency for that allele is significantly increased in the disease population over that of the normal healthy control population. True associations are due to linkage disequilibrium, where the disease causing allele at the 'disease' locus remains on the same haplotype as those alleles which were present at closely flanking loci when the ancestral mutation occurred. Thus, the frequency of any allele on the 'disease haplotype' (including, of course, the disease allele itself) will be increased in the disease population. Recombination over extremely small distances is very low, but as the time from the ancestral mutation increases, the distance over which linkage disequilibrium acts decreases reducing the length of the 'disease haplotype'. It is therefore easier to detect association in young, isolated populations with a single founder mutation effect where linkage extends over larger distances, than in large mixed populations.

Association studies are at present only suited to the candidate gene approach due to the small distances over which associations are detectable. In the future it is proposed that genome-wide association studies will be performed using several biallelic markers in every gene. Care must be taken when selecting the disease population in an association study, since spurious positive results may occur as an artefact of population admixture. It is usually advisable to investigate within a single ethnic group, since allele frequencies may vary between different groups. Similarly, if a control population is needed, it must be matched to the disease group for ethnicity, and ideally sex and age.

Case control studies can be performed for both qualitative and quantitative phenotypes. Obvious advantages of this approach include the ease of collection of large populations, the possibility of recruitment of patients with "early disease" phenotypes, and the possibility of analyzing late-onset diseases, where parental DNA may not be available.

For qualitative phenotypic studies, the candidate gene locus, allele frequencies or alternatively genotype frequencies, within the disease and control populations are calculated. The analysis is simple, comprising of a 2 x n contingency table (n denoting the number of categories, 2 for allele frequencies or 3 for genotypes at a biallelic locus), which a chi-square test may be used to determine whether the proportions differ significantly between the disease and control populations.

For quantitative phenotypic studies looking for a disease susceptibility allele, the individuals in both populations are first phenotyped quantitatively (usually the disease is classified as attaining a certain threshold value, therefore the unaffected controls are individuals failing below this). All individuals are then subdivided into the three (or more) genotypes. If an allele responsible for the inflated phenotype value of the diseased individuals exists, it would be expected that these individuals carry at least, one copy of it. Thus the median of these genotype groups would be higher than those of the non-carrier groups. The non-parametric test involves testing for significant difference between the medians of the different genotype (or carriage) groups. This may be done via a Mann-Whitney test (for 2 groups), or a Kruskall-Wallis (for >2 groups), although several other tests also exist. In exactly the same way, this type of analysis may also be performed solely within the disease group.

For use of qualitative traits in studies employing more than one IL-1 polymorphic locus, the simple one locus case-control analysis can be extended to one involving several loci (given a sufficient sample size). In a similar way, a larger contingency table can be calculated, with groups corresponding now to composite genotypes. As before, a chi-squared statistic can be calculated. With these large contingency tables, it is likely that the validity of the chi-square test is violated (<80% of expected values >5, and expected values <1). With smaller contingency tables, the usual remedy to violations of validity is to use Fishers Exact test, but in this larger case, it is not viable. Instead a null distribution for the evaluated chi-square statistic is simulated, and significance assessed from this. This test has been named the Monte Carlo Composite Genotype (MCCG) test.

### 5.12 Example 12

In this example, haplotype relative risk (HRR) analysis is discussed. This analysis is only suitable for qualitative traits (quantitative traits may be used if, dichotomised), and as with all association tests, the candidate gene approach. Haplotype analysis investigates the association between specific genetic markers for diseases and the way a set of markers may influence the outcome of the disease. Analyzing the relationship between specific genetic markers and disease is an extremely complex process. The analysis needs to take into account (i) the relation between genetic markers in neighboring genes, (ii) the way the polymorphic markers affect expression of the gene in question, (iii) the distribution of the genetic markers for a specific polymorphism over both chromosomes, and (iv) the way the expressed gene product(s) affect the disease process.
The relationship between these factors can be identified by statistical equations that look at multipoint linkage analysis, transmission/disequilibrium test (TDT), multipoint quantitative trait loci (QTL) analysis, identity-by-state (IBS), identity-by-descent (IBD), and grouping of multiallelic markers for biological functions related to disease. This approach has been described by Camp ((1997) American Journal of Human Genetics 61: 1424-30); Cox et al ((1998) American Journal of Human Genetics 62: 1180-88); and Almasy and Blangero ((1998) American Journal of Human Genetics 62: 1198-1211).

To perform a HRR analysis (Falk et al. (1987) Ann Hum Genet 51: 27-233) nuclear families with affected offspring are needed. This type of analysis uses an artificial internal control, and therefore the problem of collecting an independent matched control population is removed. The parents and affected offspring are genotyped. It is then established which parental alleles were passed on to the affected offspring and which were not. From this the transmitted genotype and the non-transmitted genotype (internal control) are determined and recorded in the transmitted and non-transmitted groups, respectively. The two groups are then tested for significant differences in the proportions of their genotypes.

### 5.13 Example 13

In this example, the transmission/ disequilibrium test (TDT) is discussed. This analysis is suitable for qualitative traits investigated using a candidate gene approach. Nuclear families are needed, including at least one parent, all affected offspring, and if possible an unaffected sibling.

The TDT (Spielman et al. (1993) Am J Hum Genet 52: 506-16) is a test for both association and for linkage, more specifically, it tests for linkage in the presence of association. Thus, if association does not exist at the locus of interest, linkage will not be detected even if it exists. It is for this reason that the test has been included in this section. It may be used as an initial test, but is more commonly used when tentative evidence for association has already been identified. In this case, a positive result will not only confirm the initial association, but also provide evidence for linkage.

All parents and affected offspring are genotyped. Only parents heterozygous for the allele of interest may be used in the analysis. If the allele of interest is, or is linked to, the disease allele, the transmission rate for that allele from heterozygous parents to their affected offspring should be elevated. To test if the transmission rate of the allele of interest is significantly elevated, the number of times it is transmitted, b, and the number of times other alleles are transmitted, c, are counted. The squared difference of b and c divided by their sum provides a statistic that follows a chi-square distribution with one degree of freedom, and can thus be assessed for significant deviation from the expected under no association or linkage. It is often advised to repeat this procedure using the unaffected offspring from the same parents to rule out the possibility of a spurious result due to biased meioses.

The TDT may also be used once linkage on a coarse scale has been shown to provide the fine scale mapping that is necessary to pin-point more accurately the disease locus. Of course, these tests are only valid when associations within the area also exist.

### 5.14 Example 14

In this example, the non-parametric linkage analysis is discussed. Non-parametric linkage analysis methods (such as Affected Sib-Pair analysis, the Haseman-Elston method and Variance Component Method) are based on the allele sharing status of affected relative pairs, usually sibs. These methods are suitable for whole genome screens (commonly done at 10 cM intervals) and also a candidate gene approach (although for fine localisation alternative methods such as the TDT (section 4.2.1.3) should be used).

### 5.15 Example 15

In this example the analysis of significance and power of the data is examined. Throughout this section, evidence strong enough to suggest association or linkage has been termed significant. The significance level of a test is left to the discretion of the investigator, but conventionally a 5% significance level is used. This means that it is accepted that there is enough evidence to suggest an association (or linkage) if the result would have occurred only 1 in 20 (0.05) times by chance in data where no association (linkage) existed, that is, there is only a 0.05 chance that the result is a false-positive. For each test a p-value may be calculated which indicates the probability of the result occurring by chance. In a single test, if this value is less than 0.05 then significant evidence may be claimed. This concept becomes more complicated when multiple, independent tests are performed. For example, if two tests were performed, and each was tested at the 5% level of significance, overall there is a 2 in 20 (0.1) chance of at least one result being a false-positive. Thus, for two independent tests, to maintain an overall significance level of 0.05 (0.05 chance of at least one test being a false positive) either the individual significance level for each test must be lowered to 0.05/2 = 0.025, or the p-values doubled before assessing the result. This method of correction is called the Bonferroni correction. More generally, if *n* independent tests were carried out, each individual test should be tested at the 0.05/n level, or alternatively, every p-value multiplied by n before assessing the results. With non-independent tests, however, the Bonferroni correction may be too conservative.

Many investigators may find that they lose their potential significances through the dilution of p-values due to the correction criteria for multiple tests. Unfortunately these corrections are necessary for statistical correctness and cannot be discarded. However, if the results from the first set of observations are real, a second replication sample need only test those interesting results found from the first. This reduces the number of tests necessary on the second set of observations and thus reduces the dilution, increasing the chance of maintaining the statistical significance that may have been lost the first time. For complex diseases where there are so many questions to be answered it is perhaps unreasonable to expect that a single sample would be sufficient, and instead anticipate the necessity for a two-stage analysis and prepare accordingly. This is especially true for whole genome screens where the corrections necessary are massive. Lander et al. ((1995) Nature Genet 11: 241-7) list sensible guidelines for claiming significance in linkage analyses, specifically in the case of genome screens.

Along with significance, a second, and equally important issue is that of power, the ability to pick up significant evidence where it actually exists. Given the phenotype, data structure and number of observations, it is important to choose the method of analysis which is most likely to determine associations or linkages if they exist. In fact, it is advisable that in the planning stages of these studies the number of observations that are necessary to reach a predetermined power level are calculated. Unfortunately, this task is not as simple as it sounds, since power depends on several factors, of which some may be unknown, for example, allele frequencies, marker informativeness, familial clustering of the disease, recombination between marker and disease locus. Even if these factors are known, the power cannot be explicitly calculated for some methods, and instead empirical powers must be worked out via simulations.

There is no clear answer to which analyses should be done in different situations because of the many variables that are involved. However, it is strongly advisable to make the most informed choice possible, using previous work that has been done, to increase the chances of detection and location of genes responsible, or involved in complex diseases.

## Claims

1. A method for determining whether a subject has or is predisposed to developing an arterial restenosis, comprising detecting an IL-1RN (VNTR) allele in a nucleic acid sample from the subject, wherein detection of the restenosis IL-1RN (VNTR) allele 2 indicates that the subject is not predisposed to the development of a restenosis.

2. A method for determining whether a subject has or is predisposed to developing an arterial restenosis, comprising detecting an IL-1RN (VNTR) allele in a nucleic acid sample from the subject, wherein detection of IL-1RN (VNTR) allele 1 indicates that the subject is predisposed to the development of a restenosis.

3. A method of claim 1 or claim 2 wherein said subject is a candidate for percutaneous transluminal coronary angioplasty.

4. A method of any one of claims 1 to 3, wherein said detecting step is:
a) allele specific oligonucleotide hybridization;
b) size analysis;
c) sequencing;
d) hybridization;
e) 5' nuclease digestion;
f) single-stranded conformation polymorphism;
g) allele specific hybridization;
h) primer specific extension; or
j) oligonucleotide ligation assay.

5. A method of any one of claims 1 to 4, wherein prior to or in conjunction with detection, the nucleic acid sample is subject to an amplification step.

6. A method of claim 5, wherein said amplification step employs a primer selected from the group consisting of SEQ IDs Nos. 7-8.

7. A method of claim 6, wherein said amplification step employs a primer pair consisting of SEQ ID Nos. 7 and 8.

8. A method of claim 4, wherein said size analysis is preceded by a restriction enzyme digestion.

9. A method of claim 8, wherein said restriction enzyme digestion uses a restriction enzyme selected from the group consisting of Alu I, Msp I, Nco I, Fnu 4HI, Ava I, Bsu 361 and TaqI.

## Patentansprüche

1. Methode zum Bestimmen, ob ein Subjekt eine arterielle Restenose aufweist oder für deren Entwicklung prädisponiert ist, umfassend das Nachweisen eines IL-1RN- (VNTR) -Allels in einer Nukleinsäure-Probe von dem Subjekt, wobei der Nachweis des Restenose IL-1RN (VNTR) Allels 2 anzeigt, dass das Subjekt nicht für die Entwicklung eines Restenose prädisponiert ist.

2. Methode zum Bestimmen, ob ein Subjekt eine arterielle Restenose aufweist oder für deren Entwicklung prädisponiert ist, umfassend das Nachweisen eines IL-1RN- (VNTR) -Allels in einer Nukleinsäure-Probe von dem Subjekt, wobei der Nachweis des IL-1RN- (VNTR) -Allels 1 anzeigt, dass das Subjekt für die Entwicklung einer Restenose prädisponiert ist.

3. Methode nach Anspruch 1 oder Anspruch 2, wobei das Subjekt ein Kandidat für eine perkutane transluminale koronare Angioplastie ist.

4. Methode nach einem der Ansprüche 1 bis 3, worin der Nachweisschritt ist:
a) Allel-spezifische Oligonukleotid-Hybridisation;
b) Größenanalyse;
c) Sequenzierung;
d) Hybridisation;
e) 5'-Nuklease-Verdau;
f) einzelsträngiger Konformationspolymorphismus;
g) Allel-spezifische Hybridisation;
h) Primer-spezifische Extension; oder
i) Oligonukleotid-Ligationsassay.

5. Methode nach einem der Ansprüche 1 bis 4, worin vor oder in Verbindung mit dem Nachweis, die Nukleinsäure-Probe einem Amplifikationsschritt unterzogen wird.

6. Methode nach Anspruch 5, wobei der Amplifikationsschritt einen Primer verwendet, ausgewählt aus der Gruppe, bestehend aus SEQ ID Nrn. 7-8.

7. Methode nach Anspruch 6, wobei der Amplifikationsschritt ein Primer-Paar verwendet, bestehend aus SEQ ID Nrn. 7 und 8.

8. Methode nach Anspruch 4, wobei der Größenanalyse ein Restriktionsenzym-Verdau vorangeht.

9. Methode nach Anspruch 8, wobei der Restriktionsenzym-Verdau ein Restriktionsenzym verwendet, ausgewählt aus der Gruppe, bestehend aus Alu I, Msp I, Nco I, Fnu, 4HI, Ava I, Bsu 361 und Taq I.

## Revendications

1. Procédé pour déterminer si un sujet présente une resténose artérielle ou est prédisposé au développement d'une resténose artérielle, comprenant la détection d'un allèle IL-1RN (VNTR) dans un échantillon d'acide nucléique prélevé sur le sujet, la détection de l'allèle 2 IL-1RN (VNTR) de la resténose indiquant que le sujet n'est pas prédisposé au développement d'une resténose.

2. Procédé pour déterminer si un sujet présente une resténose artérielle ou est prédisposé au développement d'une resténose artérielle, comprenant la détection d'un allèle IL-1RN (VNTR) dans un échantillon d'acide nucléique prélevé sur le sujet, la détection de l'allèle 1 IL-1RN (VNTR) indiquant que le sujet est prédisposé au développement d'une resténose.

3. Procédé selon la revendication 1 ou la revendication 2, dans lequel ledit sujet est candidat à une angioplastie coronarienne transluminale percutanée.

4. Procédé selon l'une quelconque des revendications 1 à 3, dans lequel ladite étape de détection est :
a) une hybridation d'un oligonucléotide spécifique d'un allèle ;
b) une analyse de taille ;
c) un séquençage ;
d) une hybridation ;
e) une digestion par une nucléase 5' ;
f) un polymorphisme conformationnel simple brin ;
g) une hybridation allèle-spécifique ;
h) un allongement spécifique d'une amorce ; ou
j) un essai de ligature d'oligonucléotide.

5. Procédé selon l'une quelconque des revendications 1 à 4, dans lequel, avant ou conjointement avec la détection, l'échantillon d'acide nucléique est soumis à une étape d'amplification.

6. Procédé selon la revendication 5, dans lequel ladite étape d'amplification emploie une amorce choisie dans le groupe constitué par les SEQ ID N° 7 à 8.

7. Procédé selon la revendication 6, dans lequel ladite étape d'amplification emploie une paire d'amorces constituée de SEQ ID N° 7 et 8.

8. Procédé selon la revendication 4, dans lequel l'analyse de taille est précédée d'une digestion par enzyme de restriction.

9. Procédé selon la revendication 8, dans lequel ladite digestion par enzyme de restriction utilise une enzyme de restriction choisie dans le groupe constitué par Alu I, Msp I, Nco I, Fnu 4HI, Ava I, Bsu 36I et TaqI.
